(19) Europäisches Patentamt
European Patent Office
Office européen des brevets

(11) **EP 4 559 920 A1**

(12) **EUROPEAN PATENT APPLICATION**
published in accordance with Art. 153(4) EPC

(43) Date of publication:
**28.05.2025 Bulletin 2025/22**

(21) Application number: **23842051.7**

(22) Date of filing: **29.06.2023**

(51) International Patent Classification (IPC):
***C07D 498/18*** *(2006.01)* ***C07D 498/22*** *(2006.01)*
***A61K 31/439*** *(2006.01)* ***A61P 35/00*** *(2006.01)*

(52) Cooperative Patent Classification (CPC):
**A61K 31/439; A61P 35/00; C07D 498/18; C07D 498/22**

(86) International application number:
**PCT/CN2023/103621**

(87) International publication number:
**WO 2024/016986 (25.01.2024 Gazette 2024/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.07.2022 PCT/CN2022/106998**
**29.09.2022 PCT/CN2022/122422**
**22.12.2022 PCT/CN2022/140964**
**23.05.2023 PCT/CN2023/095846**

(71) Applicant: **Betta Pharmaceuticals Co., Ltd**
**Yuhang**
**Hangzhou**
**Zhejiang 311100 (CN)**

(72) Inventors:
- **LIU, Xiangyong**
  **Beijing 100176 (CN)**
- **FU, Bang**
  **Beijing 100176 (CN)**
- **QIU, Changyong**
  **Beijing 100176 (CN)**
- **SONG, Xiaodong**
  **Beijing 100176 (CN)**
- **REN, Wei**
  **Beijing 100176 (CN)**
- **WANG, Yiqian**
  **Beijing 100176 (CN)**
- **CHEN, Jie**
  **Beijing 100176 (CN)**
- **BAI, Jinlong**
  **Beijing 100176 (CN)**
- **SUN, Yun**
  **Beijing 100176 (CN)**
- **ZHANG, Jian**
  **Beijing 100176 (CN)**
- **ZHANG, Chunhui**
  **Beijing 100176 (CN)**
- **LI, Yinlong**
  **Beijing 100176 (CN)**
- **LIU, Lijia**
  **Beijing 100176 (CN)**
- **GUO, Jing**
  **Beijing 100176 (CN)**
- **LAN, Hong**
  **Beijing 100176 (CN)**
- **DING, Lieming**
  **Hangzhou, Zhejiang 311100 (CN)**
- **WANG, Jiabing**
  **Beijing 100176 (CN)**

(74) Representative: **Staeger & Sperling Partnerschaftsgesellschaft mbB**
**Sonnenstraße 19**
**80331 München (DE)**

# (54) MACROCYCLIC COMPOUND AND PHARMACEUTICAL COMPOSITION AND USE THEREOF

(57) The present invention relates to a macrocyclic inhibitor, a composition and a use thereof. In particular, the present invention relates to a compound as shown in formula (I), a pharmaceutical composition comprising the compound of the present invention, and a use thereof as an EGFR inhibitor in the treatment of cancer-related diseases.

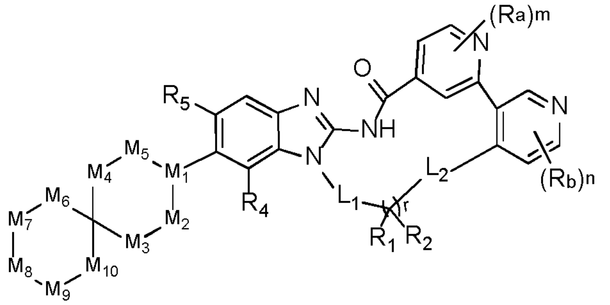

(I)

## Description

### Technical field

The invention belongs to the field of medicine, and specifically relates to macrocyclic compounds, pharmaceutical composition and application thereof.

### Background

Epidermal growth factor receptor (EGFR) is a transmembrane glycoprotein that belongs to the ErbB family of tyrosine kinase receptors. Activation of EGFR results in autophosphorylation of the receptor tyrosine kinase, which initiates a cascade of downstream signaling pathways involved in regulating cell proliferation, differentiation, and survival. EGFR is abnormally activated by various mechanisms, such as receptor overexpression, mutation, ligand-dependent receptor dimerization, and ligand-independent activation, and is associated with the development of various human cancers.

In the field of cancer, lung cancer is one of the malignant tumors with the highest increase in morbidity and mortality and the greatest threat to human health and life. Therefore, the treatment of lung cancer is a major research focus of anti-tumor drugs. And, since the epidermal growth factor receptor (EGFR) is the main driver of lung cancer (mutations account for more than 20%, and it is more prevalent to Asian populations), the development of EGFR inhibitors for targeted treatment of lung cancer patients has attracted the extensive attention of drug researchers.

EGFR mutations account for approximately 12-47% of non-small cell lung cancer (NSCLC).The two most common mutations are deletion mutations in exon 19 (del 19) and point mutations in exon 21 (mainly L858R).The resulting abnormal ligand-independent activation of epidermal growth factor receptor (EGFR) will promote the proliferation of tumor cells, which is also the scientific basis for the development of EGFR inhibitors. The clinical first-generation (Gefitinib" Iressa®", Erlotini" Tarceva®", and icotinib" Conmana®"), second-generation (Afatinib"Gilotrif®", Dacomitinib"VIZIMPRO®" and Neratinib"Nerlynx®") and third-generation (osimertinib, AZD9291) EGFR inhibitors have an objective response rate of approximately 60-85% in NSCLC tumors with these two mutations. However, this response does not last for life. Patients usually develop varying degrees of drug resistance 9.2 to 14.7 months after taking first- or second-generation EGFR inhibitors. Approximately 50-70% of these drug resistance mutations occur in the gatekeeper T790M on EGFR. This mutation can cause changes in the spatial conformation of EGFR, increase the affinity of EGFR for ATP, and thereby weaken the binding ability of the inhibitor to EGFR. Although the second-generation EGFR inhibitor Afatinib has inhibitory activity against EGFR-T790M mutation in vitro, it still fails to overcome the resistance caused by T790M mutation in clinical application. And first- or second-generation EGFR inhibitors are difficult to eliminate inhibition of wild-type EGFR, resulting in significant skin toxicity (such as acne-like rash). This situation was not resolved until the emergence of the third-generation inhibitor osimertinib.

Although osimertinib has addressed the issue of the T790M mutation, but clinically, resistance has been observed in EGFR T790M-positive NSCLC patients treated with osimertinib as second-line therapy, with resistance emerging after 10 months of treatment, among which 20-40% are due to the C797S mutation (i.e., triple mutations in cis or trans configuration including del19/T790M/C797S or L858R/T790M/C797S). At the same time, osimertinib also has a good effect on EGFR-sensitive mutations (del19 or L858R), and has been approved for first-line indications. But resistance mutations occur after about 19 months of use, among which 7% are C797S double mutations, namely del19/C797S or L858R/C797S.

At present, there are three generations of EGFR inhibitors that have been approved and launched. With the approval of first-line and second-line indications, they are widely used in clinical practice. However, drug resistance often occurs after a period of use, which reduces the effective survival period of patients. Therefore, there is an urgent need to develop next-generation EGFR inhibitors to meet more clinical needs.

### Summary

To address the aforementioned issues, the present invention provides a macrocyclic compound, which can be used as an inhibitor of various mutations of EGFR for the treatment of cancer.

The macrocyclic compound provided by the present invention is a compound as shown in formula (I), or its stereoisomer, tautomer, solvate, pharmaceutically acceptable salt, or deuterated derivative:

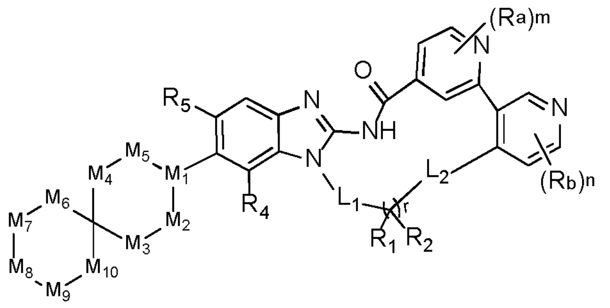

formula (I)

wherein,

$L_1$, $L_2$ are independently selected from the group consisting of bond, $-CR_hR_i-$, $-NR_h-$ and -O-;

each of $R_a$, $R_b$ is the same or different, independently selected from the group consisting of H, deuterium, halogen, CN, $NO_2$, $-OR_e$, $-SR_e$, $-S(O)R_e$, $-S(O)_2R_e$, $-NR_eR_f$, $C_{1-6}$ alkyl, $C_{2-6}$ alkenyl and $C_{2-6}$ alkynyl; wherein, the $C_{1-6}$ alkyl, $C_{2-6}$ alkenyl, $C_{2-6}$ alkynyl, $R_e$ and $R_f$ are optionally substituted with one or more deuterium, halogen, CN, oxo, $C_{1-6}$ alkyl, $C_{1-6}$ haloalkyl, $C_{2-6}$ alkenyl, $C_{2-6}$ alkynyl, $-OR_h$ or $-NR_hR_i$;

each of $R_1$ and $R_2$ is the same or different, independently selected from $R_h$; or

$R_1$ and $R_2$, together with the atoms to which they are attached, form a 3-14 membered heteroaryl group or a $C_{3-14}$ carbocyclic group; wherein the 3-14 membered heteroaryl group or $C_{3-14}$ carbocyclic group is optionally substituted with one or more $R_h$;

$M_1$ is selected from the group consisting of $CR_c$ and N;

$M_3$, $M_4$, $M_7$, $M_8$ and $M_9$ are independently selected from the group consisting of $CR_cR_c$ and $NR_c$;

$M_2$, $M_5$, $M_6$ and $M_{10}$ are independently selected from the group consisting of absent, $CR_cR_c$, $-CR_cR_c-CR_cR_c-$ and $NR_c$;

$R_4$ is H, deuterium, halogen, $-NH_2$, $C_{1-6}$ alkyl, $C_{1-6}$ haloalkyl, $C_{2-6}$ alkenyl, $C_{2-6}$ haloalkenyl, $C_{2-6}$ alkynyl, $C_{2-6}$ halogenated alkynyl, $C_{1-6}$ alkoxy, $C_{1-6}$ haloalkoxy, $C_{3-6}$ cycloalkyl, $C_{3-6}$ halocycloalkyl or cyano;

$R_5$ is H, deuterium, halogen, $-NH_2$, -N=S(O)ReR$_f$, -OH, -C(=O)OH, $-C(=O)NH_2$, $C_{1-6}$ alkyl, $C_{2-6}$ alkenyl, $C_{2-6}$ alkynyl, $C_{1-6}$ alkoxy, $-NH(C_{1-6}$ alkyl), $-N(C_{1-6}$ alkyl$)_2$, $C_{3-8}$ cycloalkyl, 3-8 membered heterocyclyl, $C_{6-10}$ aryl or 5-10 membered heteroaryl; wherein, the $-NH_2$, -N=S(O)ReR$_f$, -OH, -C(=O)OH, $-C(=O)NH_2$, $C_{1-6}$ alkyl, $C_{2-6}$ alkenyl, $C_{2-6}$ alkynyl, $C_{1-6}$ alkoxy, $-NH(C_{1-6}$ alkyl), $-N(C_{1-6}$ alkyl$)_2$, $C_{3-8}$ cycloalkyl, 3-8 membered heterocyclyl, $C_{6-10}$ aryl and 5-10 membered heteroaryl are optionally substituted with one or more $R_h$;

each of $R_c$ is the same or different, and is independently selected from the group consisting of H, deuterium, halogen, CN, $NO_2$, oxo, $C_{1-6}$ alkyl, $C_{2-6}$ alkenyl, $C_{2-6}$ alkynyl, $-C_{0-6}$ alkylene $-C_{6-14}$ aryl, $-C_{0-6}$ alkylene -5-14 membered heteroaryl, $-C_{0-6}$ alkylene $-C_{3-14}$ carbocyclyl, $-C_{0-6}$ alkylene-3-14 membered heterocyclyl, $-OR_e$, $-NR_eR_f$, $-SR_e$, $-C(O)R_e$, $-N(R_e)C(O)R_f$, $-C(O)NR_eR_f$, $-N(R_e)C(O)OR_f$, $-OC(O)NR_eR_f$, $-N(R_e)C(O)NR_fR_g$, $-S(O)R_e$, $-S(O)(=NR_e)R_f$, $-S(O)_2R_e$, $-S(O)NR_eR_f$, $-S(O)_2NR_eR_f$, $-N(R_e)S(O)R_f$, $-N(R_e)S(O)_2R_f$, $-C(O)OR_e$, $-OC(O)R_f$ and $-OC(O)OR_g$; wherein, the $C_{1-6}$ alkyl, $C_{2-6}$ alkenyl, $C_{2-6}$ alkynyl, $-C_{0-6}$ alkylene $-C_{6-14}$ aryl, $-C_{0-6}$ alkylene -5-14 membered heteroaryl, $-C_{0-6}$ alkylene $-C_{3-14}$ carbocyclyl, $-C_{0-6}$ alkylene -3-14 membered heterocyclyl, $R_e$, $R_f$ and $R_g$ are optionally substituted with one or more $R_h$, $-OR_h$, $-NR_hR_i$, $-C(O)OR_h$, $-OC(O)R_h$, $-COR_h$, $-C(O)NR_hR_i$;

$R_e$, $R_f$, $R_g$, $R_h$, $R_i$ are independently selected from the group consisting of hydrogen, deuterium, halogen, CN, OH, $NH_2$, -COOH, $-NH(C_{1-6}$ alkyl), $-N(C_{1-6}$ alkyl$)_2$, oxo, $C_{1-6}$ alkyl, $C_{1-6}$ alkoxy, $C_{2-6}$ alkenyl, $C_{2-6}$ alkynyl, $-O-C_{3-14}$ cycloalkyl, $-O-C_{3-14}$ heterocyclyl, $-S-C_{3-14}$ heterocyclyl, $-S-C_{3-14}$ cycloalkyl, $-S-C_{1-6}$ alkyl, $-CONH_2$, $-CONH(C_{1-6}$ alkyl), $-CON(C_{1-6}$ alkyl$)_2$, $-CONH(C_{2-6}$ alkenyl), $-CON(C_{2-6}$ alkenyl$)_2$, $-CONH(C_{2-6}$ alkynyl), $-CON(C_{2-6}$ alkynyl$)_2$, $-S(O)-C_{1-6}$ alkyl, $-S(O)_2-C_{1-6}$ alkyl, $-S(O)-C_{2-6}$ alkynyl, $-S(O)_2-C_{2-6}$ alkynyl, $-S(O)-C_{2-6}$ alkynyl, $-S(O)_2-C_{2-6}$ alkynyl, $-S(O)-C_{3-14}$ cycloalkyl, $-S(O)_2-C_{3-14}$ heterocyclyl, $-S(O)NH_2$, $-S(O)NHC_{1-6}$ alkyl, $-S(O)N(C_{1-6}$ alkyl$)_2$, -CHO, $-C(O)-C_{1-6}$ alkyl, $-C(O)-C_{2-6}$ alkenyl, $-C_{0-6}$ alkylene $-C_{6-14}$ aryl, $-C_{0-6}$ alkylene -5-14 membered heteroaryl, $-C_{0-6}$ alkylene $-C_{3-14}$ carbocyclyl and $-C_{0-6}$ alkylene -3-14 membered heterocyclyl; wherein, the $-NH(C_{1-6}$ alkyl), $-N(C_{1-6}$ alkyl$)_2$, $C_{1-6}$ alkyl, $C_{1-6}$ alkoxy, $C_{2-6}$ alkenyl, $C_{2-6}$ alkynyl, $-O-C_{3-14}$ cycloalkyl, $-O-C_{3-14}$ heterocyclyl, $-S-C_{3-14}$ heterocyclyl, $-S-C_{3-14}$ cycloalkyl, $-S-C_{1-6}$ alkyl, $-CONH(C_{1-6}$ alkyl), $-CON(C_{1-6}$ alkyl$)_2$, $-CONH(C_{2-6}$ alkenyl), $-CON(C_{2-6}$ alkenyl$)_2$, $-CONH(C_{2-6}$ alkenyl), $-CON(C_{2-6}$ alkenyl$)_2$, $-S(O)-C_{1-6}$ alkyl, $-S(O)_2-C_{1-6}$ alkyl, $-S(O)-C_{2-6}$ alkenyl, $-S(O)_2-C_{2-6}$ alkenyl, $-S(O)-C_{2-6}$ alkynyl, $-S(O)_2-C_{2-6}$ alkynyl, $-S(O)-C_{3-14}$ cycloalkyl, $-S(O)_2-C_{3-14}$ heterocyclyl, $-S(O)NHC_{1-6}$ alkyl, $-S(O)N(C_{1-6}$ alkyl$)_2$, $-C(O)-C_{1-6}$ alkyl, $-C(O)-C_{2-6}$ alkenyl, $-C_{0-6}$ alkylene $-C_{6-14}$ aryl, $-C_{0-6}$ alkylene -5-14 membered heteroaryl, $-C_{0-6}$ alkylene-$C_{3-14}$ cycloalkyl and $-C_{0-6}$ alkylene-3-14 membered heterocyclyl are substituted with one or more of hydrogen, deuterium, halogen, CN, OH, $NH_2$, -COOH, oxo, $C_{1-6}$ alkyl, $C_{1-6}$ haloalkyl, $C_{2-6}$ alkenyl, $C_{2-6}$ haloalkenyl, $C_{2-6}$ alkynyl, $C_{2-6}$ haloalkynyl, $C_{1-6}$ alkoxy, $C_{1-6}$ haloalkoxy, $-NH(C_{1-6}$alkyl),

-NH($C_{1-6}$ haloalkyl), -N($C_{1-6}$alkyl)$_2$, -N($C_{1-6}$ haloalkyl)$_2$, -N($C_{1-6}$ alkyl)($C_{1-6}$ haloalkyl), -S(O)-$C_{1-6}$ alkyl, -S(O)$_2$-$C_{1-6}$ alkyl, -S(O)-$C_{2-6}$ alkenyl, -S(O)$_2$-$C_{2-6}$ alkenyl, -S(O)-$C_{2-6}$ alkynyl, -S(O)$_2$-$C_{2-6}$ alkynyl, -S(O)-$C_{3-14}$ cycloalkyl, -S(O)$_2$-$C_{3-14}$ heterocyclyl, -S(O)NH$C_{1-6}$ alkyl, -S(O)N($C_{1-6}$ alkyl)$_2$, -COO($C_{1-6}$ alkyl), -COO($C_{1-6}$ haloalkyl), -CONH($C_{1-6}$ alkyl), -CONH($C_{1-6}$ haloalkyl), -CON($C_{1-6}$ alkyl)$_2$, -CON($C_{1-6}$ haloalkyl)$_2$, -CON($C_{1-6}$ alkyl)($C_{1-6}$ haloalkyl), -$C_{0-6}$ alkylene-NHC(=O) $C_{1-6}$ alkyl, -$C_{0-6}$ alkylene-NHC(=O) $C_{1-6}$ haloalkyl, -$C_{0-6}$ alkylene -NHC(=O) $C_{2-6}$ alkenyl, substituted or unsubstituted -$C_{0-6}$ alkylene-$C_{3-14}$ carbocyclyl, substituted or substituted -$C_{0-6}$ alkylene -3-14 membered heterocyclyl;
m is 0, 1, 2 or 3;
n is 0, 1, 2 or 3; or
r is 0, 1, 2, 3, 4 or 5.

In some embodiments, $L_1$ in formula (I) is -$CR_hR_i$-.
In some embodiments, $L_1$ in formula (I) is -$CH_2$-.
In some embodiments, $L_2$ in formula (I) is selected from the group consisting of bond and -O-.
In some embodiments, $R_1$ or $R_2$ in formula (I) is independently selected from the group consisting of H, $C_{1-6}$ alkyl, deuterated $C_{1-6}$ alkyl, hydroxy, $C_{1-6}$ haloalkyl, halogen, -$NH_2$, $C_{1-6}$ alkoxy and $C_{1-6}$ haloalkoxy.
In some embodiments, $R_1$ or $R_2$ in formula (I) is H or $C_{1-3}$ alkyl.
In some embodiments, $R_1$ and $R_2$ in formula (I) together with the atoms to which they are attached, form

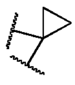, 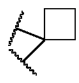, 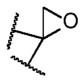, 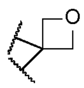, 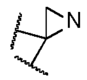 or 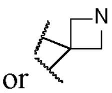.

In some embodiments,

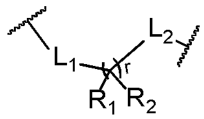

in formula (I) is

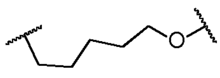,

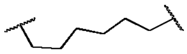, 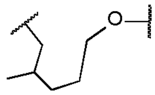, 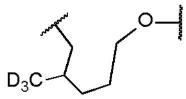, 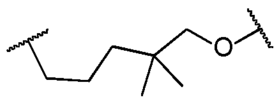,

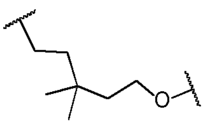 or 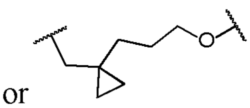.

In some embodiments, $R_a$ in formula (I) is H, deuterium, halogen, CN, OH, $NH_2$, $C_{1-6}$ alkyl, deuterated $C_{1-6}$ alkyl, $C_{2-6}$ alkenyl, $C_{2-6}$ alkynyl, $C_{1-6}$ alkoxy, $C_{1-6}$ haloalkyl or -NH-C(O)-$C_{1-6}$ alkyl.
In some embodiments, $R_a$ in formula (I) is H, deuterium, F, Cl, CN, $NH_2$, $C_{1-3}$ alkyl, deuterated $C_{1-3}$ alkyl, $C_{2-4}$ alkynyl, $C_{1-3}$ alkoxy or $C_{1-3}$ haloalkyl.
In some embodiments, $R_a$ in formula (I) is H or $CH_3$.
In some embodiments, $R_b$ in formula (I) is H, deuterium, halogen, CN, OH, $NH_2$, $C_{1-6}$ alkyl, deuterated $C_{1-6}$ alkyl, $C_{1-6}$ haloalkyl, $C_{1-6}$ alkoxy, $C_{1-6}$ haloalkoxy, -S-$C_{1-6}$ alkyl, -S(=O)-$CH_3$, -S(=O)$_2$-$C_{1-6}$ alkyl, -S(=O)$_2$-$C_{3-6}$ cycloalkyl or -O-3-6 membered heterocyclyl.
In some embodiments, $R_b$ in formula (I) is H, deuterium, F, Cl, -$OCH_3$, $C_{1-3}$ alkyl, $CD_3$, $CF_3$, CN, $NH_2$, -S-$CH_3$, -S(=O)-$CH_3$,

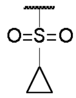 or 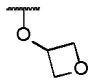.

In some embodiments, $R_b$ in formula (I) is H or $C_{1-3}$ alkyl; preferably, $R_b$ is H or $CH_3$.

In some embodiments, $R_4$ in formula (I) is H.

In some embodiments, $R_5$ in formula (I) is H, halogen, $C_{1-6}$ alkyl, $-NH_2$, $-NH(C_{1-6}$ alkyl), $-N(C_{1-6}$ alkyl$)_2$, $C_{3-6}$ cycloalkyl or 4-6 membered heterocyclyl; wherein, the $C_{1-6}$ alkyl, $-NH_2$, $-NH(C_{1-6}$ alkyl), $-N(C_{1-6}$ alkyl$)_2$, $C_{3-6}$ cycloalkyl and 4-6 membered heterocyclyl are optionally substituted with one or more OH, $NH_2$, oxo, $C_{1-6}$ alkyl, $C_{1-6}$ alkoxy, $-S(O)_2-C_{1-6}$ alkyl, $-C_{1-6}$ alkyl - $S(O)_2-C_{1-6}$ alkyl, $-NH(C_{1-6}$ alkyl) or $-N(C_{1-6}$ alkyl$)_2$.

In some embodiments, $R_5$ in formula (I) is H, F, Cl, $CH_3$, $-NH_2$,

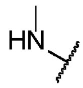, 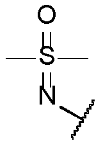,

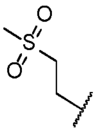, 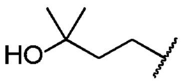, 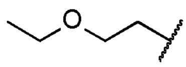, 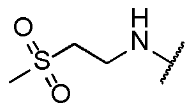, 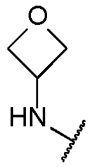, 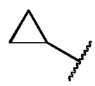,

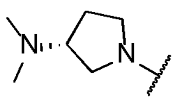, 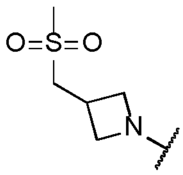, 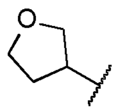, 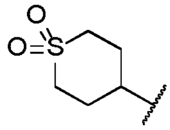, 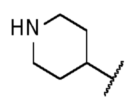, 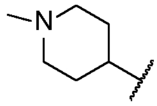

or

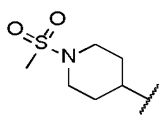.

In some embodiments, $R_5$ in formula (I) is H.

In some embodiments, $R_c$ in formula (I) is H, deuterium, halogen, $NO_2$, CN, OH, $NH_2$, $C_{1-6}$ alkyl, $C_{2-6}$ alkenyl, $C_{2-6}$ alkynyl, $C_{1-6}$ alkoxy, $C_{1-6}$ haloalkyl, $C_{3-6}$ cycloalkyl or 3-6 membered heterocyclyl; wherein, the -OH, $NH_2$, $C_{1-6}$ alkyl, $C_{2-6}$ alkenyl, $C_{2-6}$ alkynyl, $C_{1-6}$ alkoxy, $C_{1-6}$ haloalkyl, $C_{3-6}$ cycloalkyl and 3-6 membered heterocyclyl are optionally substituted with one or more halogen, $C_{1-6}$ alkyl, $C_{1-6}$ alkoxy, $NH_2$, -COOH, $-NH(C_{1-6}$ alkyl), $-N(C_{1-6}$ alkyl$)_2$, $C_{3-6}$ cycloalkyl, 3-6 membered heterocyclyl, $-CONH_2$, $-CONH(C_{1-6}$ alkyl), $-CON(C_{1-6}$ alkyl$)_2$, $-CONH(C_{2-6}$ alkenyl), $-CON(C_{2-6}$ alkenyl$)_2$, $-CONH(C_{2-6}$ alkynyl), $-CON(C_{2-6}$ alkynyl$)_2$, substituted or unsubstituted CO-3-6 membered heterocyclyl, $-O-C_{1-6}$ haloalkyl, $-OC(=O)NH_2$, $-OCONH(C_{1-6}$ alkyl) or $-OCON(C_{1-6}$ alkyl$)_2$.

In some embodiments, $R_c$ in formula (I) is H, deuterium, $CH_3$, $CD_3$, $CH_2CH_3$, $CH(CH_3)_2$, $-C(CH_3)_3$, OH, F, Cl, $NH_2$,

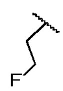, 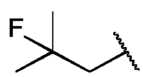, 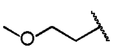, 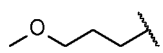,

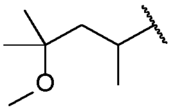, 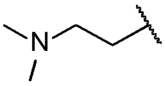, 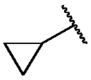, 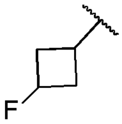, 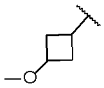, 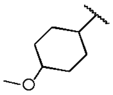, 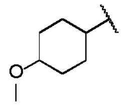,

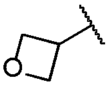, 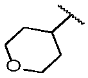, 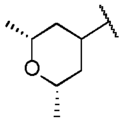, 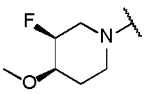, 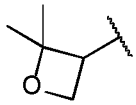, 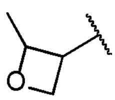, 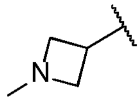,

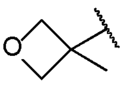, 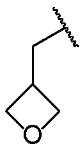, 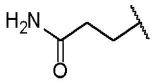, 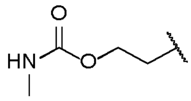, 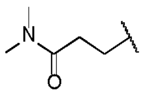, 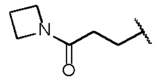,

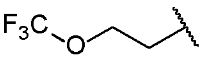, 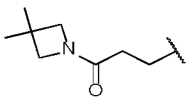 or 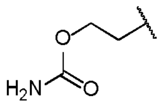.

In some embodiments,

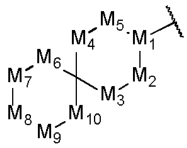

in formula (I) is

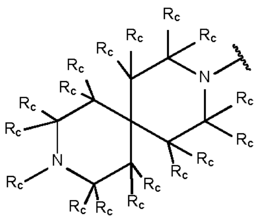,

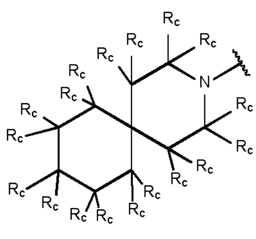 or 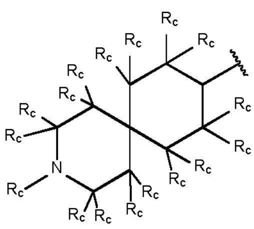.

In some embodiments,

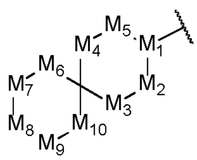

in formula (I) is

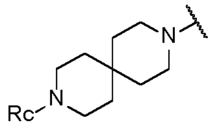

,

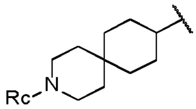

or

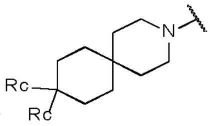

.

In some embodiments,

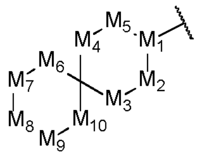

in formula (I) is

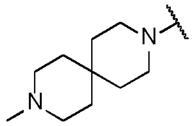

,

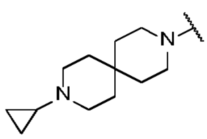

,

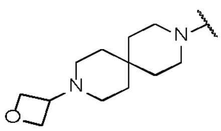

,

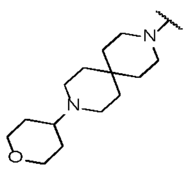

,

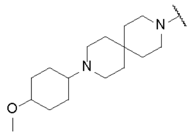

,

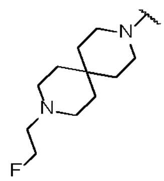

,

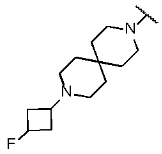

,

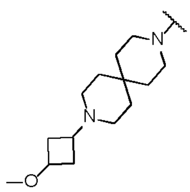

,

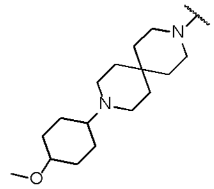

,

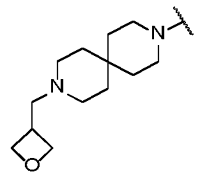

,

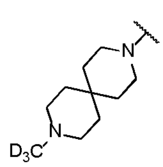

,

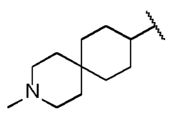

,

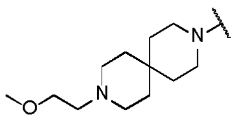

,

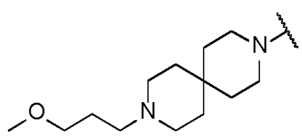

,

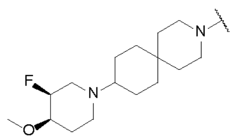

,

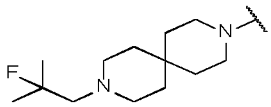

,

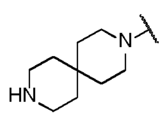

,

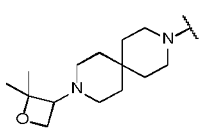

,

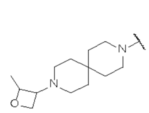

,

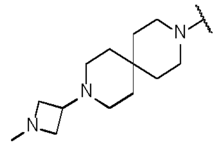

,

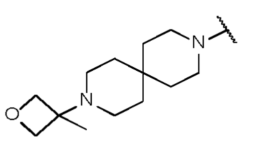, 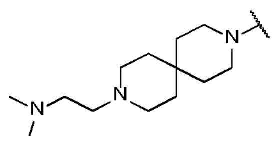, 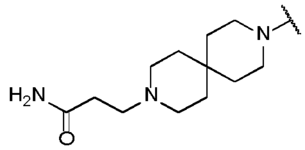,

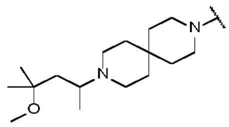, 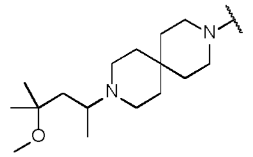, 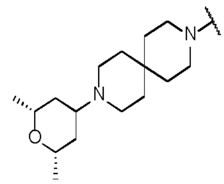, 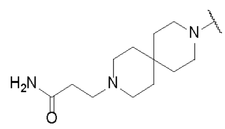,

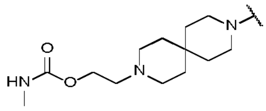, 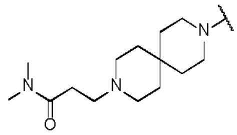, 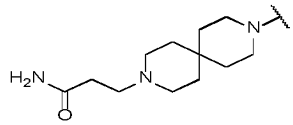, 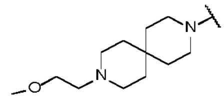,

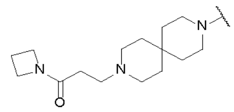, 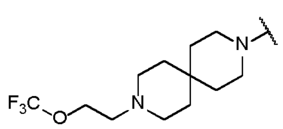, 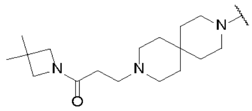 or 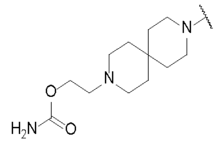.

In some embodiments, m in formula (I) is 0, 1 or 2.

In some embodiments, n in formula (I) is 0, 1 or 2.

In some embodiments, r in formula (I) is 2, 3 or 4.

In some embodiments, the compound of formula (I) is selected from the compounds of formula (II) as shown below:

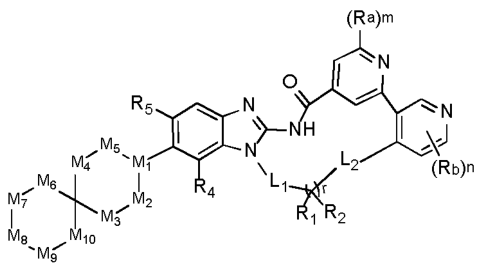

formula (II)

wherein, $L_1$, $L_2$, $R_a$, $R_b$, $R_1$, $R_2$, $R_4$, $R_5$, $M_1$, $M_2$, $M_3$, $M_4$, $M_5$, $M_6$, $M_7$, $M_8$, $M_9$, $M_{10}$, m, n and r in the compound of formula (II) are as defined above in formula (I).

In some embodiments, the compound of formula (I) is selected from the compounds of formula (III) as shown below:

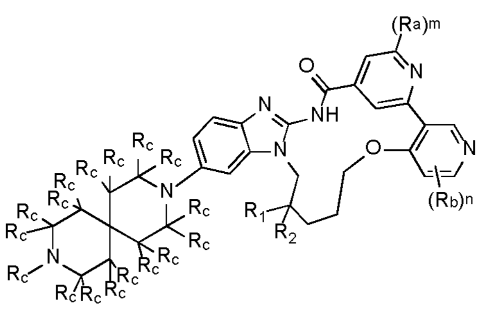

formula (III)

wherein, $R_a$, $R_b$, $R_c$, $R_1$, $R_2$, m and n in the compound of formula (III) are as defined above in formula (I).

In some embodiments, the compound of formula (I) is selected from the compounds of formula (IV) as shown below:

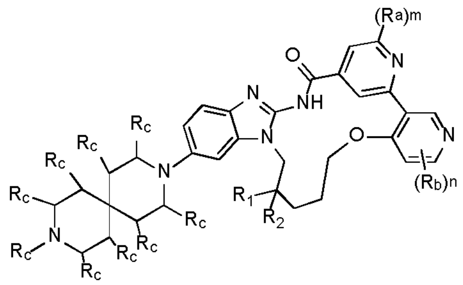

formula (IV)

wherein, $R_a$, $R_b$, $R_c$, $R_1$, $R_2$, m and n in the compound of formula (IV) are as defined above in formula (I).

In some embodiments, the compound of formula (I) is selected from the compounds of formula (V) as shown below:

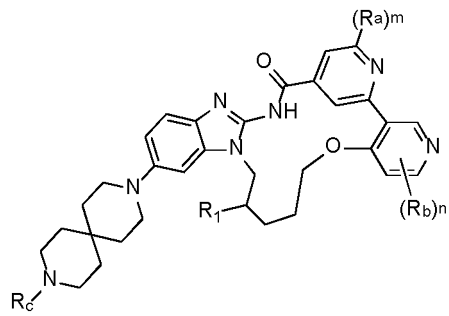

formula (V)

wherein, $R_a$, $R_b$, $R_c$, $R_1$, m and n in the compound of formula (V) are as defined above in formula (I).

In some embodiments, the compound of formula (I) is selected from the group consisting of the compounds below, or its stereoisomer, tautomer, solvate, pharmaceutically acceptable salt or deuterated derivative:

$2^6$-methyl-$5^6$-(9-methyl-3,9-diazaspiro[5.5]undecan-3-yl)-$5^1$H-11-oxa-4-aza-5(2, 1)-benzo[d]imidazola-1(3,4),2(2,4)-dipyridinacycloundecaphan-3-one;

$2^6$-methyl-$5^6$-(9-methyl-3,9-diazaspiro[5.5]undecan-3-yl)-$5^1$H-4-aza-5(2,1)-benz o[d]imidazola-1(3,4),2(2,4)-dipyridinacycloundecaphan-3-one;

(R)-$2^6$,7-dimethyl-$5^6$-(9-methyl-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-one;

(R)-$2^6$,7-dimethyl-$5^6$-(9-(oxetan-3-yl)-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-one;

(R)-$5^5$-fluoro-$2^6$,7-dimethyl-$5^6$-(9-methyl-3,9-diazaspiro[5.5]undecan-3-yl)-$S^1$H-11-oxa-4-aza-5(2,1)-benzo[d]imidazola-1(3,4),2(2,4)-dipyridinacycloundecaphan-3-o ne;

(R)-$5^5$-fluoro-$2^6$,7-dimethyl-$5^6$-(9-(oxetan-3-yl)-3,9-diazaspiro[5.5]undecan-3-yl) -$5^1$H-11-oxa-4-aza-5(2,1)-benzo [d]imidazola-1(3,4),2(2,4)-dipyridinacycloundecapha;

$2^6$,9,9-trimethyl-$5^6$-(9-methyl-3,9-diazaspiro[5.5]undecan-3-yl)-$5^1$H-11-oxa-4-az a-5(2,1)-benzo[d]imidazola-1(3,4),2(2,4)-dipyridinacycloundecaphan-3-one;

(R)-$5^5$-((dimethyl(oxo)-$1^6$-sulfanylidene)amino)-$2^6$,7-dimethyl-$5^6$-(9-methyl-3,9-diazaspiro[5.5]undecan-3-yl)-$5^1$H-11-oxa-4-aza-5(2,1)-benzo[d]imidazola-1(3,4),2(2, 4)-dipyridinacycloundecaphan-3-one;

(R)-$1^6$-fluoro-$2^6$,7-dimethyl-$5^6$-(3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-one;

(R)-$1^6$-fluoro-$2^6$,7-dimethyl-$5^6$-(9-methyl-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-o ne;

(R)-$2^6$,$5^5$,7-trimethyl-$5^6$-(9-methyl-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-one;

(R)-$2^6$,$5^5$,7-trimethyl-$5^6$-(9-(oxetan-3-yl)-3,9-diazaspiro[5.5]undecan-3-yl)-$5^1$H-1 1-oxa-4-aza-5(2,1)-benzo[d]imidazola-1(3,4),2(2,4)-dipyridinacycloundecaphan-3-on e;

$2^{6}$,$5^{5}$-dimethyl-$5^{6}$-(9-methyl-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

$5^{5}$-fluoro-$2^{6}$-methyl-$5^{6}$-(9-methyl-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

$5^{6}$-(9-((3S,4R)-3-fluoro-4-methoxypiperidin-1-yl)-3-azaspiro[5.5]undecan-3-yl)-$2^{6}$-methyl-$5^{1}$H-11-oxa-4-aza-5(2,1)-benzo[d]imidazola-1(3,4),2(2,4)-dipyridinacyclou ndecaphan-3-one;

(R)-$1^{6}$,$5^{5}$-difluoro-$5^{6}$-(9-(3-methoxypropyl)-3,9-diazaspiro[5.5]undecan-3-yl)-26, 7-dimethyl-$5^{1}$H-11-oxa-4-aza-5(2,1)-benzo[d]imidazola-1(3,4),2(2,4)-dipyridinacyclo undecaphan-3-one;

$1^{6}$-fluoro-$5^{6}$-(9-(3-methoxypropyl)-3,9-diazaspiro[5.5]undecan-3-yl)-$2^{6}$-methyl-5 $^{1}$H-11-oxa-4-aza-5(2,1)-benzo[d] imidazola-1(3,4),2(2,4)-dipyridinacycloundecaphan-3-one;

3-(9-($2^{6}$-methyl-3-oxo-$5^{1}$H-11-oxa-4-aza-5(2,1)-benzo[d]imidazola-1(3,4),2(2,4) -dipyridinacycloundecaphane-$5^{6}$-yl)-3,9-diazaspiro[5.5]undecan-3-yl)propanamide;

(R)-$2^{6}$,7-dimethyl-$5^{6}$-(9-methyl-3,9-diazaspiro[5.5]undecan-3-yl)-$5^{5}$-((2-(methyls ulfonyl)ethyl)amino)-$5^{1}$H-11-oxa-4-aza-5(2,1)-benzo[d]imidazola-1(3,4),2(2,4)-dipyri dinacycloundecaphan-3-one;

(R)-$2^{6}$,7-dimethyl-$5^{6}$-(9-methyl-3,9-diazaspiro[5.5]undecan-3-yl)-$5^{5}$-(methylami no)-$5^{1}$H-11-oxa-4-aza-5(2,1)-benzo[d]imidazola-1(3,4),2(2,4)-dipyridinacycloundeca phan-3-one;

(R)-7-methyl-$2^{6}$-(methyl-$d_{3}$)-$5^{6}$-(9-(oxetan-3-yl)-3,9-diazaspiro[5.5]undecan-3-yl )-$5^{1}$H-11-oxa-4-aza-5(2,1)-benzo [d]imidazola-1(3,4),2(2,4)-dipyridinacycloundecaph an-3-one;

(R)-$1^{6}$-fluoro-$5^{6}$-(9-(2-methoxyethyl)-3,9-diazaspiro[5.5]undecan-3-yl)-$2^{6}$,7-dim ethyl-$5^{1}$H-11-oxa-4-aza-5(2,1)-benzo[d]imidazola-1(3,4),2(2,4)-dipyridinacycloundec aphan-3-one;

$1^{6}$,$2^{6}$-dimethyl-$5^{6}$-(9-(oxetan-3-yl)-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

$1^{2}$,$2^{6}$-dimethyl-$5^{6}$-(9-(oxetan-3-yl)-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

$1^{2}$-fluoro-$2^{6}$-methyl-$5^{6}$-(9-(oxetan-3-yl)-3,9-diazaspiro[5.5]undecan-3-yl)-$5^{1}$H-11 -oxa-4-aza-5(2,1)-benzo[d]imidazola-1(3,4),2(2,4)-dipyridinacycloundecaphan-3-one;

$1^{2}$-fluoro-$1^{5}$,$2^{6}$-dimethyl-$5^{6}$-(9-(oxetan-3-yl)-3,9-diazaspiro[5.5]undecan-3-yl)-$5^{1}$ H-11-oxa-4-aza-5(2,1)-benzo[d] imidazola-1(3,4),2(2,4)-dipyridinacycloundecaphan-3 -one;

$2^{6}$-methyl-$5^{6}$-(9-(oxetan-3-yl)-3,9-diazaspiro[5.5]undecan-3-yl)-$1^{6}$-(trifluorometh yl)-$5^{1}$H-11-oxa-4-aza-5(2,1)-benzo[d]imidazola-1(3,4),2(2,4)-dipyridinacycloundecap han-3-one;

$1^{5}$,$2^{6}$-dimethyl-$5^{6}$-(9-(oxetan-3-yl)-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

$1^{6}$-amino-$2^{6}$-methyl-$5^{6}$-(9-(oxetan-3-yl)-3,9-diazaspiro[5.5]undecan-3-yl)-$5^{1}$H-11 -oxa-4-aza-5(2,1)-benzo[d]imidazola-1(3,4),2(2,4)-dipyridinacycloundecaphan-3-one;

$1^{6}$-fluoro-$2^{6}$-methyl-$5^{6}$-(9-(oxetan-3-yl)-3,9-diazaspiro[5.5]undecan-3-yl)-$5^{1}$H-11 -oxa-4-aza-5(2,1)-benzo[d]imidazola-1(3,4),2(2,4)-dipyridinacycloundecaphan-3-one;

$1^{6}$-chloro-$2^{6}$-methyl-$5^{6}$-(9-(oxetan-3-yl)-3,9-diazaspiro[5.5]undecan-3-yl)-$5^{1}$H-1 1-oxa-4-aza-5(2,1)-benzo[d]imidazola-1(3,4),2(2,4)-dipyridinacycloundecaphan-3-on e;

$1^{5}$-fluoro-$2^{6}$-methyl-$5^{6}$-(9-(oxetan-3-yl)-3,9-diazaspiro[5.5]undecan-3-yl)-$5^{1}$H-11 -oxa-4-aza-5(2,1)-benzo[d]imidazola-1(3,4),2(2,4)-dipyridinacycloundecaphan-3-one;

$1^{6}$-methoxy-$2^{6}$-methyl-$5^{6}$-(9-(oxetan-3-yl)-3,9-diazaspiro[5.5]undecan-3-yl)-$5^{1}$H -11-oxa-4-aza-5(2,1)-benzo[d]imidazola-1(3,4),2(2,4)-dipyridinacycloundecaphan-3-o ne;

$1^{5}$-methoxy-$2^{6}$-methyl-$5^{6}$-(9-(oxetan-3-yl)-3,9-diazaspiro[5.5]undecan-3-yl)-$5^{1}$H -11-oxa-4-aza-5(2,1)-benzo[d]imidazola-1(3,4),2(2,4)-dipyridinacycloundecaphan-3-o ne;

$2^{6}$-methyl-$5^{6}$-(9-(oxetan-3-yl)-3,9-diazaspiro[5.5]undecan-3-yl)-3-oxo-$5^{1}$H-11-ox a-4-aza-5(2,1)-benzo[d]imidazola-1(3,4),2(2,4)-dipyridinacycloundecaphane-$1^{2}$-carbo nitrile;

$2^{6}$-methyl-$5^{6}$-(9-(oxetan-3-yl)-3,9-diazaspiro[5.5]undecan-3-yl)-3-oxo-$5^{1}$H-11-ox a-4-aza-5(2,1)-benzo[d]imidazola-1(3,4),2(2,4)-dipyridinacycloundecaphane-$1^{6}$-carbo nitrile;

$2^{6}$-methyl-$5^{6}$-(9-(oxetan-3-yl)-3,9-diazaspiro[5.5]undecan-3-yl)-3-oxo-$5^{1}$H-11-ox a-4-aza-5(2,1)-benzo[d]imidazola-1(3,4),2(2,4)-dipyridinacycloundecaphane-$1^{5}$-carbo nitrile;

$2^{6}$-methyl-$5^{6}$-(9-(oxetan-3-yl)-3,9-diazaspiro[5.5]undecan-3-yl)-$1^{5}$-(oxetan-3-ylo xy)-$5^{1}$H-11-oxa-4-aza-5(2,1)-benzo[d]imidazola-1(3,4),2(2,4)-dipyridinacycloundeca phan-3-one;

$1^{5}$-fluoro-$1^{6}$-methoxy-$2^{6}$-methyl-$5^{6}$-(9-(oxetan-3-yl)-3,9-diazaspiro[5.5]undecan-3-yl)-$5^{1}$H-11-oxa-4-aza-5(2,1)-benzo[d]imidazola-1(3,4),2(2,4)-dipyridinacycloundec aphan-3-one;

(R)-$5^{6}$-(9-(3-methoxycyclobutyl)-3,9-diazaspiro[5.5]undecan-3-yl)-$2^{6}$,7-dimethyl -$5^{1}$H-11-oxa-4-aza-5(2,1)-benzo [d]imidazola-1(3,4),2(2,4)-dipyridinacycloundecapha n-3-one;

(R)-$2^{6}$,7-dimethyl-$5^{6}$-(9-(tetrahydro-2H-pyran-4-yl)-3,9-diazaspiro[5.5]undecan-3-yl)-$5^{1}$H-11-oxa-4-aza-5(2,1)-benzo[d]imidazola-1(3,4),2(2,4)-dipyridinacycloundec aphan-3-one;

(R)-$5^6$-(9-(2-fluoroethyl)-3,9-diazaspiro[5.5]undecan-3-yl)-$2^6$,7-dimethyl-51H-1 1-oxa-4-aza-5(2,1)-benzo[d]imidazola-1(3,4),2(2,4)-dipyridinacycloundecaphan-3-on e;
(R)-$5^6$-(9-(3-fluorocyclobutyl)-3,9-diazaspiro[5.5]undecan-3-yl)-$2^6$,7-dimethyl-$5^1$H-11-oxa-4-aza-5(2,1)-benzo[d]imidazola-1(3,4),2(2,4)-dipyridinacycloundecaphan-3-one;
(R)-$5^6$-(9-(4-methoxycyclohexyl)-3,9-diazaspiro[5.5]undecan-3-yl)-$2^6$,7-dimethy 1-$5^1$H-11-oxa-4-aza-5(2,1)-benzo[d]imidazola-1(3,4),2(2,4)-dipyridinacycloundecapha n-3-one;
(R)-$5^6$-(9-cyclopropyl-3,9-diazaspiro[5.5]undecan-3-yl)-$2^6$,7-dimethyl-$5^1$H-11-o xa-4-aza-5(2,1)-benzo[d]imidazola-1(3,4),2(2,4)-dipyridinacycloundecaphan-3-one;
(R)-$5^5$-fluoro-$5^6$-(9-(3-methoxycyclobutyl)-3,9-diazaspiro[5.5]undecan-3-yl)-$2^6$,7 -dimethyl-$5^1$H-11-oxa-4-aza-5(2,1)-benzo[d]imidazola-1(3,4),2(2,4)-dipyridinacyclou ndecaphan-3-one;
(R)-$5^5$-fluoro-$5^6$-(9-(2-fluoroethyl)-3,9-diazaspiro[5.5]undecan-3-yl)-$2^6$,7-dimeth yl-$5^1$H-11-oxa-4-aza-5(2,1)-benzo[d]imidazola-1(3,4),2(2,4)-dipyridinacycloundecap han-3-one;
(R)-$5^5$-fluoro-$2^6$,7-dimethyl-$5^6$-(9-(tetrahydro-2H-pyran-4-yl)-3,9-diazaspiro[5.5] undecan-3-yl)-$5^1$H-11-oxa-4-aza-5(2,1)-benzo[d]imidazola-1(3,4),2(2,4)-dipyridinacy cloundecaphan-3-one;
(R)-$5^5$-fluoro-$5^6$-(9-(4-methoxycyclohexyl)-3,9-diazaspiro[5.5]undecan-3-yl)-$2^6$, 7-dimethyl-$5^1$H-11-oxa-4-aza-5(2,1)-benzo[d]imidazola-1(3,4),2(2,4)-dipyridinacyclo undecaphan-3-one;
(R)-$5^6$-(9-cyclopropyl-3,9-diazaspiro[5.5]undecan-3-yl)-$5^5$-fluoro-$2^6$,7-dimethyl-$5^1$H-11-oxa-4-aza-5(2,1)-benzo[d]imidazola-1(3,4),2(2,4)-dipyridinacycloundecaphan -3-one;
(R)-$5^6$-(9-(3-methoxycyclobutyl)-3,9-diazaspiro[5.5]undecan-3-yl)-$2^6$,$5^5$,7-trimet hyl-$5^1$H-11-oxa-4-aza-5(2,1)-benzo[d]imidazola-1(3,4),2(2,4)-dipyridinacycloundeca phan-3-one;
$2^6$-methyl-$5^6$-(9-(oxetan-3-yl)-3,9-diazaspiro[5.5]undecan-3-yl)-$5^1$H-11-oxa-4-az a-5(2,1)-benzo[d]imidazola-1(3,4),2(2,4)-dipyridinacycloundecaphan-3-one;
$2^6$-methyl-$5^6$-(9-(oxetan-3-yl)-3,9-diazaspiro[5.5]undecan-3-yl)-$5^1$H-10-oxa-4-az a-5(2,1)-benzo[d]imidazola-1(3,4),2(2,4)-dipyridinacyclodecaphan-3-one;
$5^6$-(9-(3-methoxycyclobutyl)-3,9-diazaspiro[5.5]undecan-3-yl)-$2^6$-methyl-$5^1$H-11 -oxa-4-aza-5(2,1)-benzo[d]imidazola-1(3,4),2(2,4)-dipyridinacycloundecaphan-3-one;
$5^6$-(9-(3-fluorocyclobutyl)-3,9-diazaspiro[5.5]undecan-3-yl)-$2^6$,$5^5$-dimethyl-$5^1$H-11-oxa-4-aza-5(2,1)-benzo[d]imidazola-1(3,4),2(2,4)-dipyridinacycloundecaphan-3-o ne;
$5^6$-(9-cyclopropyl-3,9-diazaspiro[5.5]undecan-3-yl)-$2^6$,$5^5$-dimethyl-$5^1$H-11-oxa-4 -aza-5(2,1)-benzo[d]imidazola-1(3,4),2(2,4)-dipyridinacycloundecaphan-3-one;
$5^6$-(9-(3-methoxycyclobutyl)-3,9-diazaspiro[5.5]undecan-3-yl)-$2^6$,$5^5$-dimethyl-$5^1$ H-11-oxa-4-aza-5(2,1)-benzo[d]imidazola-1(3,4),2(2,4)-dipyridinacycloundecaphan-3 -one;
$2^6$,$5^5$-dimethyl-$5^6$-(9-(oxetan-3-ylmethyl)-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-o ne;
$5^6$-(9-(2-fluoroethyl)-3,9-diazaspiro[5.5]undecan-3-yl)-$2^6$,$5^5$-dimethyl-$5^1$H-11-ox a-4-aza-5(2,1)-benzo[d]imidazola-1(3,4),2(2,4)-dipyridinacycloundecaphan-3-one;
$5^6$-(9-(2-fluoroethyl)-3,9-diazaspiro[5.5]undecan-3-yl)-$2^6$,$5^5$-dimethyl-$5^1$H-12-ox a-4-aza-5(2,1)-benzo[d]imidazola-1(3,4),2(2,4)-dipyridinacyclododecaphan-3-one;
$5^5$-fluoro-$5^6$-(9-(2-fluoroethyl)-3,9-diazaspiro[5.5]undecan-3-yl)-$2^6$-methyl-$5^1$H-11-oxa-4-aza-5(2,1)-benzo[d]imidazola-1(3,4),2(2,4)-dipyridinacycloundecaphan-3-o ne;
$5^5$-fluoro-$2^6$-methyl-$5^6$-(9-(oxetan-3-ylmethyl)-3,9-diazaspiro[5.5]undecan-3-yl)-$5^1$H-11-oxa-4-aza-5(2,1)-benzo[d]imidazola-1(3,4),2(2,4)-dipyridinacycloundecaphan -3-one;
$5^5$-fluoro-$2^6$-methyl-$5^6$-(9-(oxetan-3-yl)-3,9-diazaspiro[5.5]undecan-3-yl)-$5^1$H-11 -oxa-4-aza-5(2,1)-benzo[d]imidazola-1(3,4),2(2,4)-dipyridinacycloundecaphan-3-one;
$5^5$-fluoro-$5^6$-(9-(3-fluorocyclobutyl)-3,9-diazaspiro[5.5]undecan-3-yl)-$2^6$-methyl-$5^1$H-11-oxa-4-aza-5(2,1)-benzo[d]imidazola-1(3,4),2(2,4)-dipyridinacycloundecaphan -3-one;
$5^6$-(9-cyclopropyl-3,9-diazaspiro[5.5]undecan-3-yl)-$5^5$-fluoro-$2^6$-methyl-$5^1$H-11-oxa-4-aza-5(2,1)-benzo[d]imidazola-1(3,4),2(2,4)-dipyridinacycloundecaphan-3-one;
(R)-$2^6$,7-bis(methyl-$d_3$)-$5^6$-(9-(methyl-$d_3$)-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-o ne;
$2^6$-methyl-$5^6$-(9-(methyl-$d_3$)-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-one;
(R)-$5^5$-fluoro-$2^6$,7-dimethyl-$5^6$-(9-(methyl-$d_3$)-3,9-diazaspiro[5.5]undecan-3-yl)-$5^1$H-11-oxa-4-aza-5(2,1)-benzo[d]imidazola-1(3,4),2(2,4)-dipyridinacycloundecaphan -3-one;
$5^5$-fluoro-$2^6$-methyl-$5^6$-(9-(methyl-$d_3$)-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-one;
$2^6$,$5^5$-dimethyl-$5^6$-(9-(methyl-$d_3$)-3,9-diazaspiro[5.5]undecan-3-yl)-$5^1$H-11-oxa-4 -aza-5(2,1)-benzo[d]imidazola-1(3,4),2(2,4)-dipyridinacycloundecaphan-3-one;

(R)-$2^{6}$,$5^{5}$,7-trimethyl-$5^{6}$-(9-(methyl-$d_{3}$)-3,9-diazaspiro[5.5]undecan-3-yl)-$5^{1}$H-11 -oxa-4-aza-5(2,1)-benzo[d]imidazola-1(3,4),2(2,4)-dipyridinacycloundecaphan-3-one;
$2^{6}$-methyl-$5^{6}$-(3-methyl-3-azaspiro[5.5]undecan-9-yl)-$5^{1}$H-4-aza-5(2,1)-benzo[d] imidazola-1(3,4),2(2,4)-dipyridinacycloundecaphan-3-one;
$5^{6}$-(9-(2-methoxyethyl)-3,9-diazaspiro[5.5]undecan-3-yl)-$2^{6}$-methyl-$5^{1}$H-4-aza-5 (2,1)-benzo[d]imidazola-1(3,4),2(2,4)-dipyridinacycloundecaphan-3-one;
(R)-$5^{6}$-(9-(3-methoxypropyl)-3,9-diazaspiro[5.5]undecan-3-yl)-$2^{6}$,7-dimethyl-$5^{1}$ H-4-aza-5(2,1)-benzo[d]imidazola-1(3,4),2(2,4)-dipyridinacycloundecaphan-3-one;
$5^{6}$-(9-(2-methoxyethyl)-3,9-diazaspiro[5.5]undecan-3-yl)-$2^{6}$-methyl-$5^{1}$H-11-oxa-4-aza-5(2,1)-benzo[d]imidazola-1(3,4),2(2,4)-dipyridinacycloundecaphan-3-one;
$5^{6}$-(9-(3-methoxypropyl)-3,9-diazaspiro[5.5]undecan-3-yl)-$2^{6}$-methyl-$5^{1}$H-11-ox a-4-aza-5(2,1)-benzo[d]imidazola-1(3,4),2(2,4)-dipyridinacycloundecaphan-3-one;
(R)-$5^{6}$-(9-(3-methoxypropyl)-3,9-diazaspiro[5.5]undecan-3-yl)-$2^{6}$,7-dimethyl-$5^{1}$ H-11-oxa-4-aza-5(2,1)-benzo[d] imidazola-1(3,4),2(2,4)-dipyridinacycloundecaphan-3 -one;
$5^{6}$-(9-(2-fluoro-2-methylpropyl)-3,9-diazaspiro[5.5]undecan-3-yl)-$2^{6}$-methyl-$5^{1}$H -11-oxa-4-aza-5(2,1)-benzo[d]imidazola-1(3,4),2(2,4)-dipyridinacycloundecaphan-3-o ne;
(R)-$5^{5}$-amino-$2^{6}$,7-dimethyl-$5^{6}$-(9-methyl-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-o ne;
(R)-$1^{6}$-fluoro-$5^{6}$-(9-(2-fluoroethyl)-3,9-diazaspiro[5.5]undecan-3-yl)-$2^{6}$,7-dimeth yl-$5^{1}$H-11-oxa-4-aza-5(2,1)-benzo [d]imidazola-1(3,4),2(2,4)-dipyridinacycloundecap han-3-one;
(R)-$1^{6}$-fluoro-$2^{6}$,7-dimethyl-$5^{6}$-(9-(oxetan-3-yl)-3,9-diazaspiro[5.5]undecan-3-yl) -$5^{1}$H-11-oxa-4-aza-5(2,1)-benzo [d]imidazola-1(3,4),2(2,4)-dipyridinacycloundecapha n-3-one
(7R)-$5^{6}$-(9-(2,2-dimethyloxetan-3-yl)-3,9-diazaspiro[5.5]undecan-3-yl)-$2^{6}$,7-dim ethyl-$5^{1}$H-11-oxa-4-aza-5(2,1)-benzo[d]imidazola-1(3,4),2(2,4)-dipyridinacycloundec aphan-3-one;
(7R)-$2^{6}$,7-dimethyl-$5^{6}$-(9-(2-methyloxetan-3-yl)-3,9-diazaspiro[5.5]undecan-3-yl )-$5^{1}$H-11-oxa-4-aza-5(2,1)-benzo [d]imidazola-1(3,4),2(2,4)-dipyridinacycloundecaph an-3-one;
(R)-$2^{6}$,7-dimethyl-$5^{6}$-(9-(1-methylazetidin-3-yl)-3,9-diazaspiro[5.5]undecan-3-yl )-$5^{1}$H-11-oxa-4-aza-5(2,1)-benzo [d]imidazola-1(3,4),2(2,4)-dipyridinacycloundecaph an-3-one;
(R)-$2^{6}$,7-dimethyl-$5^{6}$-(9-(3-methyloxetan-3-yl)-3,9-diazaspiro[5.5]undecan-3-yl)-$5^{1}$H-11-oxa-4-aza-5(2,1)-benzo[d] imidazola-1(3,4),2(2,4)-dipyridinacycloundecaphan -3-one;
(R)-$5^{5}$-fluoro-$5^{6}$-(9-(3-methoxypropyl)-3,9-diazaspiro[5.5]undecan-3-yl)-$2^{6}$,7-di methyl-$5^{1}$H-11-oxa-4-aza-5(2,1)-benzo[d]imidazola-1(3,4),2(2,4)-dipyridinacyclound ecaphan-3-one;
$5^{6}$-(9-(2-(dimethylamino)ethyl)-3,9-diazaspiro[5.5]undecan-3-yl)-$2^{6}$-methyl-$5^{1}$H-11-oxa-4-aza-5(2,1)-benzo[d]imidazola-1(3,4),2(2,4)-dipyridinacycloundecaphan-3-o ne;
(7R)-$5^{6}$-(9-(4-methoxy-4-methylpentan-2-yl)-3,9-diazaspiro[5.5]undecan-3-yl)-2 $^{6}$,7-dimethyl-$5^{1}$H-11-oxa-4-aza-5(2,1)-benzo[d]imidazola-1(3,4),2(2,4)-dipyridinacycl oundecaphan-3-one;
$5^{6}$-(9-cyclopropyl-3,9-diazaspiro[5.5]undecan-3-yl)-$2^{6}$-methyl-$5^{1}$H-11-oxa-4-aza -5(2,1)-benzo[d]imidazola-1(3,4),2(2,4)-dipyridinacycloundecaphan-3-one;
$5^{6}$-(9-(4-methoxy-4-methylpentan-2-yl)-3,9-diazaspiro[5.5]undecan-3-yl)-$2^{6}$-met hyl-$5^{1}$H-11-oxa-4-aza-5(2,1)-benzo[d]imidazola-1(3,4),2(2,4)-dipyridinacycloundeca phan-3-one;
$5^{6}$-(9-((2R,6S)-2,6-dimethyltetrahydro-2H-pyran-4-yl)-3,9-diazaspiro[5.5]undeca n-3-yl)-$2^{6}$-methyl-$5^{1}$H-11-oxa-4-aza-5(2,1)-benzo[d]imidazola-1(3,4),2(2,4)-dipyridin acycloundecaphan-3-one;
$5^{5}$-chloro-$2^{6}$-methyl-$5^{6}$-(9-methyl-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;
(R)-$5^{5}$-chloro-$2^{6}$,7-dimethyl-$5^{6}$-(9-methyl-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-o ne;
6'-methyl-6'-(9-methyl-3,9-diazaspiro[5.5]undecan-3-yl)spiro[cyclopropane-1,7'-11-oxa-4-aza-5(2,1)-benzo[d]imidazola-1(3,4),2(2,4)-dipyridinacycloundecaphan]-3'-one;
$2^{6}$,8,8-trimethyl-$5^{6}$-(9-methyl-3,9-diazaspiro[5.5]undecan-3-yl)-$5^{1}$H-11-oxa-4-az a-5(2,1)-benzo[d]imidazola-1(3,4),2(2,4)-dipyridinacycloundecaphan-3-one;
(R)-3-(9-($2^{6}$,7-dimethyl-3-oxo-$5^{1}$H-11-oxa-4-aza-5(2,1)-benzo[d]imidazola-1(3,4 ),2(2,4)-dipyridinacycloundecaphane-56-yl)-3,9-diazaspiro[5.5]undecan-3-yl)propana mide;
(R)-$2^{6}$,7-dimethyl-$5^{6}$-(9-methyl-3,9-diazaspiro[5.5]undecan-3-yl)-$5^{5}$-(oxetan-3-yl amino)-$5^{1}$H-11-oxa-4-aza-5(2,1)-benzo[d]imidazola-1(3,4),2(2,4)-dipyridinacyclound ecaphan-3-one;
(R)-2-(9-($1^{6}$-fluoro-$2^{6}$,7-dimethyl-3-oxo-$5^{1}$H-11-oxa-4-aza-5(2,1)-benzo[d]imida zola-1(3,4),2(2,4)-dipyridinacycloundecaphane-$5^{6}$-yl)-3,9-diazaspiro[5.5]undecan-3-y 1)ethyl methylcarbamate;
(R)-3-(9-($2^{6}$,7-dimethyl-3-oxo-$5^{1}$H-11-oxa-4-aza-5(2,1)-benzo[d]imidazola-1(3,4 ),2(2,4)-dipyridinacycloundecaphane-$5^{6}$-yl)-3,9-diazaspiro[5.5]undecan-3-yl)-N,N-di methylpropanamide;

(R)-3-(9-($1^6$,$5^5$-difluoro-$2^6$,7-dimethyl-3-oxo-$5^1$H-11-oxa-4-aza-5(2,1)-benzo[d]i midazola-1(3,4),2(2,4)-dipyridinacycloundecaphane-$5^6$-yl)-3,9-diazaspiro[5.5]undeca n-3-yl)propanamide;
(R)-$1^6$,$5^5$-difluoro-$5^6$-(9-(2-methoxyethyl)-3,9-diazaspiro[5.5]undecan-3-yl)-$2^6$,7-dimethyl-$5^1$H-11-oxa-4-aza-5(2,1)-benzo[d]imidazola-1(3,4),2(2,4)-dipyridinacycloun decaphan-3-one;
(R)-3-(9-($1^6$-fluoro-$2^6$,7-dimethyl-3-oxo-$5^1$H-11-oxa-4-aza-5(2,1)-benzo[d]imida zola-1(3,4),2(2,4)-dipyridinacycloundecaphane-$5^6$-yl)-3,9-diazaspiro[5.5]undecan-3-y l)-N,N-dimethylpropanamide;
(R)-$2^6$,7-dimethyl-$5^6$-(9-(oxetan-3-yl)-3,9-diazaspiro[5.5]undecan-3-yl)-3-oxo-$5^1$ H-11-oxa-4-aza-5(2,1)-benzo[d]imidazola-1(3,4),2(2,4)-dipyridinacycloundecaphane-$1^6$-carbonitrile;
(R)-$5^6$-(9-(3-(azetidin-1-yl)-3-oxopropyl)-3,9-diazaspiro[5.5]undecan-3-yl)-$2^6$,7-dimethyl-$5^1$H-11-oxa-4-aza-5(2,1)-benzo[d]imidazola-1(3,4),2(2,4)-dipyridinacycloun decaphan-3-one;
(R)-$5^6$-(9-(3-(azetidin-1-yl)-3-oxopropyl)-3,9-diazaspiro[5.5]undecan-3-yl)-$1^6$-fl uoro-$2^6$,7-dimethyl-$5^1$H-11-oxa-4-aza-5(2,1)-benzo[d]imidazola-1(3,4),2(2,4)-dipyridi nacycloundecaphan-3-one;
(R)-$2^6$,7-dimethyl-$5^6$-(9-(2-(trifluoromethoxy)ethyl)-3,9-diazaspiro[5.5]undecan-3-yl)-$5^1$H-11-oxa-4-aza-5(2,1)-benzo[d]imidazola-1(3,4),2(2,4)-dipyridinacycloundec aphan-3-one;
(R)-$1^6$-fluoro-$2^6$,7-dimethyl-$5^6$-(9-(2-(trifluoromethoxy)ethyl)-3,9-diazaspiro[5.5 ]undecan-3-yl)-$5^1$H-11-oxa-4-aza-5(2,1)-benzo[d]imidazola-1(3,4),2(2,4)-dipyridinac ycloundecaphan-3-one;
(R)-$5^6$-(9-(3-(3,3-dimethylazetidin-1-yl)-3-oxopropyl)-3,9-diazaspiro[5.5]undeca n-3-yl)-$2^6$,7-dimethyl-$5^1$H-11-oxa-4-aza-5(2,1)-benzo[d]imidazola-1(3,4),2(2,4)-dipyr idinacycloundecaphan-3-one; or
(R)-2-(9-($2^6$,7-dimethyl-3-oxo-$5^1$H-11-oxa-4-aza-5(2,1)-benzo[d]imidazola-1(3,4 ),2(2,4)-dipyridinacycloundecaphane-$5^6$-yl)-3,9-diazaspiro[5.5]undecan-3-yl)ethyl carbamate.

A pharmaceutical composition, wherein the pharmaceutical composition comprises a therapeutically effective amount of at least one compound of fomula (I), or a stereoisomer, tautomer, solvate, pharmaceutically acceptable salt or deuterated derivative thereof and at least one pharmaceutically acceptable excipient.
The present invention also provides the use of the above-mentioned compound and/or pharmaceutical composition in the preparation of an anti-tumor drug. In some embodiments, the anti-tumor drug is used for the following diseases: head and neck cancer, melanoma, bladder cancer, esophageal cancer, anaplastic large cell lymphoma, renal cell carcinoma, breast cancer, colorectal cancer, ovarian cancer, cervical cancer, pancreatic cancer, glioma, glioblastoma, prostate cancer, leukemia, lymphoma, non-Hodgkin's lymphoma, gastric cancer, lung cancer, hepatocellular carcinoma, gastrointestinal stromal tumor, thyroid cancer, bile duct cancer, endometrial cancer, multiple myeloma or mesothelioma.
In some embodiments, the tumor is malignant tumor carrying EGFR gene mutation; preferably, the EGFR gene mutation is selected from the group consisting of EGFR Del19 gene mutation, EGFR L858R gene mutation, EGFR T790M gene mutation, an EGFR C797S gene mutation, or a combination thereof.
The present invention also provides a method for treating tumor in a patient in need thereof, comprising administering to the patient a therapeutically effective amount of the above compound or pharmaceutical composition, wherein the patient is preferably a mammal, and the mammal is preferably a human.
In some embodiments, administration during treatment includes oral, mucosal, sublingual, ocular, topical, parenteral, rectal, intracisternal, vaginal, peritoneal, bladder, and nasal administration.
In some embodiments, the tumor comprises: head and neck cancer, melanoma, bladder cancer, esophageal cancer, anaplastic large cell lymphoma, renal cell carcinoma, breast cancer, colorectal cancer, ovarian cancer, cervical cancer, pancreatic cancer, glioma, glioblastoma, prostate cancer, leukemia, lymphoma, non-Hodgkin lymphoma, gastric cancer, lung cancer, hepatocellular carcinoma, gastrointestinal stromal tumor, thyroid cancer, bile duct cancer, endometrial cancer, multiple myeloma or mesothelioma.
In some embodiments, the tumor is a malignant tumor carrying an EGFR gene mutation; preferably, the EGFR gene mutation is selected from the group consisting of an EGFR Del19 gene mutation, an EGFR L858R gene mutation, an EGFR T790M gene mutation, an EGFR C797S gene mutation, or a combination thereof.

**Definition and Description**

The general chemical terms used in the formula above have their usual meanings.
For example, unless otherwise specified, the terms "halo" and "halogen" as used herein refer to fluorine, chlorine, bromine or iodine. Preferred halogen includes F, Cl and Br.
In the present invention, unless otherwise specified, the term "alkyl" includes monovalent saturated alkyl groups, whether straight or branched. The alkyl groups used herein can be optionally substituted with one or more substituents. Non limiting examples of alkyl groups include methyl, ethyl, propyl, isopropyl, n-butyl, isobutyl, sec butyl, tert butyl, n-pentyl, 3- (2-methyl) butyl, 2-pentyl, 2-methylbutyl, neopentyl, n-hexyl, 2-hexyl, 2-methylpentyl, etc. Similarly, the term '$C_{1-6}$ alkyls' refers to straight or branched chain groups containing 1, 2, 3, 4, 5, or 6 carbon atoms.
The term "alkenyl" and "alkynyl" include straight or branched alkenyl and alkynyl. Similarly, "$C_{2-6}$ alkenyl" and

"$C_{2-6}$ alkynyl" refer to alkenyl or alkynyl containing 2, 3, 4, 5, 6 carbon atoms arranged in a straight or branched form.

The term "alkoxy" refers to the oxygen ether form of the aforementioned straight-chain or branched-chain alkyl groups.

The term "haloalkyl" means that the aforementioned "alkyl" is substituted with one or more halogens.

**In** the present invention, "a", "an", "the", "at least one" and "one or more" are used interchangeably. Thus, for example, a composition comprising "a" pharmaceutically acceptable excipient can be interpreted as indicating that the composition includes "one or more" pharmaceutically acceptable excipients.

The term "aryl", in the present invention, unless otherwise specified, refers to an unsubstituted or substituted monocyclic, fused or condensed aromatic group comprising carbon atoms, the aryl is preferably a $C_{6-14}$ aryl, and the $C_{6-14}$ aryl is further preferably a $C_{6-10}$ aryl. Examples of these aromatic rings include, but are not limited to, phenyl and naphthyl.

The term "heteroaryl" refers to a monocyclic or polycyclic (e.g., fused bicyclic) aromatic heterocycle having at least one heteroatom ring member (e.g., 1 to 4 heteroatoms, or preferably 1 to 3 heteroatoms), wherein the heteroatom is one or more selected from the group consisting of N, O and S. And wherein the nitrogen or sulfur heteroatom may be optionally oxidized, and the nitrogen heteroatom may be optionally quaternized. The heteroaryl group may be attached to any heteroatom or carbon atom to produce a stable structure. The heteroaryl group is preferably a 5-14-membered heteroaryl group, and the 5-14-membered heteroaryl group is further preferably a 5-10-membered heteroaryl group or a 5-6-membered heteroaryl group. Examples of heteroaryl groups include, but are not limited to, thienyl, furanyl, imidazolyl, isoxazolyl, oxazolyl, pyrazolyl, pyrrolyl, thiazolyl, thiadiazolyl, triazolyl, pyridinyl, pyridazinyl, indolyl, azaindolyl, indazolyl, benzimidazolyl, benzofuranyl, benzothienyl, benzisoxazolyl, benzoxazolyl, benzopyrazolyl, benzothiazolyl, benzothiadiazolyl, benzotriazolyladeninyl, quinolinyl, or isoquinolinyl.

The term "carbocyclyl" refers to a saturated or unsaturated cyclic group that is not aromatic. Depending on the degree of saturation, it may include "cycloalkyl", "cycloalkenyl" or "cycloalkynyl". Monocyclic carbocyclyl groups include, but are not limited to, cyclopropane, cyclobutane, cyclopentane, cyclohexane or cyclohexene and the like; polycyclic carbocyclyl groups include spirocyclic, condensed ring and bridged ring carbocyclyls. The carbocyclyl is preferably a $C_{3-14}$ carbocyclyl, and the $C_{3-14}$ carbocyclyl is further preferably a $C_{3-8}$ carbocyclyl, and the $C_{3-8}$ carbocyclyl is further preferably a $C_{3-6}$ carbocyclyl, and the $C_{3-8}$ carbocyclyl is further preferably a $C_{5-6}$ carbocyclyl.

The term "cycloalkyl" refers to a saturated monocyclic and polycyclic system containing only carbon atoms in the ring, and may optionally be substituted with one to more substituents." cycloalkyl" may have a ring system including a bridged ring, a condensed ring, and a spiro ring. Non-limiting examples of cycloalkyl include, for example, cyclopropyl, cyclobutyl, cyclopentyl, cyclohexyl, spiro [3.4] octyl, bicyclo [2.2.1] heptane, etc. The cycloalkyl is preferably $C_{3-14}$ cycloalkyl, the $C_{3-14}$ cycloalkyl is further preferably $C_{3-8}$ cycloalkyl, the $C_{3-8}$ cycloalkyl is further preferably $C_{3-6}$ cycloalkyl, and the $C_{3-8}$ cycloalkyl is further preferably $C_{5-6}$ cycloalkyl.

The term " heterocyclyl", in the present invention, unless otherwise specified, refers to an unsubstituted or substituted monocyclic and polycyclic system consisting of carbon atoms and 1-3 heteroatoms selected from the group consisting of N, O and S, and includes saturated or unsaturated cyclic systems and polycyclic systems with unsaturated parts and/or aromatic parts. Wherein the nitrogen or sulfur heteroatom can be selectively oxidized, and the nitrogen heteroatom can be selectively quaternized. The heterocyclic group can be connected to any heteroatom or carbon atom to form a stable structure. It should be understood that the polycyclic heterocycloalkyl group can have a ring system including fused rings, bridged rings and spiro rings. The heterocyclic group is preferably a 3-14-membered heterocyclic group, and the 3-14-membered heterocyclic group is further preferably a 3-8-membered heterocyclic group or a 5-10-membered heterocyclic group, and the 3-8-membered heterocyclic group is further preferably a 3-6-membered heterocyclic group, and the 3-6-membered heterocyclic group is further preferably a 5-6-membered heterocyclic group. The heterocycloalkyl group used herein can be optionally substituted with one or more substituents. Examples of such heterocyclic groups include, but are not limited to, azetidinyl, pyrrolidinyl, piperidinyl, piperazinyl, oxopiperazinyl, oxopiperidinyl, oxetane, tetrahydrofuranyl, dioxolanyl, tetrahydroimidazolyl, tetrahydrothiazolyl, tetrahydrooxazolyl, tetrahydropyranyl, morpholinyl, thiomorpholinyl, thiomorpholinyl sulfoxide, thiomorpholinyl sulfone, and tetrahydrooxadiazolyl.

However, in no case will heterocyclyl and carbocyclyl groups cross or contain each other. Therefore, according to the above definition, if at least one all-carbocyclic ring is fused with a heterocyclyl to form a di-, poly- or spiro-ring, it will still be defined as a heterocyclyl.

In addition, if a heteroaryl group is fused with a heterocyclyl to form a di-, poly- or spiro-ring, it will be defined as a heterocyclyl group rather than a heteroaryl group.

The term "substituted" means that one or more hydrogen atoms in the group are replaced by the same or different substituents. Typical substituents include, but are not limited to, halogen (F, Cl, Br or I), $C_{1-8}$ alkyl, $C_{3-12}$ cycloalkyl, $-OR^1$, $-SR^1$, =O, =S, $-C(O)R^1$, $-C(S)R^1$, $=NR^1$, $-C(O)OR^1$, $-C(S)OR^1$, $-NR^1R^2$, $-C(O)NR^1R^2$, cyano, nitro, $-S(O)_2R^1$, $-O-S(O_2)OR^1$, $-O-S(O)_2R^1$, $-OP(O)(OR^1)(OR^2)$; wherein $R_1$ and $R^2$ are independently selected from the group consisting of -H, $C_{1-6}$ alkyl, and $C_{1-6}$ haloalkyl. In some embodiments, the substituents are independently groups selected from the group consisting of -F, -Cl, -Br, -I, methyl, ethyl, -OH, trifluoromethoxy, ethoxy, propoxy, isopropoxy, n-butoxy, isobutoxy, tert-butoxy, $-SCH_3$, $-SC_2H_5$, formaldehyde, $-C(OCH_3)$, cyano, nitro, $-CF_3$, $-OCF_3$, amino, dimethylamino, methylthio, sulfonyl,

and acetyl.

The term "pharmaceutically acceptable salt" refers to a salt prepared from a pharmaceutically acceptable base or acid. When the compound provided by the present invention is an acid, its corresponding salt can be conveniently prepared from a pharmaceutically acceptable base, including inorganic bases and organic bases.

"Deuterated" as described herein refers to a compound or chemical group structure when hydrogen atoms are partially or completely replaced by their isotope deuterium, resulting in a compound or group. Any position specifically designated as "D" or "deuterium" is understood to be a deuterium enrichment of 50%, 80%, 90%, 95%, 98% or 99%. "Deuterium enrichment" is a molar percentage determined by dividing the number of deuterium-containing compounds at the indicated position by the total number of all compounds. When a position is designated as "H" or "hydrogen", the position has hydrogen at natural abundance. When a position is silent on the presence of hydrogen or deuterium, the hydrogen at the position is at its natural abundance. A specific alternative embodiment relates to a compound of the present disclosure, which has at least 5%, 10%, 25%, 50%, 80%, 90%, 95%, 98% or 99% deuterium enrichment at one or more positions not specifically designated as "D" or "deuterium". In some embodiments, one or more hydrogen atoms of any compound described herein can be replaced with deuterium to provide the corresponding labeled or enriched compound.

The "deuterated $C_{1-6}$ alkyl" of the present invention refers to a group obtained by partially or completely replacing the hydrogen atoms in the "$C_{1-6}$ alkyl" structure with its isotope deuterium; "deuterated methyl" refers to a group obtained by partially or completely replacing the hydrogen atoms in the methyl structure with its isotope cyano. $CD_3$ is a group obtained by completely replacing the hydrogen atoms in the methyl structure with its isotope deuterium. The deuterated $C_{1-6}$ alkyl is preferably a deuterated $C_{1-3}$ alkyl; the deuterated $C_{1-3}$ alkyl is preferably a deuterated methyl.

Since the compound of formula (I) is to be used as a medicine, it is preferably used with a certain purity, for example, at least 60% purity, more preferably at least 75% purity, and particularly preferably at least 98% purity (% is weight ratio).

Prodrugs of the compounds of the present invention are included in the scope of protection of the present invention. Generally, the prodrug refers to a functional derivative that is easily converted into the desired compound in vivo. For example, any pharmaceutically acceptable salt, ester, salt of an ester or other derivative of the compound of the present application, which can directly or indirectly provide the compound of the present application or its pharmaceutically active metabolite or residue after administration to a receptor. Particularly preferred derivatives or prodrugs are those compounds that can increase the bioavailability of the compound of the present application when administered to a patient (for example, it can make the oral compound more easily absorbed into the blood), or promote the delivery of the parent compound to biological organs or sites of action (such as the brain or lymphatic system). Therefore, the term "administration" in the treatment methods provided by the present invention refers to the administration of the compounds disclosed in the present invention that can treat different diseases, or although not explicitly disclosed, it can be converted into the compounds disclosed in the present invention in vivo after administration to a subject. Conventional methods for selecting and preparing suitable prodrug derivatives have been recorded in books such as "Design of Prodrugs, ed. H. Bundgaard, Elsevier, 1985".

Obviously, the definition of any substituent or variable at a specific position in a molecule is independent of other positions in the molecule. It is easy to understand that those skilled in the art can select substituents or substitution patterns of the compounds of the present invention by means of prior art means and the methods described in the present invention to obtain chemically stable and easy to synthesize compounds.

The compounds of the present invention may contain one or more asymmetric centers and may thus produce diastereomers and optical isomers. The present invention includes all possible diastereomers and racemic mixtures thereof, their substantially pure resolved enantiomers, all possible geometric isomers and pharmaceutically acceptable salts thereof.

The above formula (I) does not precisely define the stereostructure of a certain position of the compound. The present invention includes all stereoisomers of the compound of formula (I) and pharmaceutically acceptable salts thereof. Further, mixtures of stereoisomers and isolated specific stereoisomers are also included in the present invention. In the synthetic process for preparing such compounds, or in the process of racemization or epimerization known to those skilled in the art, the obtained product may be a mixture of stereoisomers.

When the compound of formula (I) exists in tautomerism, unless otherwise stated, the present invention includes any possible tautomerism and pharmaceutically acceptable salts thereof, and mixtures thereof.

When the compound of formula (I) and its pharmaceutically acceptable salt exist in solvates or polymorphs, the present invention includes any possible solvates and polymorphs. The type of solvent forming the solvate is not particularly limited, as long as the solvent is pharmaceutically acceptable.

The term "composition", as used herein, is intended to include products comprising the specified amounts of the specified ingredients, as well as any product produced directly or indirectly by the combination of the specified amounts of the specified ingredients. Therefore, pharmaceutical compositions containing the compounds of the present invention as active ingredients and methods for preparing the compounds of the present invention are also part of the present invention.

**In** addition, some crystalline forms of the compound may exist as polymorphs, and such polymorphs are included in the present invention. **In** addition, some compounds may form solvates with water (i.e., hydrates) or common organic solvents, and such solvates also fall within the scope of the present invention.

The pharmaceutical composition provided by the present invention comprises a compound represented by formula (I) (or a pharmaceutically acceptable salt thereof) as an active ingredient, a pharmaceutically acceptable excipient and other optional therapeutic components or adjuvants. Although in any given case, the most suitable mode of administration of the active ingredient depends on the specific subject receiving the administration, the subject's properties and the severity of the disease, the pharmaceutical composition of the present invention includes a pharmaceutical composition suitable for oral, rectal, topical and parenteral (including subcutaneous administration, intramuscular injection, intravenous administration) administration. The pharmaceutical composition of the present invention can be conveniently present in a unit dosage form known in the art and prepared by any preparation method known in the pharmaceutical field.

The pharmaceutical composition of the present invention can be prepared by any pharmaceutical method. Generally, such method includes the step of associating the active ingredient with a carrier constituting one or more essential ingredients. Generally, the pharmaceutical composition is prepared by uniformly and closely mixing the active ingredient with a liquid carrier or a finely divided solid carrier or a mixture of the two. In addition, the product can be conveniently prepared into a desired appearance.

Therefore, the pharmaceutical composition of the present invention comprises a pharmaceutically acceptable carrier and a compound of formula (I) or its stereoisomers, tautomers, polymorphs, solvates, pharmaceutically acceptable salts thereof, and prodrugs thereof. The compound of formula (I) or its pharmaceutically acceptable salts, in combination with one or more other therapeutically active compounds, is also included in the pharmaceutical composition of the present invention.

The pharmaceutical carrier employed in the present invention can be, for example, a solid, liquid, or gas.

## Detailed description

In order to make the above content clearer and more specific, the present invention will further illustrate the technical scheme of the present invention with the following examples. The following examples are only used to illustrate the specific embodiments of the present invention so that those skilled in the art can understand the present invention, but are not used to limit the scope of protection of the present invention. In the specific embodiments of the present invention, the technical means or methods not specifically described are conventional technical means or methods in the art.

Unless otherwise specified, all parts and percentages of the present invention are calculated by weight, and all temperatures are in degrees Celsius.

The following abbreviations are used in the examples:

AcOH: acetic acid;
$(Boc)_2O$: Di- tert -butyl Dicarbonate;
CNBr: Cyanogen bromide;
CMBP: CynoMethylenetributyl phosphorane;
DCM: Dichloromethane;
DIEA/DIPEA: N,N- diisopropylethylamine;
Dioxane: dioxane;
DMAP: 4- dimethylaminopyridine;
DMF: N,N- dimethylformamide;
EA/EtOAc: ethyl acetate;
EtOH: ethanol;
Fe: iron powder;
HATU: O-(7-Azabenzotriazol-1-yl)-N,N,N',N'-tetraMethyluroniuM hexafluorophosphate;
LC-MS or LCMS: Liquid chromatography-mass spectrometry;
Ruphos: 2-dicyclohexylphosphino-2',6'-diisopropoxybiphenyl;
STAB: Sodium triacetoxyborohydride;
t-BuONa: Sodium tert-butoxide;
TBAF: Tetrabutylammonium fluoride;
TEA: Triethylamine;
TFA: Trifluoroacetic acid;
THF: Tetrahydrofuran;
TLC: Thin layer chromatography;
$Na_2CO_3$: sodium carbonate;

$K_2CO_3$: potassium carbonate;
$Pd(Amphos)Cl_2$:

Bis(di-tert-butyl(4-dimethylaminophenyl)phosphine)dichloropalladium(II);
$Pd_2(dba)_3$: Tris(dibenzylideneacetone)dipalladium;
Pd/C: Palladium on carbon;
PE: Petroleum ether;
Prep-HPLC: Prep High Performance Liquid Chromatography;
PLC: preparative liquid chromatography;
Xphos: 2-(Dicyclohexylphosphino)-2',4',6'-triisopropylbiphenyl.

The raw materials and reagents used in the examples can be obtained from commercial sources or prepared by conventional methods in this field.

Example 1 Synthesis of Compound 1

*$2^6$-methyl-$5^6$-(9-methyl-3,9-diazaspiro[5.5]undecan-3-yl)-$5^1$H-11-oxa-4-aza-5(2,1)-be nzo[d]imidazola-1(3,4),2(2,4)-di-pyridinacycloundecaphan-3-one*

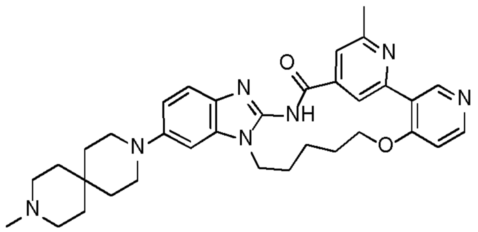

Step 1: Synthesis of compound 1-1

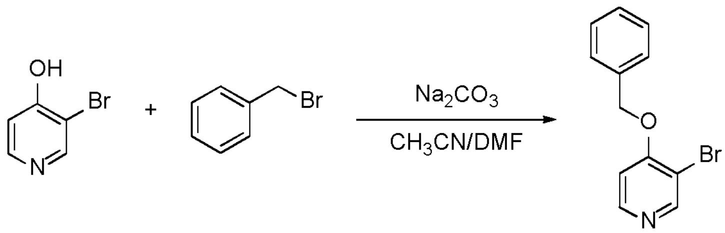


1-1

Dissolve 3-bromo-4-hydroxypyridine (1g, 5.75mmol) and benzyl bromide (1.03g, 6.03mmol) in acetonitrile (20mL) and DMF (5mL), and add sodium carbonate (1.22g, 11.49mmol). Heat the oil bath to 80 °C and react for 3 hours. LCMS monitoring, reaction completed. Reduce to room temperature. Add 100mL ethyl acetate and wash with 4 × 30mL of saturated saline solution. Dry the organic phase, filter, and concentrate. Purify by column chromatography(DCM/-MeOH=15/1). The product compound 1-1 (1.20g, yield 79.05%) was obtained. MS:263.99[M+H]+.

Step 2: Synthesis of Compounds 1-2

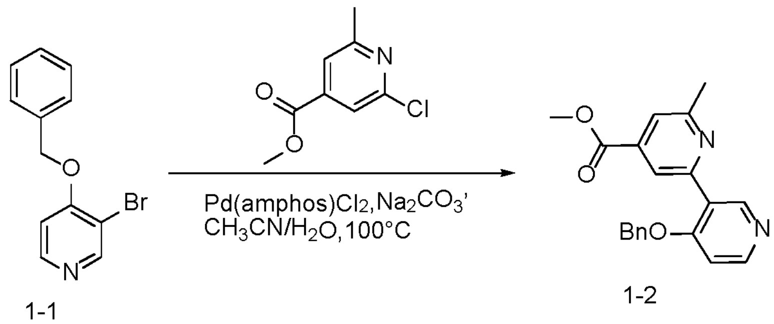


1-1 1-2

Compound 1-1 (500 mg, 1.89 mmol), 2-chloro-6-methylisonicotinic acid methyl ester (1.05 g, 5.68 mmol), biboric acid pinacol ester (1.45 g, 5.68 mmol), Pd(Amphos)$Cl_2$ (135 mg, 0.19 mmol) and sodium carbonate (502 mg, 4.73 mmol) were dispersed in acetonitrile (15 mL) and water (3 mL). Nitrogen protection, oil bath heated to 100 ° C for 2 hours. LCMS monitoring, the reaction was completed. Cool to room temperature. Add 30 mL water and extract with 50 mL ethyl acetate. The organic phase was dried, filtered and concentrated. Purify by Column chromatography (DCM/MeOH=20/1). The product compound 1-2 (200 mg, yield 31.60%) was obtained. MS: 335.13 $[M+H]^+$.

Step 3: Synthesis of compound 1-3

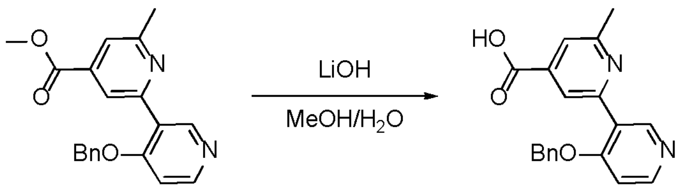


1-2 1-3

Dissolve compound 1-2 (200 mg, 0.6 mmol) in methanol (6 mL), add lithium hydroxide (51 mg, 1.2 mmol) and water (2 mL). React at room temperature for 1 hour. LCMS monitoring shows that the reaction is complete. Add 6 mL of water and concentrate to remove methanol. Adjust pH to 6 with 2 mol/L dilute hydrochloric acid, then concentrate. Purify by reverse phase column chromatography. (A: 0.1% formic acid aqueous solution, B: acetonitrile; acetonitrile from 10-40%, 15 mins). Product 1-3 was obtained (110 mg, yield: 57.41%). MS: 321.12 $[M+H]^+$.

Step 4: Synthesis of compound 1-4

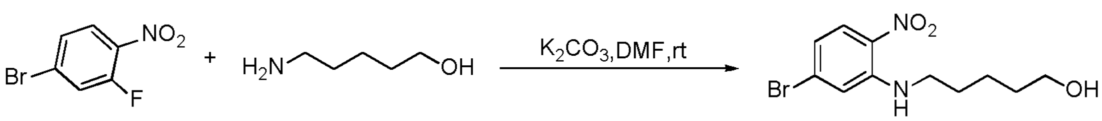


1-4

In a reaction flask, 4-bromo-2-fluoro-1-nitrobenzene (1.00 g, 4.55 mmol), 5-aminopentane-1-ol (0.52 g, 5.00 mmol), potassium carbonate (1.88 g, 13.64 mmol), and DMF (10 mL) were mixed evenly and stirred at room temperature overnight. The reaction solution was poured into 50 mL ice water and filtered. The filter cake was rinsed with 3 x 15 mL water and dried in vacuo to obtain the product compound 1-4 (1.2 g, yield: 87.01%). MS: 303.03, 305.03 $[M+H]^+$

Step 5: Synthesis of Compounds 1-5

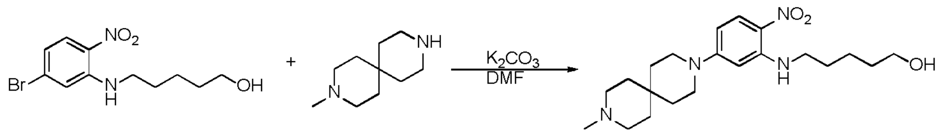


1-4 1-5

In a reaction flask, compound 1-4 (24.00 g, 90.91 mmol), 2-methyl-2,7-diazaspiro[3.5]nonane (15.61 g, 92.78 mmol), anhydrous potassium carbonate (25.64 g, 185.55 mmol) were dissolved in DMF (200 mL), heated to 120 ° C and stirred for 6.5 h. LCMS monitoring showed that the raw material reaction was complete and stirring was stopped. The reaction solution was cooled to room temperature, poured into 1 L water, stirred at room temperature for 1 h, filtered, and the filter cake was collected. The filtrate was extracted twice with ethyl acetate, the organic phases were combined, washed three times with water, washed once with saturated brine, dried, filtered, concentrated, the residue and the filter cake were combined, 200 mL ethyl acetate was added for pulping, filtered, the filter cake was rinsed with ethyl acetate, the filter cake was collected, and dried to obtain compound 1-5 (24.15 g, yield: 75.35%) as a yellow solid. MS: 391.26 $[M+H]^+$

Step 6: Synthesis of Compound 1-6

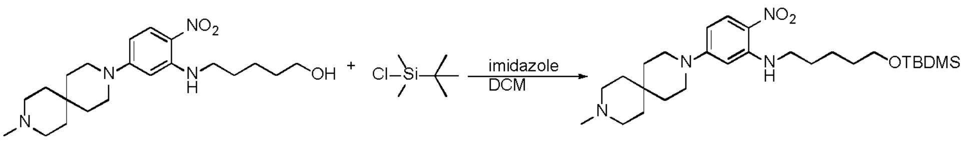


1-5 1-6

Compound 1-5 (16.00 g, 40.97 mmol) and imidazole (5.58 g, 61.46 mmol) were dissolved in dichloromethane (240 mL) in a 500 mL single neck flask, and compound TBDMSCl (9.26 g, 61.46 mmol) was added dropwise under an ice bath. After addition, the mixture was heated to room temperature and stirred for 12 hours. LCMS monitoring showed that the reaction was complete and stirring was stopped. 300 mL water was added to the reaction solution to quench the reaction, and the layers were separated by stirring. The DCM phase was collected, washed once with water, washed once with saturated brine, dried, filtered, and concentrated. The residue was mixed with silica gel and purified by Flash silica gel column (A: DCM, B: MeOH, phase from 0-20%, 15mins). Compound 1-6 (19.64 g, yield: 94.96%) was obtained as a yellow oil. MS: 505.35 $[M+H]^+$

Step 7: Synthesis of Compounds 1-7

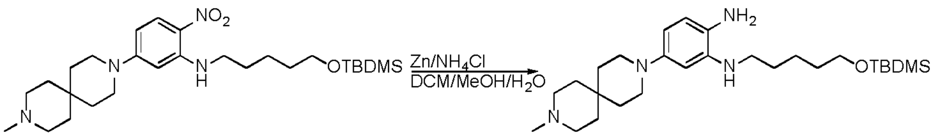


1-6 1-7

In a 1000 mL single neck flask, compounds 1-6 (15.00 g, 29.72 mmol) were dissolved in DCM/MeOH/$H_2O$ (100 mL/100 mL/150 mL). Zinc powder (10.00 g, 297.16 mmol) and ammonium chloride (15.89 g, 297.16 mmol) were added in an ice bath, and the reaction was allowed to proceed at room temperature for 3 hours. LCMS monitoring, the raw material reaction was complete, the reaction was stopped. Celite pad was used for suction filtration, the filter cake was rinsed with dichloromethane, the filtrate was collected, the layers were separated, the dichloromethane phase was collected, washed twice with water, washed once with saturated brine, dried, filtered, and concentrated to obtain compound 1-7 (13.26 g, yield: 94.00%). MS: 475.38 $[M+H]^+$

Step 8: Synthesis of Compound 1-8

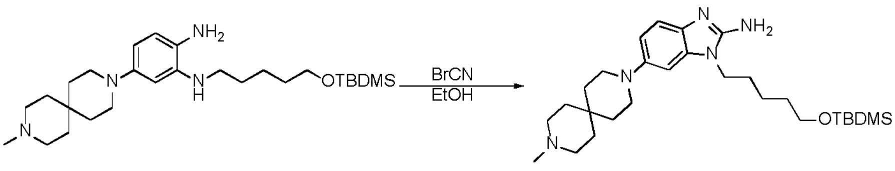


1-7 1-8

In a 250 mL single-necked flask, compound 1-7 (8.00 g, 16.85 mmol) was dissolved in anhydrous ethanol (100 mL), and solid cyanogen bromide (3.14 g, 20.22 mmol) was added under ice bath, and the mixture was stirred for 2 h. LCMS monitored the complete reaction of the raw material. Aqueous potassium carbonate solution (0.5 N) and DCM were added to the reaction solution, and the layers were stirred and separated. The dichloromethane phase was collected, washed twice with water, washed once with saturated brine, dried, filtered, and concentrated. The residue was purified by Flash C18 reverse phase column (A: 0.1% formic acid aqueous solution, B: acetonitrile; acetonitrile from 0-50%, 15 mins) to obtain compound 1-8 (3.80 g, yield: 45.12%).MS:500.37 $[M+H]^+$

Step 9: Synthesis of Compound 1-9

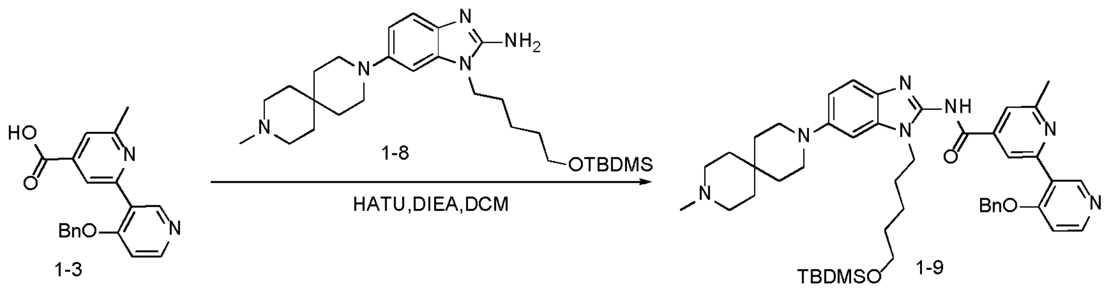


1-3 1-8 1-9

Compound 1-3 (90 mg, 0.28 mmol), compound 1-8 (141 mg, 0.28 mmol) and HATU (118 mg, 0.31 mmol) were dissolved in DCM (3 mL). Stirred and cooled. DIEA (91 mg, 0.70 mmol) was added at 0°C. After the addition was completed, the temperature was naturally raised to room temperature for 2 hours. The reaction was completed by LCMS monitoring. 30 mL DCM was added and washed with 20 mL saturated brine. The organic phase was dried, filtered and concentrated. The crude compound 1-9 (300 mg) was obtained.MS:802.48$[M+H]^+$.

Step 10: Synthesis of Compound 1-10

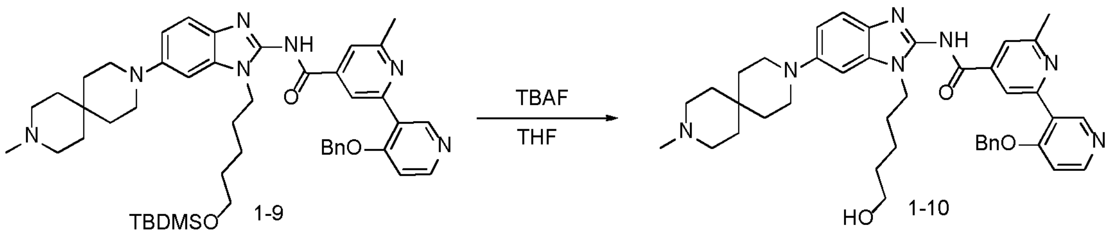


1-9 1-10

Compound 1-9 (300 mg, 0.37 mmol) was dissolved in THF (8 mL), and TBAF (1 mL) was added. The oil bath was heated to 60 °C for 6 hours. The reaction was completed by LCMS monitoring. The system was directly concentrated, purified by Flash C18 reverse phase column (A: 0.1% formic acid aqueous solution, B: acetonitrile; acetonitrile from 0-100%, 20 mins). Compound 1-10 (200 mg, yield 77.74%) was obtained.MS:688.39$[M+H]^+$.

Step 11: Synthesis of Compound 1-11

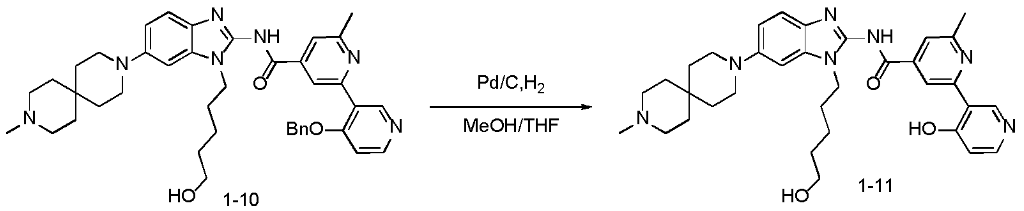

1-10 1-11

Compound 1-10 (170 mg, 0.25 mmol) was dissolved in methanol (6 mL) and THF (2 mL), and palladium carbon (132 mg) was added. Replace hydrogen gas. The reaction was carried out at room temperature for 12 hours. The reaction was completed by LCMS monitoring. The system was filtered through diatomaceous earth. The filtrate was concentrated. Purification was performed by Flash C18 reverse phase column (A: 0.1% formic acid aqueous solution, B: acetonitrile; acetonitrile from 0-80%, 15 mins) to obtain the product compound 1-11 (100 mg, yield: 67.70%).MS:598.34$[M+H]^+$.

Step 12: Synthesis of Compound 1

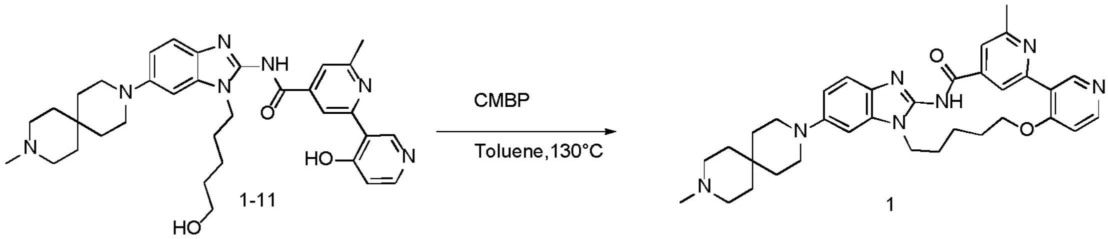

1-11 1

Compound 1-11 (100 mg, 0.17 mmol) was dissolved in toluene (2 mL), and CMBP (130 mg, 0.54 mmol) was added. The oil bath was heated to 130 °C for 1 hour. The reaction was completed by LCMS monitoring. The temperature was reduced to room temperature. The system was directly concentrated and purified by Prep-HPLC, chromatographic column luna C18, 30*250 mm, mobile phase (A: 0.1% formic acid aqueous solution, B: acetonitrile; acetonitrile from 17-50%, 20 mins). The product compound 1 (15 mg, yield: 16%) was obtained.MS:580.33$[M+H]^+$.

$^1H$ NMR (500 MHz, DMSO-$d_6$) δ 12.49 (s, 1H), 9.25 (s, 1H), 9.04 (s, 1H), 8.46 (d, *J* = 5.8 Hz, 1H), 7.71 (s, 1H), 7.36 (d, *J* = 8.6 Hz, 1H), 7.19 (d, *J* = 5.8 Hz, 1H), 7.14 (s, 1H), 7.06 (d, *J* = 2.2 Hz, 1H), 6.91 (dd, *J* = 8.8, 2.2 Hz, 1H), 4.23 (dt, *J* = 44.5, 5.3 Hz, 4H), 3.16 (dd, *J* = 11.4, 5.7 Hz, 3H), 2.81 (s, 3H), 2.64 (s, 3H), 2.57 (s, 1H), 2.53 (s, 3H), 2.01 (h, *J* = 7.3 Hz, 2H), 1.91 (dp, *J* = 19.3, 6.8, 5.6 Hz, 4H), 1.61 (d, *J*= 6.1 Hz, 9H).

Example 2 Synthesis of Compound 2

*$2^6$-methyl-$5^6$-(9-methyl-3,9-diazaspiro[5.5]undecan-3-yl)-$5^1$H-4-aza-5(2,1)-ben zo[d]imidazola-1(3,4),2(2,4)-dipyridinacycloundecaphan-3-one*

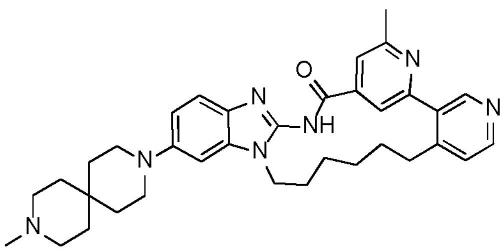

Step 1: Synthesis of compound 2-2

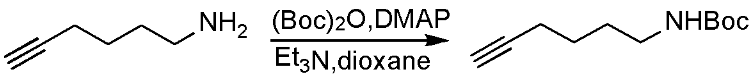


2-1 2-2

Compound 2-1 (5.0 g, 51.46 mmol), $(Boc)_2O$ (13.48 g, 61.75 mmol), TEA (15.62 g, 154 mmol), DMAP (1.26 g, 10.29 mmol) were added to 1,4-dioxane (80 mL) in a 250 mL single-necked flask and stirred at room temperature for 5 h. The reaction was completed by TLC monitoring, concentrated, and purified by column chromatography (dichloromethane: methanol = 40: 1). Compound 2-2 (9.83 g, yield: 96.85%) was obtained. MS: 198.14 $[M+H]^+$

Step 2: Synthesis of compound 2-3

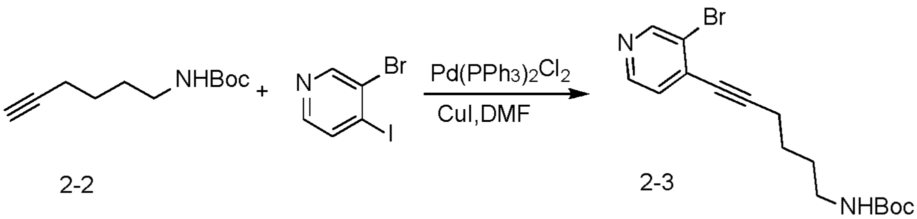


2-2 2-3

In a 250 mL reaction bottle, compound 2-2 (9.83 g, 49.84 mmol), 3-bromo-4-iodopyridine (16.98 g, 59.81 mmol), bis(triphenylphosphine)palladium dichloride (7.00 g, 9.97 mmol), CuI (4.75 g, 24.92 mmol), and DMF (80 mL) were mixed evenly, heated to 100°C under nitrogen protection for 4 h, cooled, 200 mL EA was added to the reaction solution, 200 mL water was used for extraction, and the organic layer was washed with saturated brine (200 mL*3), the organic layer was dried, filtered, and the filtrate was concentrated. The residue was separated and purified by column chromatography with an eluent of PE:EA=2:1. The eluent was concentrated to obtain the product compound 2-3 (13.81 g, yield: 78.42%).MS:353.08,355.08 $[M+H]^+$

Step 3: Synthesis of Compounds 2-4

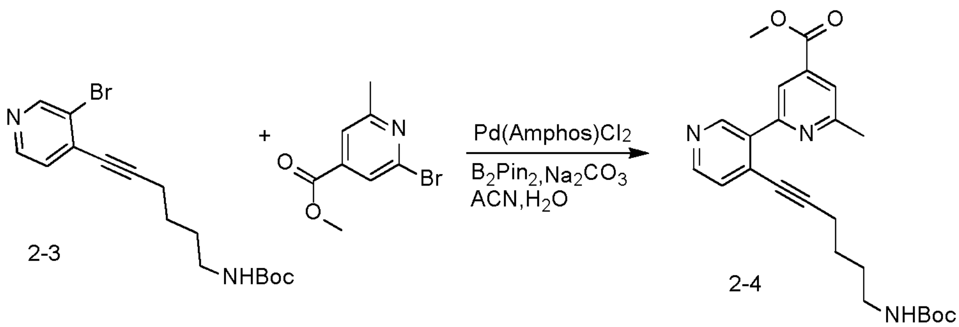


2-3 2-4

In a 250 mL reaction bottle, compound 2-3 (10.0 g, 28.31 mmol), 2-bromo-6-methylisonicotinic acid methyl ester (14.32 g, 62.27 mmol), $Pd(Amphos)_2Cl_2$ (1.60 g, 2.26 mmol), $B_2Pin_2$ (14.38 g, 56.62 mmol), sodium carbonate (9.00 g, 84.93 mmol), acetonitrile (100 mL), water (20 mL) were mixed evenly, heated to 100°C under nitrogen protection for 2 h, cooled, and the solvent was evaporated under reduced pressure. The residue was dissolved in 200 mL DCM, washed with water (200 mL) and saturated brine (200 mL) in turn, the organic layer was dried, filtered, and the filtrate was concentrated. The residue was separated and purified by column chromatography, the eluent was DCM:MeOH=25:1, and the eluent was concentrated to obtain the product compound 2-4 (5.18 g, yield: 43.22%). MS: 424.22 $[M+H]^+$

Step 4: Synthesis of compound 2-5

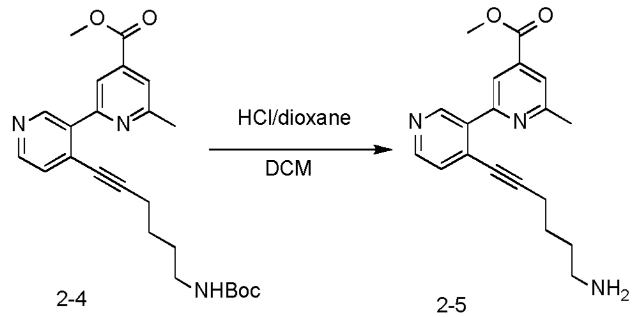


2-4 2-5

Compound 2-4 (5.00 g, 11.81 mmol) was added to DCM (20 mL) and stirred to dissolve. A solution of hydrogen chloride in 1,4-dioxane (4 M, 30 mL) was added dropwise. After the addition was completed, the mixture was stirred at room temperature for 1 hour. LCMS was used to monitor the reaction until it was complete, and the mixture was concentrated to obtain a crude compound 2-5 (4.10 g), which was directly used for the next step. MS: 324.16 [M+H] $^+$

Step 5: Synthesis of Compound 2-6

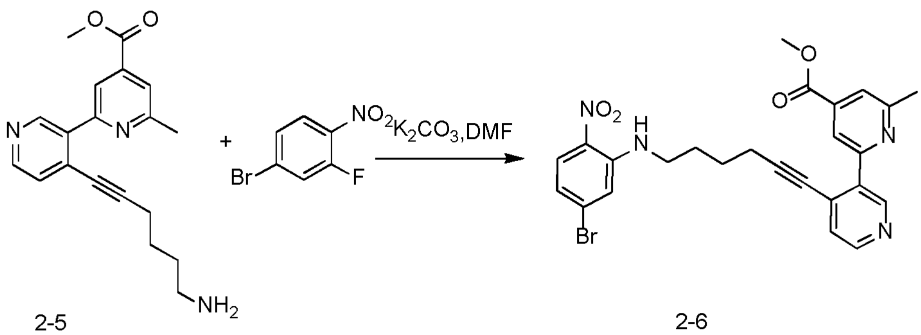


2-5 2-6

In a 100 mL reaction bottle, compound 2-5 (4.10 g, 11.81 mmol), 4-bromo-2-fluoro-1-nitrobenzene (3.12 g, 14.17 mmol), potassium carbonate (4.90 g, 35.43 mmol), and DMF (20 mL) were mixed evenly, heated to 50 ° C for overnight reaction, cooled, and 100 mL EA was added to the reaction solution, and 100 mL water was extracted. The organic layer was washed with saturated brine (100 mL * 3), the organic layer was dried, filtered, and the filtrate was concentrated. The residue was separated and purified by column chromatography, and the eluent was DCM: MeOH = 18: 1. The eluent was concentrated to obtain the product compound 2-6 (5.45 g, yield: 88.2%). MS: 523.09, 525.09 [M+H]$^+$

Step 6: Synthesis of Compound 2-7

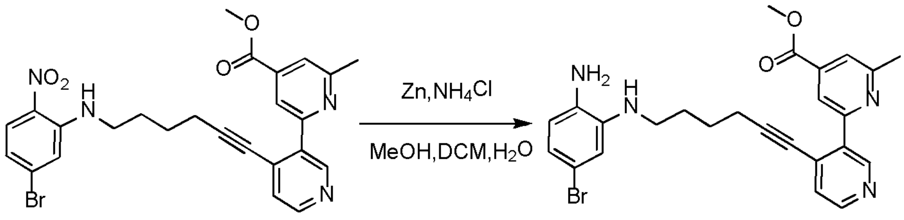


2-6 2-7

In a 100 mL single-necked flask, compound 2-6 (2.48 g, 4.73 mmol) was dissolved in DCM/MeOH/$H_2O$ (20 mL/10 mL/20 mL), and zinc powder (2.47 g, 37.84 mmol) and ammonium chloride (5.06 g, 94.6 mmol) were added under ice bath, and the temperature was raised to room temperature for 4 hours. LCMS monitoring showed that the reaction of the raw materials was complete and the reaction was stopped. Post-treatment: Filter by suction, rinse the filter cake with dichloromethane and methanol, collect the filtrate, separate the layers, collect the organic phase, wash twice with water, wash once with saturated brine, dry, filter, and concentrate to obtain compound 2-7 (2.22 g, yield: 95.20%). MS: 493.12,

495.12 [M+H]+

Step 7: Synthesis of Compound 2-8

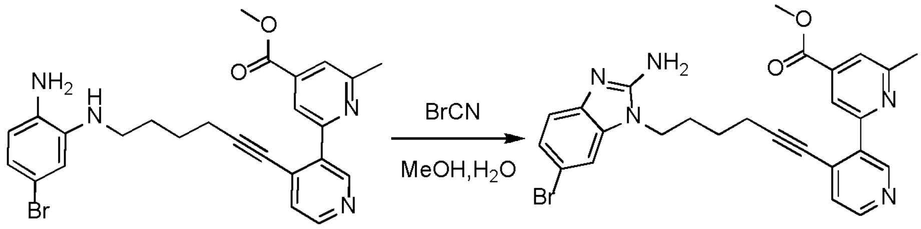


2-7 2-8

In a 100 mL single-necked flask, compound 2-7 (2.22 g, 4.50 mmol) was dissolved in anhydrous methanol (30 mL) and water (6 mL), and solid cyanogen bromide (0.52 g, 4.95 mmol) was added at 20°C. After the addition, the mixture was stirred at this temperature for 4 h. LCMS monitored the complete reaction of the raw material. Sodium bicarbonate aqueous solution (0.5 N) and DCM were added to the reaction solution, stirred and separated, and the organic phase was collected, washed twice with water, once with saturated brine, dried, filtered, and concentrated. The residue was mixed with silica gel and passed through Flash column chromatography (eluent: DCM: MeOH = 12:1) to obtain compound 2-8 (1.99 g, yield: 85.4%).MS:518.11,520.11[M+H]+

Step 8: Synthesis of compound 2-9

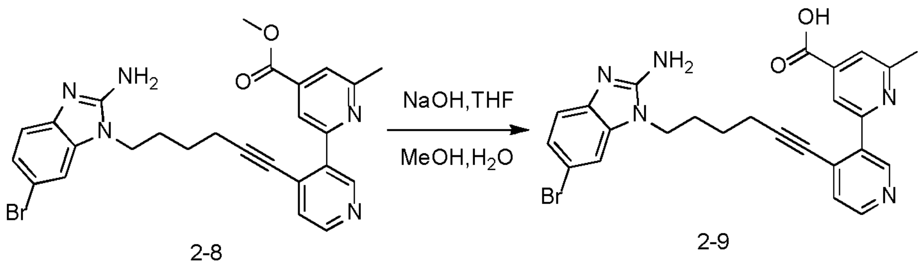


2-8 2-9

Compound 2-8 (1.99 g, 3.84 mmol) was dissolved in methanol (8 mL) and tetrahydrofuran (8 mL), sodium hydroxide (0.31 g, 7.68 mmol) and water (8 mL) were added. The reaction was allowed to react at room temperature for 2 hours. The reaction was complete as monitored by LCMS. The methanol and tetrahydrofuran were removed by concentration. The pH was adjusted to 7 with 2N dilute hydrochloric acid. The system was concentrated, purified by Flash C18 reverse phase column (A: 0.1% formic acid aqueous solution, B: acetonitrile; acetonitrile from 10-100%, 20 mins). The product compound 2-9 (1.89 g, yield: 97.8%) was obtained. MS: 504.10, 506.10 [M+H]+.

Step 9: Synthesis of Compounds 2-10

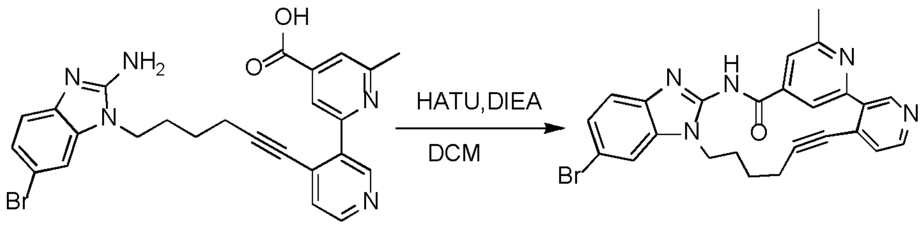


2-9 2-10

Compound 2-9 (1.89 g, 3.75 mmol) was dissolved in DCM (30 mL). Stir and cool. HATU (1.43 g, 3.75 mmol) and DIEA (3.1 mL, 18.75 mmol) were added at 0°C. After the addition was completed, stirring was continued at this temperature for 2 hours. The reaction was completed by LCMS monitoring. 30 mL DCM was added, and washed with 50 mL water and 50 mL saturated brine in turn. The organic phase was dried, filtered, and concentrated. The residue was mixed with silica gel and passed through a Flash column chromatography (eluent: DCM: MeOH = 15: 1) to obtain compound 2-10 (0.99 g, yield: 54.32%). MS: 486.09, 488.09 [M+H]+

Step 10: Synthesis of compound 2-11

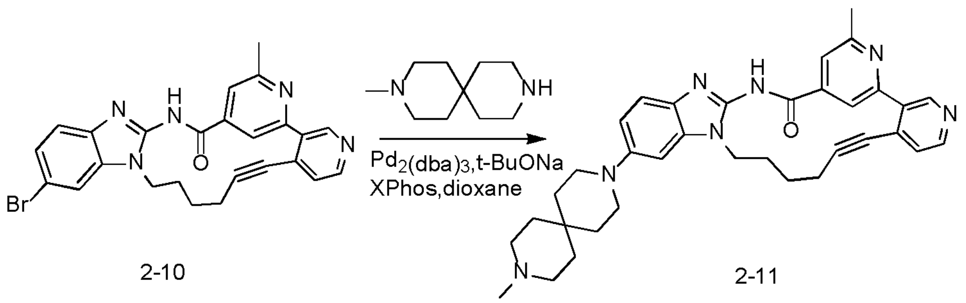


2-10 2-11

Compound 2-10 (0.99 g, 2.03 mmol) was added to the reaction flask, anhydrous dioxane (20 mL) was added as solvent, and then sodium tert-butoxide (0.39 g, 4.06 mmol), Xphos (195 mg, 0.41 mmol), $Pd_2(dba)_3$ (183 mg, 0.2 mmol) and 3-methyl-3,9-diazaspiro[5,5]undecane (0.68 g, 4.06 mmol) were added in sequence, replace with nitrogen gas, and the reaction was carried out at 100°C for 3 hours. The reaction was monitored by LCMS, heating was stopped, the solvent was evaporated under reduced pressure, and the residue was mixed with silica gel and passed through Flash column chromatography (eluent: DCM:MeOH=12:1) to obtain compound 2-11 (0.81 g, yield: 69.45%). MS: 574.32 [M+H]+

Step 11: Synthesis of Compound 2

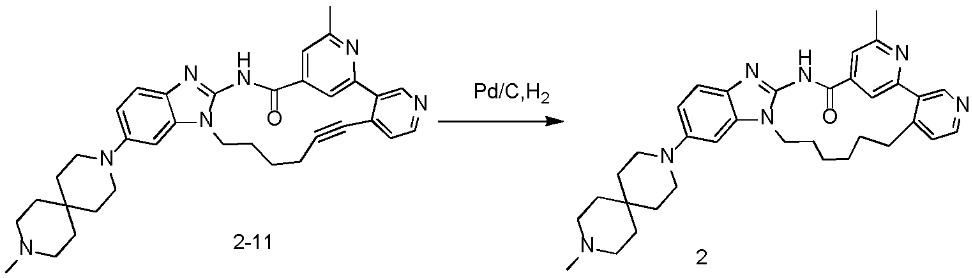


2-11 2

Compound 2-11 (100mg, 0.174mmol) was added to MeOH (8mL), followed by the addition of 15% Pd/C (15mg). After the addition is complete, replace the hydrogen gas three times and stir the reaction at room temperature for 2 hours. LCMS monitors until the reaction is complete. Stop the reaction, filter, rinse the filter cake with methanol, concentrate the filtrate, prepare by Prep HPLC, chromatography column Luna C18, 30 * 250 mm, mobile phase (A: 0.1% formic acid aqueous solution, B: acetonitrile; acetonitrile 17-50%, 20mins) yields the product compound 2 (53mg, 52.34%), MS:578.35[M+H]+

Example 3 Synthesis of Compound 3

*(R)-2^6,7-dimethyl-5^6-(9-methyl-3,9-diazaspiro[5.5]undecan-3-yl)-5^1H-11-oxa-4 -aza-5(2,1)-benzo[d]imidazola-1(3,4),2(2,4)-dipyridinacycloundecaphan-3-one*

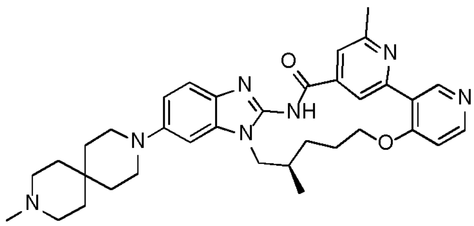

Step 1: Synthesis of compound 3-1

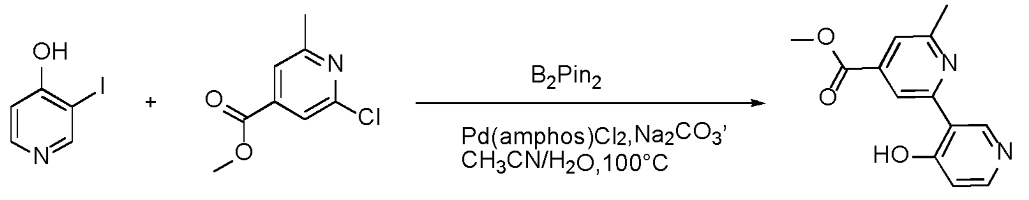


3-1

4-Hydroxy-3-iodopyridine (15 g, 67.87 mmol), 2-chloro-6-methylisonicotinic acid methyl ester (25 g, 135.74 mmol), biboric acid pinacol ester (52 g, 203.61 mmol), Pd(amphos)$Cl_2$ (4.7 g, 6.8 mmol) and sodium carbonate (28 g, 203 mmol) were dispersed in acetonitrile (900 mL) and water (90 mL). Under nitrogen protection, the oil bath was heated to 100 °C for 2 hours. The reaction was completed by LCMS monitoring. Cool to room temperature. Add 1 L water, adjust the pH to neutral with 1N dilute hydrochloric acid, and extract with 500 mL ethyl acetate three times. Combine the organic phases, dry and concentrate. Purify by column chromatography (DCM/MeOH=10/1). The product compound 3-1 (10 g, yield 62%) was obtained.MS:245.08[M+H] $^+$.

Step 2: Synthesis of compound 3-2

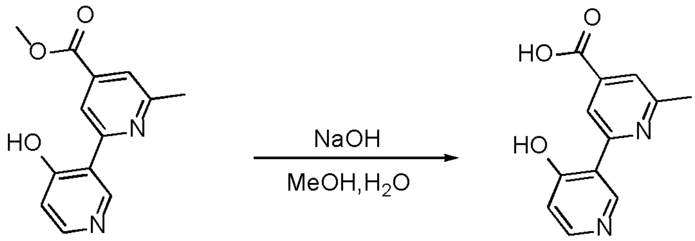


3-1 3-2

Compound 3-1 (10 g, 40.98 mmol) was added to methanol (100 mL), and NaOH (3.28 g, 81.97 mmol, dissolved in 20 mL water) aqueous solution was added dropwise. After addition, the mixture was reacted at room temperature for 1 h. The reaction was monitored by LCMS. The pH was adjusted to 4 with 1 N HCl. A large amount of solid precipitated. The solid was filtered and dried to obtain the product compound 3-2 (7.22 g, yield: 76%). MS: 231.07 [M+H] $^+$

Step 3: Synthesis of compound 3-3

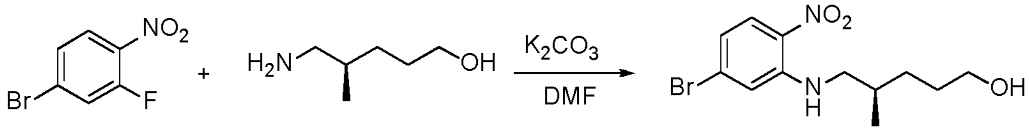


3-3

The synthesis method of compound 3-3 is the same as that of compound 1-4, except that 5-aminopentane-1-ol is replaced by (R)-5-amino-4-methylpentane-1-ol. MS of compound 3-3: 317.04, 319.04 [M+H] +

Step 4: Synthesis of compound 3-4

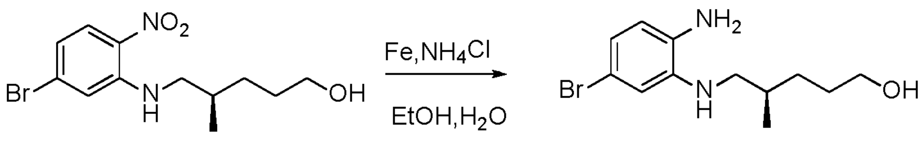


3-3 3-4

Compound 3-3 (2.90 g, 9.14 mmol), iron powder (2.55 g, 45.72 mmol), ammonium chloride (2.45 g, 45.72 mmol), ethanol (30 mL), and $H_2O$ (10 mL) were mixed evenly and reacted at 60°C for 1 h. The reaction was completed after LCMS monitoring. The mixture was filtered, dried by spin drying, and column chromatography with DCM/MeOH=10/1 was used to obtain the product compound 3-4 (2.60 g, yield: 99%).MS:287.07,289.07[M+H] +

Step 5: Synthesis of Compounds 3-5

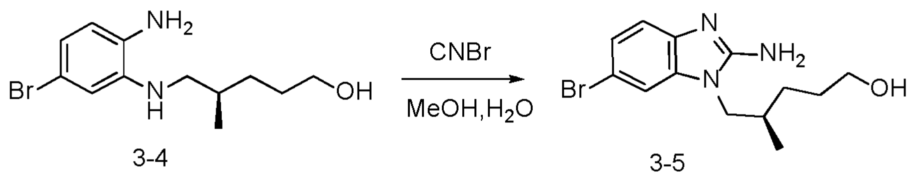


3-4 3-5

Compound 3-4 (2.60 g, 9.05 mmol), cyanogen bromide (1.15 mg, 10.86 mmol), methanol (30 mL) and water (6 mL) were mixed evenly and reacted at 60°C for 0.5 h. The reaction was complete after LCMS monitoring. The pH was adjusted to 8, and 3 x 50 mL DCM was extracted. The product compound 3-5 (2.70 mg, yield: 95%) was obtained by spin drying. MS:312.06,314.06[M+H] +

Step 6: Synthesis of compound 3-6

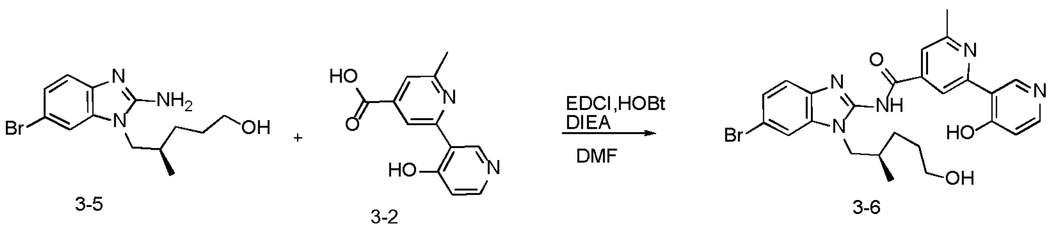


3-5 3-2 3-6

Compound 3-5 (0.40 g, 1.28 mmol), compound 3-2 (0.35 g, 1.54 mmol) and DIEA (1.06 mL, 6.41 mmol) were added to DMF (8 mL). HOBt (0.26 mg, 1.92 mmol) and EDCI (0.37 g, 1.92 mmol) were added, and the reaction was carried out at 60°C for 1 h. The reaction was monitored by LCMS. 200 mL water was added to quench the reaction, 3 x 10 mL DCM was extracted, dried over anhydrous sodium sulfate, filtered, and dried by spin drying. The product compound 3-6 (0.16 g, yield: 23.8%) was obtained. MS: 524.12, 526.12 [M+H] +

Step 7: Synthesis of Compound 3-7

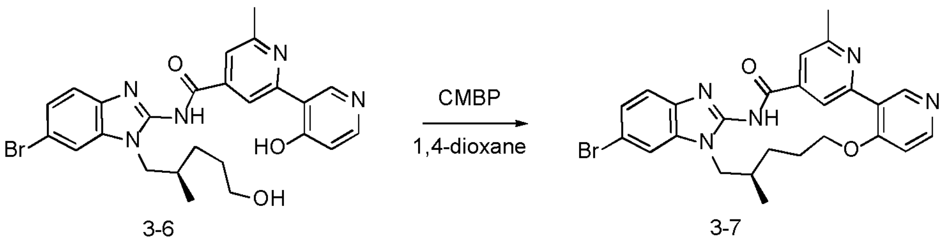


Compound 3-6 (150 mg, 0.29 mmol) was added to dioxane (4 mL). And CMBP (1380 mg, 5.72 mmol) was added. The oil bath was heated to 110°C for 2 hours. The reaction was completed by LCMS monitoring. The temperature was reduced to room temperature. The system was directly concentrated, purified by PTLC (DCM/MeOH=15/1). The product compound 3-7 (70 mg, yield: 48%) was obtained.MS:506.11,508.11[M+H] +

Step 8: Synthesis of compound 3

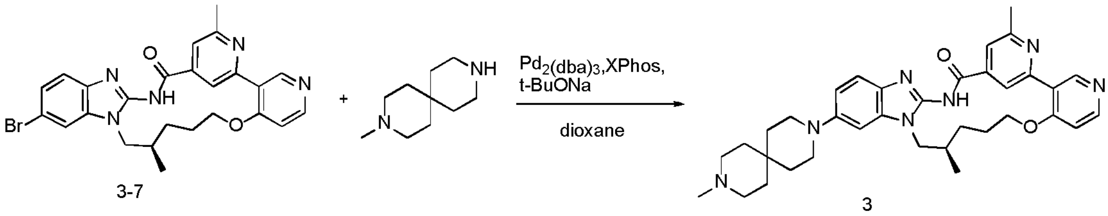


The synthesis method of compound 3 is the same as that of compound 2-11, except that compound 2-10 is replaced by compound 3-7. The crude product is purified by prep-HPLC, using a chromatographic column luna C18, 30*250 mm, and a mobile phase (A: 0.1% formic acid aqueous solution, B: acetonitrile; acetonitrile from 17-50%, 20 mins) to obtain compound 3.MS:594.35[M+H] +

Example 4 Synthesis of Compound 4

*(R)-2^6,7-dimethyl-5^6-(9-(oxetan-3-yl)-3,9-diazaspiro[5.5]undecan-3-yl)-5^1H-11-oxa-4 -aza-5(2,1)-benzo[d]imidazola-1(3,4),2(2,4)-dipyridinacycloundecaphan-3-one*

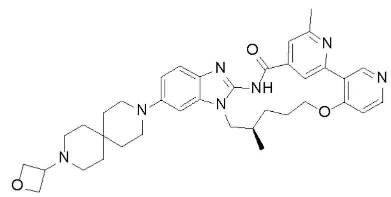

Step 1: Synthesis of compound 4-1

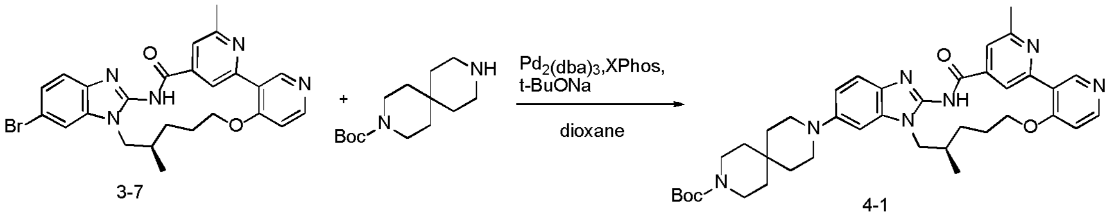


The synthesis method of compound 4-1 is the same as that of compound 3, except that 3-methyl-3,9-diazaspiro[5,5]undecane is replaced by tert-butyl 3,9-diazaspiro[5.5]undecane-3-carboxylate to obtain compound 4-1. MS: 636.36 [M+H] +

Step 2: Synthesis of compound 4-2

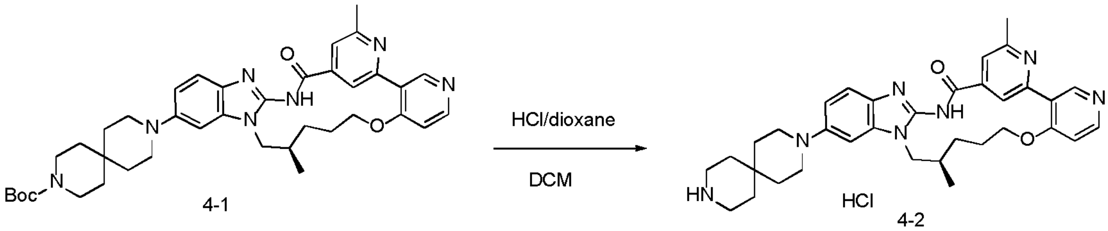


4-1 4-2

Compound 4-1 (7.2 g, 10.59 mmol) and DCM (70 mL) were added to a 100 mL reaction bottle. Dioxane hydrochloride solution (26.48 mL, 105.91 mmol, 4M) was added dropwise at room temperature. The reaction was continued for 2 h at room temperature. LCMS monitoring showed that the reaction of the raw material was complete and stirring was stopped. The reaction solution was directly filtered, and the filter cake was rinsed twice with dichloromethane and once with n-hexane. The filter cake was collected and dried to obtain the product compound 4-2 (6.50 g, yield: 99.6%). MS: 580 [M+H] +

Step 3: Synthesis of compound 4

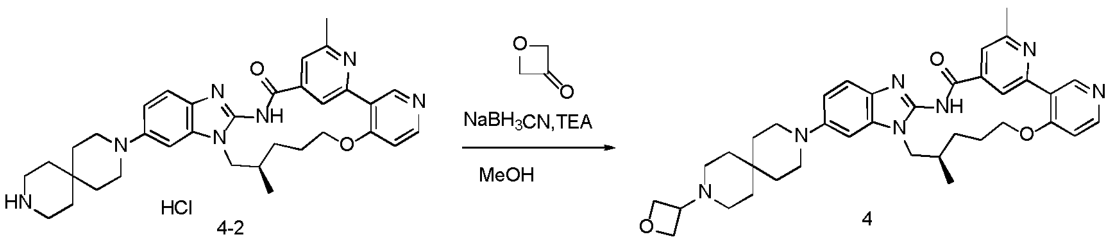


4-2 4

In a 100 mL single-necked flask, compound 4-2 (3.50 g, 5.68 mmol) was dissolved in anhydrous methanol (30 mL), and then triethylamine (1.72 g, 17.04 mmol), 3-oxetanone (4.09 g, 56.80 mmol), sodium cyanoborohydride (7.14 g, 113.60 mmol) were added in sequence, stirred and mixed evenly, and the temperature was raised to 60°C for 1 h. The reaction of the raw materials was monitored by LCMS and the stirring was stopped. The reaction solution was cooled to room temperature, and dichloromethane (150 mL) was added, and the mixture was washed twice with a saturated sodium carbonate aqueous solution, once with water, and once with a saturated saline solution, dried, filtered, and the filtrate was concentrated. During the concentration process, acetonitrile was added and steamed twice, filtered, and the filter cake was rinsed with a small amount of acetonitrile. The filter cake was collected and dried to obtain the product compound 4 (1.91 g, yield: 47.84%).MS:636 [M+H] +

$^1H$ NMR (500 MHz, DMSO-$d_6$) δ 12.53 (s, 1H), 9.14 (s, 1H), 8.97 (s, 1H), 8.45 (d, *J* = 5.8 Hz, 1H), 7.75 (s, 1H), 7.36 (d, *J* = 8.7 Hz, 1H), 7.19 (d, *J* = 5.9 Hz, 1H), 7.06 (d, *J* = 2.2 Hz, 1H), 6.88 (dd, *J* = 8.8, 2.2 Hz, 1H), 4.51 (t, *J* = 6.5 Hz, 2H), 4.40 (t, *J* = 6.1 Hz, 2H), 4.23 (td, *J* = 10.8, 6.8 Hz, 2H), 4.08 (dd, *J* = 14.0, 2.8 Hz, 1H), 4.00 (dd, *J* = 13.8, 6.6 Hz, 1H), 3.38 (q, *J* = 6.4 Hz, 1H), 3.12 (t, *J* = 5.7 Hz, 4H), 2.63 (s, 3H), 2.30 - 2.22 (m, 2H), 2.19 (q, *J* = 8.3, 5.7 Hz, 4H), 2.03 (d, *J* = 12.6 Hz, 1H), 1.72 (d, *J* = 4.4 Hz, 0H), 1.70 (s, 1H), 1.56 (t, *J* = 5.7 Hz, 5H), 1.48 (d, *J* = 5.7 Hz, 4H), 0.93 (d, *J* = 6.5 Hz, 3H).

Example 5 Synthesis of Compound 5

*(R)-5$^5$-fluoro-26,7-dimethyl-5$^6$-(9-methyl-3,9-diazaspiro[5.5]undecan-3-yl)-5$^1$H -11-oxa-4-aza-5(2,1)-benzo[d]imidazola-1(3,4),2(2,4)-dipyridinacycloundecaphan-3-one*

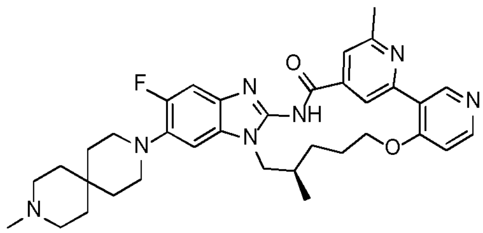

Step 1: Synthesis of compound 5-1

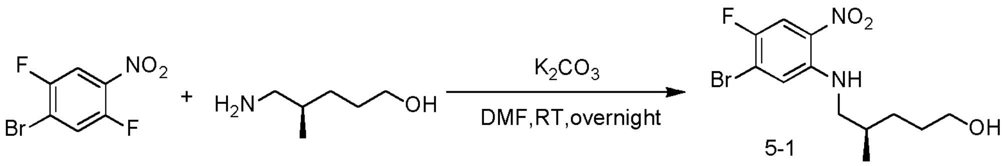


5-1

The synthesis method of compound 5-1 is the same as that of compound 3-3, except that 4-bromo-2-fluoro-1-nitrobenzene is replaced by 4-bromo-2,5-difluoronitrobenzene. MS of compound 5-1:335.03, 337.03 [M+H] $^+$

Step 2: Synthesis of compound 5-2

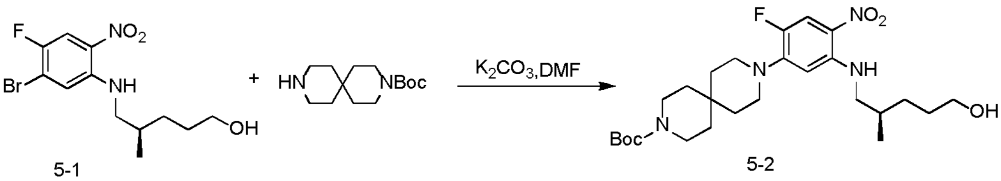


5-1 5-2

In a 500 mL single-necked flask, compound 5-1 (10.25 g, 30.58 mmol), tert-butyl 3,9-diazaspiro[5.5]undecane-3-carboxylate (11.67 g, 45.87 mmol), and anhydrous potassium carbonate (10.57 g, 76.45 mmol) were dissolved in DMF (200 mL), heated to 100 °C and stirred for 8 h. LCMS monitoring showed that the raw material reaction was complete and stirring was stopped. The reaction solution was cooled to room temperature and poured into 600 mL water. It was stirred at room temperature for 1 h, filtered, and the filter cake was collected. The filter cake was purified by column chromatography with DCM: MeOH = 50:1-15:1 to obtain compound 5-2 (13.00 g, yield: 83.58 %) as a yellow solid. MS: 509.31 [M+H]$^+$

Step 3: Synthesis of compound 5-3

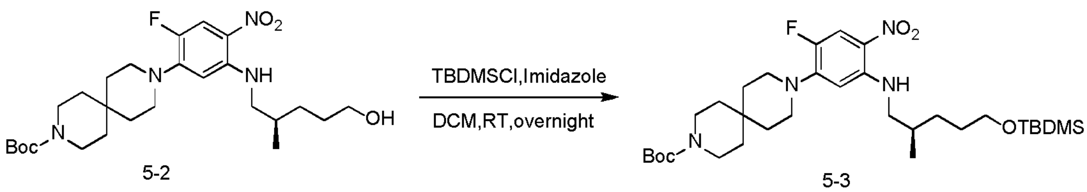


5-2 5-3

Compound 5-2 (2.00 g, 3.93 mmol) and imidazole (0.54 g, 7.86 mmol) were dissolved in dichloromethane (50 mL) in a 100 mL single-necked flask. Compound TBDMSCl (0.71 g, 4.72 mmol) was added under ice bath. After addition, the mixture was warmed to room temperature and stirred overnight. LCMS monitoring showed that the reaction was complete and stirring was stopped. The reaction solution was poured into 100 mL water to quench, stirred and separated, and the DCM phase was collected, washed once with water and once with saturated brine, dried, filtered, and concentrated. The residue was mixed with silica gel and purified by Flash silica gel column (A: PE, B: EtOAc, B phase from 0-30%, 15mins). Compound 5-3 (2.10 g, yield: 85.74%) was obtained. MS: 623.39 [M+H]$^+$

Step 4: Synthesis of compound 5-4

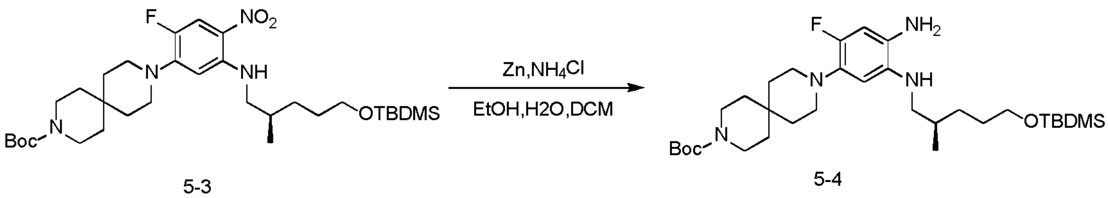


5-3 5-4

The synthesis method of compound 5-4 is the same as compound 1-7, except that compound 1-6 is replaced by compound 5-3. MS of compound 5-4:593.42 [M+H] + Step 5: Synthesis of compound 5-5

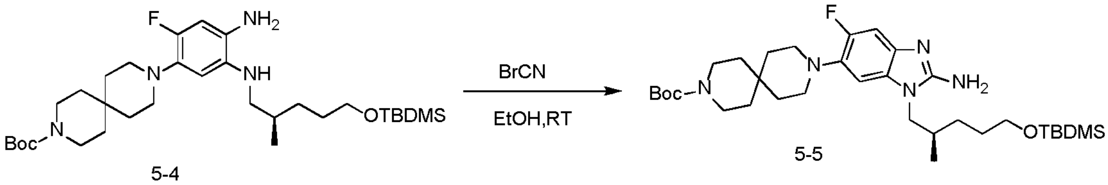


5-4 5-5

The synthesis method of compound 5-5 is the same as that of compound 1-8, except that compound 1-7 is replaced by compound 5-4. MS of compound 5-5: 618.41 [M+H] +

Step 6: Synthesis of compound 5-6

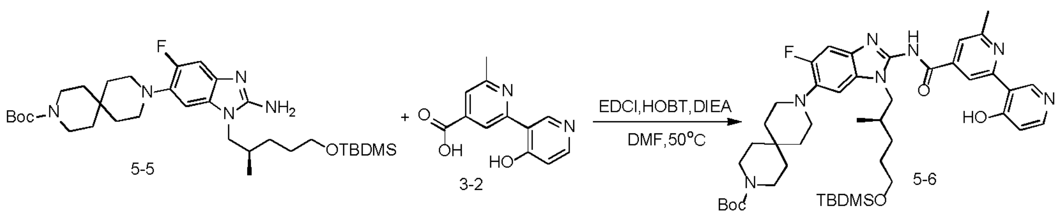


5-5 3-2 5-6

Compound 5-5 (1.70 g, 2.75 mmol), compound 3-2 (0.63 g, 2.75 mmol), EDCI (1.58 g, 8.25 mmol), HOBt (1.12 g, 8.25 mmol), DIEA (1.78 g, 13.76 mmol), and DMF (15 mL) were mixed evenly and reacted at 40 °C for 2 h. The reaction was completed after LCMS monitoring. The reaction system was poured into 100 mL ice water, extracted with 3 x 50 mL of DCM, and spin-dried by column chromatography with DCM/MeOH=30/1 to obtain the product compound 5-6 (1.70 g, yield: 74.44%).MS:830.47[M+H] +

Step 7: Synthesis of Compound 5-7

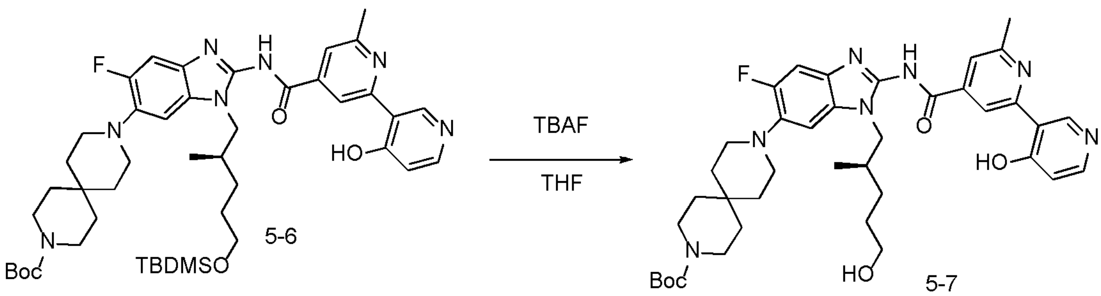


5-6 5-7

Compound 5-6 (1.60 g, 1.93 mmol), TBAF (5.78 mL, 1 mol/L), and THF (10 mL) were mixed evenly and reacted at

70°C for 4 h. The reaction was completed after monitoring by LCMS. The mixture was spin-dried, slurried with 50 mL water for 3 times, filtered, and the filter cake was rinsed with 3 x 15 mL of water. The filter cake was dried under vacuum to obtain the product compound 5-7 (1.15 g, yield: 83.35%). MS:716.39[M+H] $^+$

Step 8: Synthesis of Compound 5-8

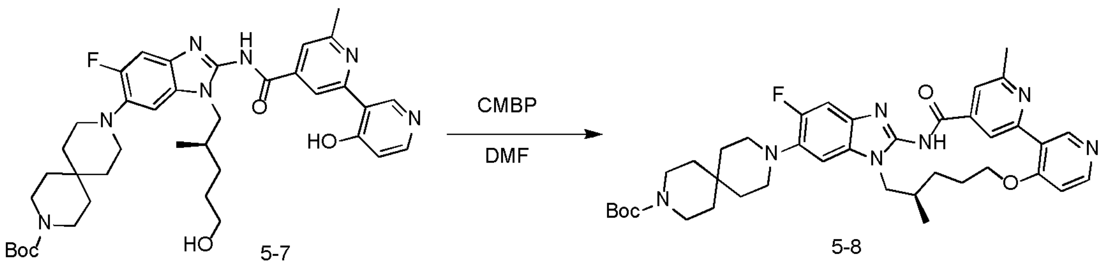

5-7 5-8

Compound 5-7 (300 mg, 0.42 mmol) was dissolved in DMF (3 mL), and CMBP (1011 mg, 4.19 mmol) was added. The oil bath was heated to 110°C for 2 hours. The reaction was completed by LCMS monitoring. The temperature was reduced to room temperature. The system was directly concentrated. Purification by Prep-HPLC, chromatographic column luna C18, 30*250 mm, mobile phase (A: 0.1% formic acid aqueous solution, B: acetonitrile; acetonitrile from 17-40%, 20mins), to obtain the product compound 5-8.MS:698.38[M+H]$^+$.

Step 9: Synthesis of Compound 5-9

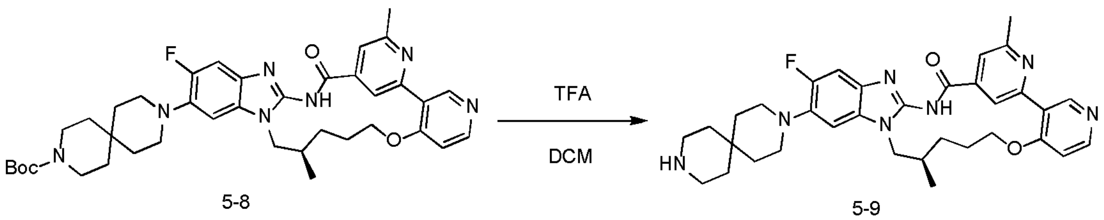

5-8 5-9

Compound 5-8 (220 mg, 0.31 mmol) was dissolved in DCM (4 mL), and TFA (2 mL) was added. After addition, the mixture was reacted at room temperature for 1 hour. The reaction was completed after LCMS monitoring. The system was directly concentrated, and the concentrate was dissolved in 20 mL DCM. The pH was adjusted to alkaline with saturated potassium carbonate aqueous solution, and the liquids were separated. The aqueous phase was extracted with DCM, and the organic phases were combined, dried over anhydrous sodium sulfate, filtered, and concentrated to obtain the product compound 5-9 (156 mg, yield: 85.37%).MS:598.32[M+H]$^+$

Step 10: Synthesis of compound 5

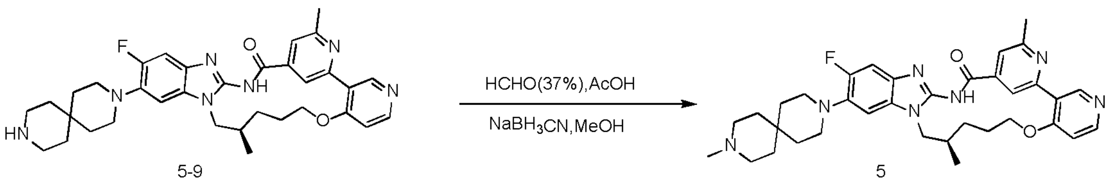

5-9 5

Compound 5-9 (50 mg, 0.08 mmol), 0.5 mL formaldehyde aqueous solution, 1 drop of HOAc and MeOH (3 mL) were mixed evenly, $NaBH_3CN$ (52.60 mg, 0.84 mmol) was added at room temperature, and reacted at room temperature for 1 h. The reaction solution was directly prepared by Prep-HPLC, chromatographic column luna C18, 30*250 mm, mobile phase (A: 0.1% formic acid aqueous solution, B: acetonitrile; acetonitrile from 17-50%, 20mins), to obtain product compound 5 (11.20 mg, yield: 21.87%).MS:612.34[M+H]$^+$

Example 6 Synthesis of Compound 6

*(R)-5⁵-fluoro-2⁶,7-dimethyl-5⁶-(9-(oxetan-3-yl)-3,9-diazaspiro[5.5]undecan-3-yl) -5¹H-11-oxa-4-aza-5(2,1)-benzo[d] imidazola-1(3,4),2(2,4)-dipyridinacycloundecapha n-3-one*

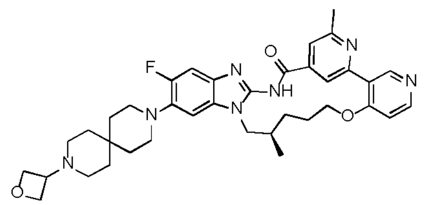

Step 1: Synthesis of compound 6

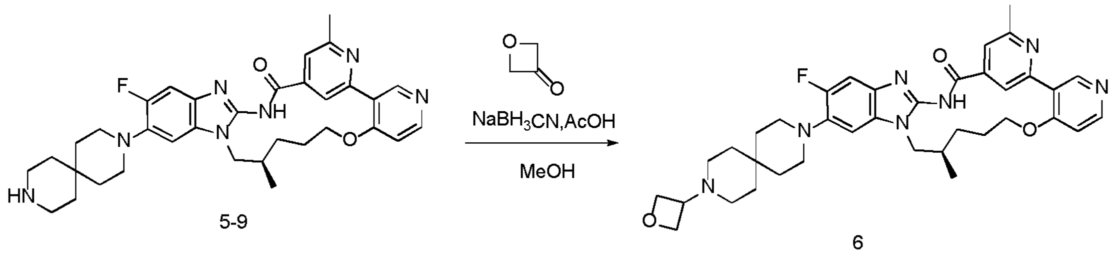


5-9

6

Compound 5-9 (100 mg, 0.17 mmol) was dissolved in anhydrous methanol (4 mL), and then glacial acetic acid (100 mg, 1.67 mmol), 3-oxetanone (121 mg, 1.67 mmol) and sodium cyanoborohydride (105 mg, 1.67 mmol) were added, and the mixture was stirred and mixed evenly. The reaction was carried out at room temperature for 2 hours. The reaction of the raw materials was monitored by LCMS and the stirring was stopped. The reaction solution was added with dichloromethane (15 mL), washed twice with saturated sodium carbonate aqueous solution, once with water, and once with saturated brine, dried, filtered, concentrated, and prepared by Prep-HPLC, with a chromatographic column luna C18, 30*250 mm, and a mobile phase (A: 0.1% formic acid aqueous solution, B: acetonitrile; acetonitrile was 17-50%, 20 mins), to obtain the product compound 6 (22.90 mg, yield: 19.56%). MS:654.35[M+H]$^+$

$^1$H NMR (500 MHz, DMSO-$d_6$) δ 12.67 (s, 1H), 9.14 (s, 1H), 8.98 (s, 1H), 8.46 (d, *J* = 5.8 Hz, 1H), 7.75 (d, *J* = 1.2 Hz, 1H), 7.32 - 7.25 (m, 2H), 7.20 (d, *J* = 5.9 Hz, 1H), 4.52 (t, *J* = 6.4 Hz, 2H), 4.41 (t, *J* = 6.1 Hz, 2H), 4.26 (d, *J*= 11.6 Hz, 2H), 4.15 - 4.00 (m, 2H), 3.39 (p, *J* = 6.4 Hz, 1H), 2.96 (dt, *J* = 17.3, 6.7 Hz, 4H), 2.64 (s, 3H), 2.24 (d, *J* = 32.8 Hz, 6H), 2.03 (s, 1H), 1.76 - 1.70 (m, 1H), 1.60 (t, *J* = 5.9 Hz, 5H), 1.53 (d, *J* = 5.8 Hz, 4H), 0.93 (d, *J* = 6.5 Hz, 3H).

Example 7 Synthesis of Compound 7

*2⁶,9,9-trimethyl-5⁶-(9-methyl-3,9-diazaspiro[5.5]undecan-3-yl)-5¹H-11-oxa-4-az a-5(2,1)-benzo[d]imidazola-1(3,4),2(2,4)-dipyridinacycloundecaphan-3-one*

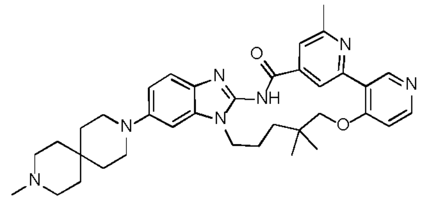

Step 1: Synthesis of compound 7-1

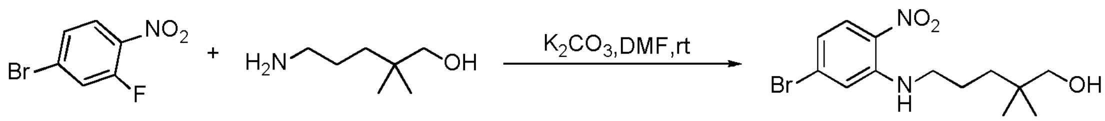


7-1

4-Bromo-2-fluoro-1-nitrobenzene (2.00 g, 9.09 mmol), 5-amino-2,2-dimethyl-pentane-1-ol (1.31 g, 10.00 mmol), potassium carbonate (3.77 g, 27.27 mmol) and DMF (20 mL) were mixed evenly, stirred at room temperature overnight, 60 mL ethyl acetate was added, 3 x 40 mL of saturated brine was washed, dried over anhydrous sodium sulfate, filtered and dried to obtain the product compound 7-1 (3.00 g, yield: 99.6%). MS: 331.06 $[M+H]^+$

Step 2: Synthesis of compound 7-2

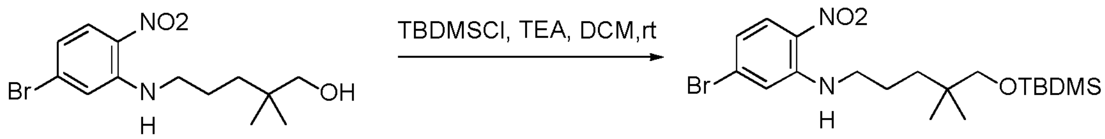


7-1 7-2

Compound 7-1 (2.10 g, 6.97 mmol), triethylamine (2.91 mL, 20.91 mmol) and DCM (20 mL) were mixed evenly, and TBDMSCl (1.58 g, 10.46 mmol) was added at room temperature, and the mixture was kept warm for 2 h. The reaction solution was spin-dried and column chromatography PE/EA=10/1 was performed to obtain the product compound 7-2 (3.00 g, yield: 96.6%).MS:445.14 $[M+H]^+$

Step 3: Synthesis of compound 7-3

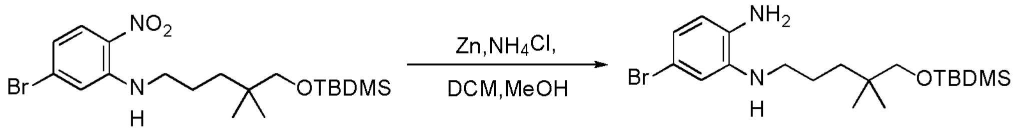


7-2 7-3

Compound 7-2 (1.50 g, 3.37 mmol), Zn (2.20 g, 33.67 mmol), ammonium chloride (1.80 g, 33.67 mmol), DCM (10 mL), $H_2O$ (10 mL) and MeOH (10 mL) were added to the reaction bottle and reacted at 40°C for 1 h. The reaction was completed after LCMS monitoring, and the mixture was filtered, separated, dried over anhydrous sodium sulfate, filtered, and dried to obtain the product compound 7-3 (0.90 g, yield: 64%).MS:415.17$[M+H]^+$

Step 4: Synthesis of compound 7-4

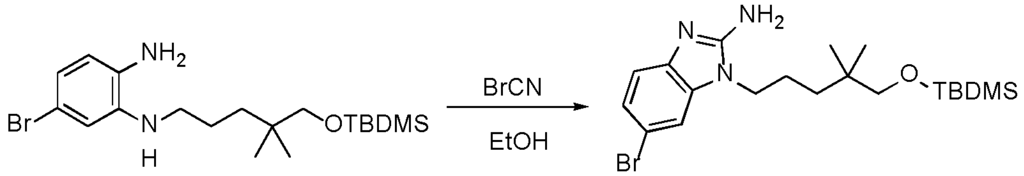


7-3 7-4

Compound 7-3 (0.90 g, 2.17 mmol), anhydrous ethanol (20 mL) and cyanogen bromide (0.25 g, 2.38 mmol) were mixed evenly, reacted at room temperature for 1h. LCMS monitored the reaction to be complete, directly spin-dried, and used for the next reaction. Compound 7-4 (0.60 g, yield: 95%).MS:440.17 $[M+H]^+$

Step 5: Synthesis of compound 7-5

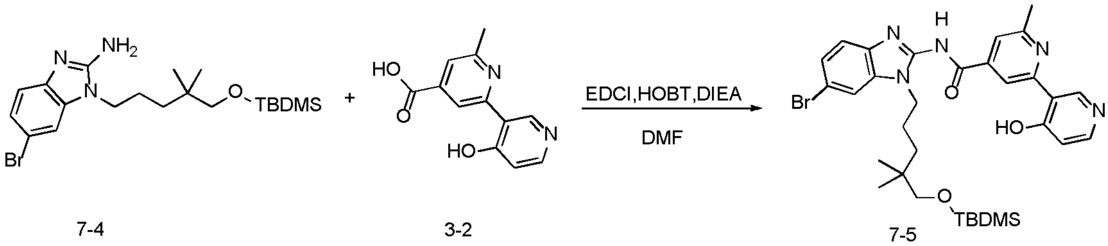


7-4 3-2 7-5

Compound 7-4 (0.90 g, 2.04 mmol), compound 3-2 (0.56 g, 2.45 mmol), EDCI (0.78 g, 4.09 mmol), HOBT (0.55 g, 4.09 mmol), DIEA (1.69 mmL, 10.22 mmol) and DMF (10 mL) were mixed and reacted at 70°C for 30 min. The reaction was monitored by LCMS, and 30 mL ethyl acetate was added, washed with 3 x 20 mL saturated brine, dried over anhydrous sodium sulfate, filtered, and dried to obtain the product compound 7-5 (1.20 g, yield: 90%).MS:652.22[M+H] $^+$

Step 6: Synthesis of compound 7-6

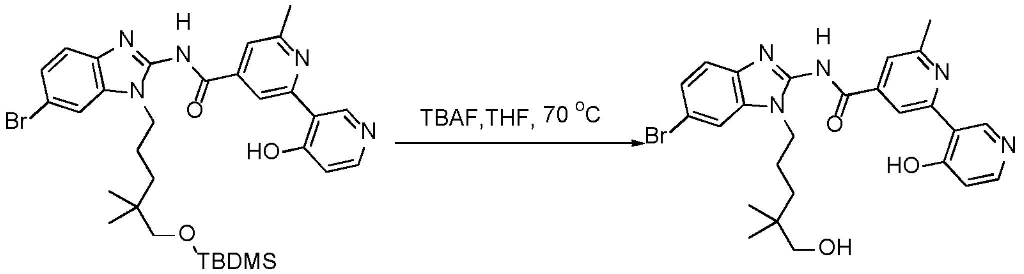


7-5 7-6

Compound 7-5 (900 mg, 1.38 mmol) and TBAF (10 mL, 1M) were mixed evenly and reacted at 70°C for 1 h. The reaction was monitored by LCMS and directly purified by Flash C18 reverse chromatography. The mobile phase (A was 0.05% formic acid aqueous solution; B was MeOH; the gradient was 60-100% B; 20 min). The product compound 7-6 (560 mg, yield: 95%) was obtained by spin drying.MS:538.14[M+H] $^+$

Step 7: Synthesis of compound 7-7

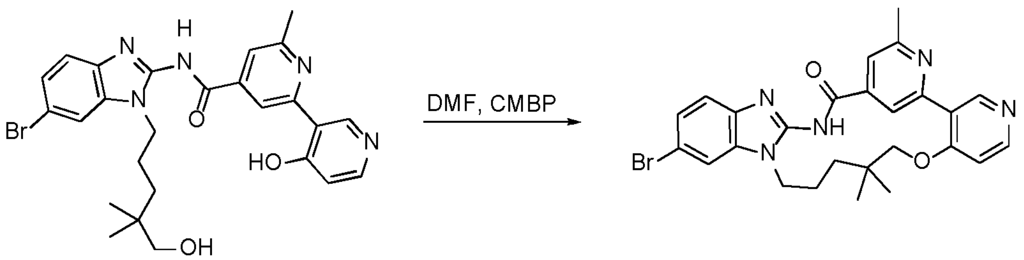


7-6 7-7

Compound 7-6 (560 mg, 1.04 mmol), CMBP (2.51 g, 10.40 mmol) and DMF (10 mL) were mixed evenly and reacted at 130°C for 4 h. The reaction was monitored by LCMS and then directly purified by reverse phase chromatography. Mobile phase A was 0.05% formic acid aqueous solution; B was MeOH; gradient was 60-100% B; 20 min. The product compound 7-7 (120 mg, yield: 22%) was obtained by spin drying. MS:520.13[M+H]$^+$

Step 8: Synthesis of compound 7

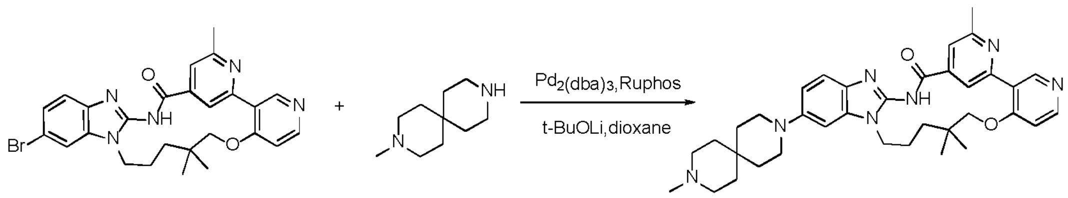


7-7 7

Compound 7-7 (35 mg, 0.07 mmol), 2-methyl-2,7-diazaspiro[3.5]nonane (45.30 mg, 0.27 mmol), $Pd_2(dba)_3$ (25 mg, 0.03 mmol), dioxane (2 mL), Ruphos (19 mg, 0.04 mmol) and lithium tert-butoxide (32 mg, 0.40 mmol) were mixed evenly, protected by nitrogen, and reacted at 100°C for 1 h. Direct Prep-HPLC preparation, chromatographic column luna C18, 30*250 mm, mobile phase (A: 0.1% formic acid aqueous solution, B: acetonitrile; acetonitrile from 17-50%, 20mins), to obtain product compound 7 (26 mg, 0.02 mmol, 60 %).MS:608.36[M+H]$^+$

$^1$H NMR (500 MHz, DMSO-$d_6$) δ 12.50 (s, 1H), 9.11 (s, 1H), 9.03 (s, 1H), 8.46 (d, *J* = 5.7 Hz, 1H), 7.75 (s, 1H), 7.35 (d, *J* = 8.8 Hz, 1H), 7.21 (d, *J* = 5.8 Hz, 1H), 7.12 (d, *J* = 2.1 Hz, 1H), 6.90 (dd, *J* = 8.8, 2.3 Hz, 1H), 4.21 - 4.15 (m, 2H), 3.94 (s, 2H), 3.15 (t, *J* = 5.7 Hz, 4H), 2.64 (s, 3H), 2.46 (s, 4H), 2.27 (s, 3H), 2.11 (dd, *J* = 11.2, 6.2 Hz, 2H), 1.83 (s, 2H), 1.58 (t, *J* = 5.7 Hz, 4H), 1.53 (t, *J* = 5.8 Hz, 4H), 1.23 (s, 2H), 0.90 (s, 6H).

Example 8 Synthesis of Compound 8

*(R)-5$^5$-((dimethyl(oxo)-l$^6$-sulfanylidene)amino)-2$^6$,7-dimethyl-5$^6$-(9-methyl-3,9-di azaspiro[5.5]undecan-3-yl)-5$^1$H-11-oxa-4-aza-5(2,1)-benzo[d]imidazola-1(3,4),2(2,4) -dipyridinacycloundecaphan-3-one*

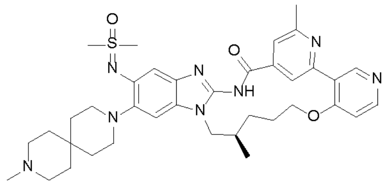

Step 1: Synthesis of compound 8-1

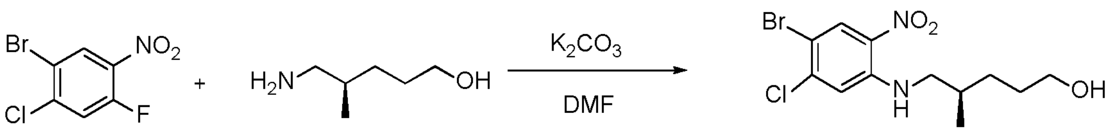


8-1

1-Bromo-2-chloro-4-fluoro-5-nitrobenzene (0.60 g, 2.36 mmol), (R)-5-amino-4-methylpentan-1-ol (0.33 g, 2.83 mmol), potassium carbonate (1.30 g, 9.43 mmol) and DMF (6 mL) were mixed evenly, protected by nitrogen, and reacted at 60°C for 1 h. LCMS monitored the reaction to be complete, cooled to room temperature, poured into 50 mL ice water, filtered, rinsed with 3 x15 mL of water, and the filter cake was dried in vacuo to obtain the product compound 8-1 (0.8 g, 2.27 mmol, yield: 96.48%). MS:351.00, 353.00[M+H] $^+$

Step 2: Synthesis of compound 8-2

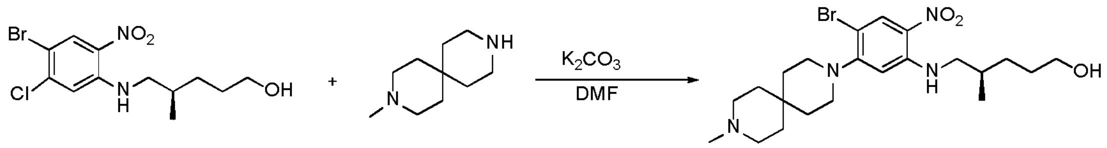


8-1 8-2

Compound 8-1 (0.8 g, 2.27 mmol), 3-methyl-3,9-diazaspiro[5,5]undecane (0.50 g, 2.96 mmol), potassium carbonate (1.26 g, 9.10 mmol) and DMF (8 mL) were mixed evenly, protected by nitrogen, and reacted at 100°C for 18 h. LCMS monitored the reaction to be complete, cooled to room temperature, poured into 50 mL ice water, filtered, rinsed with 3 x15 mL of water, and the filter cake was dried in vacuo to obtain the product compound 8-2 (1.0 g, 2.07 mmol, yield: 96.48%).

MS:483.19, 485.19[M+H] $^+$

Step 3: Synthesis of compound 8-3

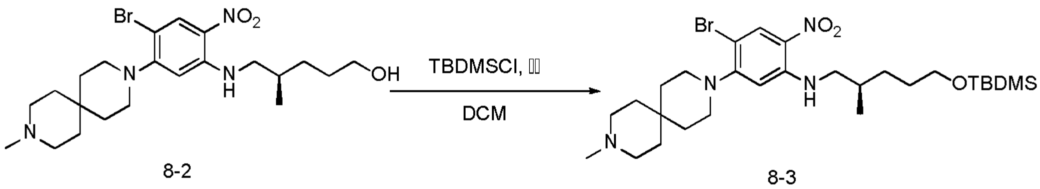


8-2 8-3

Compound 8-2 (1.0 g, 2.07 mmol), imidazole (0.51 g, 7.45 mmol), and DCM (20 mL) were added to the reaction bottle, and TBDMSCl (0.56 g, 3.72 mmol) was added at 0°C. The reaction was continued for 1 h. The reaction was monitored to be complete by LCMS. The product was spin-dried and column chromatography was performed using PE/EA = 1/1 to obtain the product compound 8-3 (1.20 g, 2.00 mmol, yield: 80.88%). MS:597.28,599.28 [M+H] $^+$

Step 4: Synthesis of compound 8-4

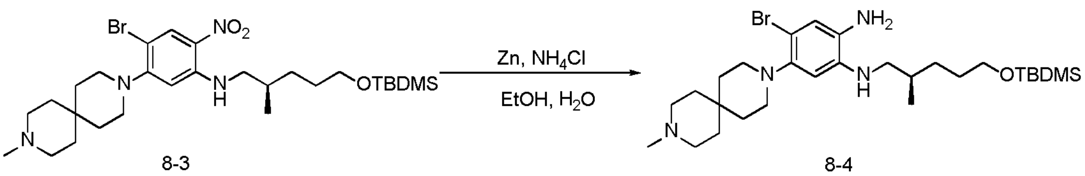


8-3 8-4

Compound 8-3 (1.90 g, 4.40 mmol), zinc powder (1.09 g, 16.73 mmol), ammonium chloride (0.89 g, 16.73 mmol), ethanol (20 mL), and $H_2O$ (10 mL) were mixed evenly and reacted at 55°C for 1 h. The reaction was completed after LCMS monitoring. The mixture was filtered, cooled and dried, and column chromatography with DCM/MeOH=10/1 was performed to obtain the product compound 8-4 (0.95 g, 1.69 mmol, yield: 99%). MS:567.30,569.30[M+H] $^+$

Step 5: Synthesis of compound 8-5

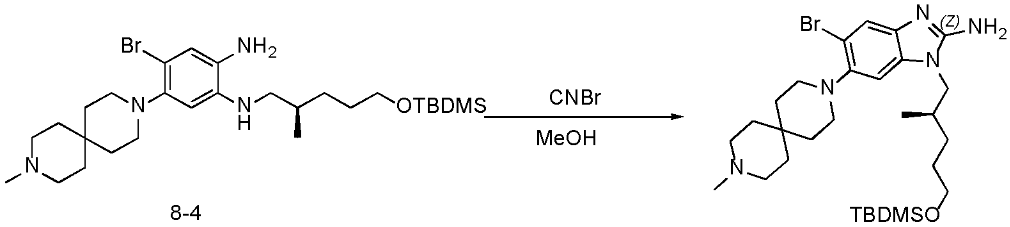


8-4 8-5

Compound 8-4 (0.95 g, 1.69 mmol), cyanogen bromide (0.21 mg, 2.03 mmol), methanol (5 mL) and water (1 mL) were mixed evenly and reacted at 15°C for 1 h. The reaction was completed after LCMS monitoring. The pH was adjusted to 8, and 3 x 50 mL DCM was extracted. The product compound 8-5 (0.90 g, 1.52 mmol, yield: 89.79%) was obtained by spin drying. MS:592.30,594.29[M+H] $^+$

Step 6: Synthesis of compound 8-6

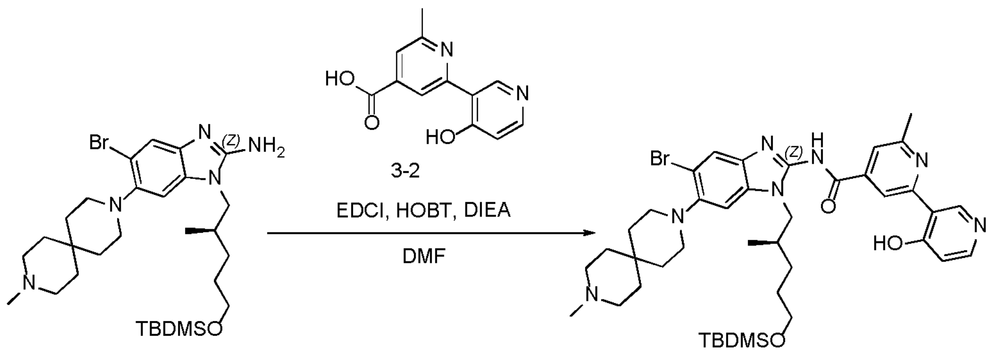


8-5 8-6

Compound 8-5 (0.90 g, 1.52 mmol), 4'-hydroxy-6-methyl-[2,3'-bipyridine]-4-carboxylic acid (0.30 g, 1.29 mmol), EDCI (0.58 g, 3.04 mmol), HOBt (0.41 g, 3.04 mmol), DIEA (0.79 g, 6.07 mmol) and DMF (10 mL) were mixed evenly and reacted at 60 °C for 12 h. The reaction was completed after LCMS monitoring. The reaction system was poured into 500 mL ice water and extracted with 3 x 50 mL DCM. The product compound 8-6 (0.8 g, 0.93 mmol, yield: 65.46%) was obtained by spin drying column chromatography with DCM/MeOH=10/1. MS:804.36,806.35[M+H] $^+$

Step 7: Synthesis of compound 8-7

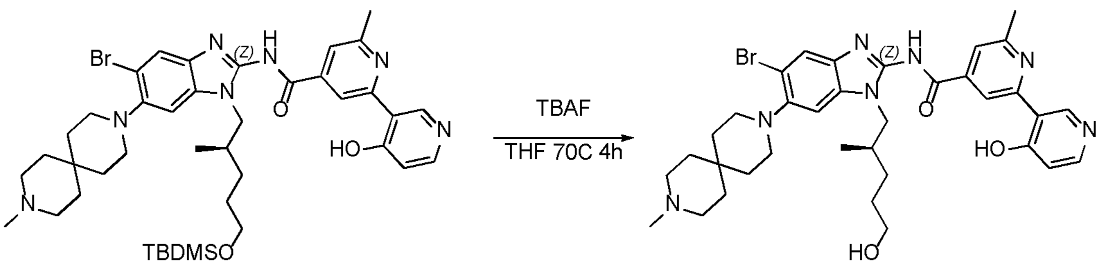


8-6 8-7

Compound 8-6 (0.8 g, 0.93 mmol), TBAF (1.57 mL, 3.98 mmol) and THF (5 mL) were mixed evenly and reacted at 70°C for 4 h. The reaction was completed under LCMS monitoring. The mixture was spin-dried, slurried with 5 mL water for 3 times, filtered, rinsed with 3 x 5 mL of water, and the filter cake was vacuum dried to obtain the product compound 8-7 (0.5 g, 0.72 mmol, yield: 72.83%). MS:690.27,692.27[M+H] $^+$

Step 8: Synthesis of compound 8-8

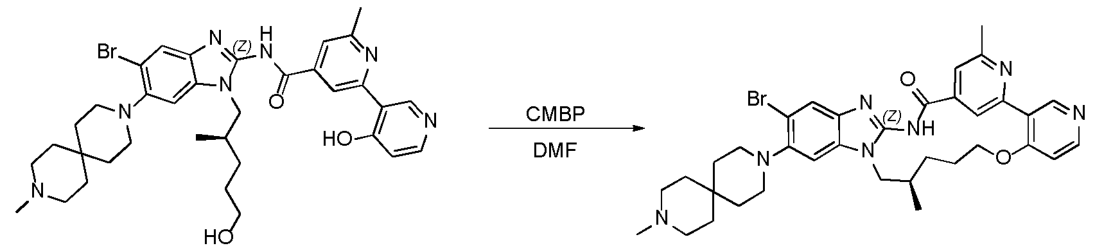


8-7 8-8

Compound 8-7 (0.5 g, 0.72 mmol), CMBP (2.62 g, 10.86 mmol) and DMF (10 mL) were mixed evenly and reacted at 100°C for 4 h. The reaction was completed after monitoring by LCMS. The mixture was spin-dried and column chromatography with DCM/MeOH=10/1 was performed to obtain the product compound 8-8 (300 mg, 4.46 mmol, yield: 61.61%).MS:672.26,674.26[M+H] $^+$

Step 9: Synthesis of compound 8

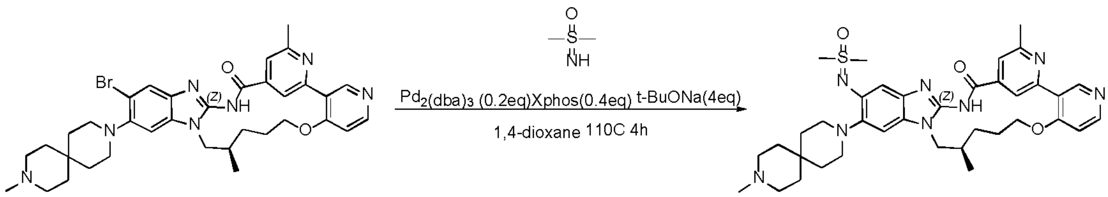


8-8 8

Compound 8-8 (300 mg, 4.46 mmol), dimethylsulfenyl imide (82.95 mg, 0.90 mmol), $Pd_2(dba)_3$ (108.5 mg, 0.12 mmol), Xphos (112.98 mg, 0.24 mmol), t-BuONa (170.8 mg, 1.76 mmol) and 1,4-dioxane (5 mL) were mixed evenly and reacted at 110°C for 4 h. The reaction was completed under the control of LCMS, and the mixture was spin-dried and prepared by Prep-HPLC using a chromatographic column luna C18, 30*250 mm and a mobile phase (A: 0.1% formic acid aqueous solution, B: acetonitrile; acetonitrile was adjusted from 17-40%, 20 mins) to give the product compound 8 (30 mg, 0.59 mmol, yield: 6.09%).
MS:685.36,686.36[M+H] $^+$

<u>Example 9 Synthesis of Compound 9</u>

*(R)-1$^6$-fluoro-2$^6$,7-dimethyl-5$^6$-(3,9-diazaspiro[5.5]undecan-3-yl)-5$^1$H-11-oxa-4-aza-5(2,1)-benzo[d]imidazola-1(3,4),2(2,4)-dipyridinacycloundecaphan-3-one*

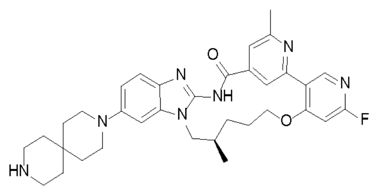

Step 1: Synthesis of compound 9-1

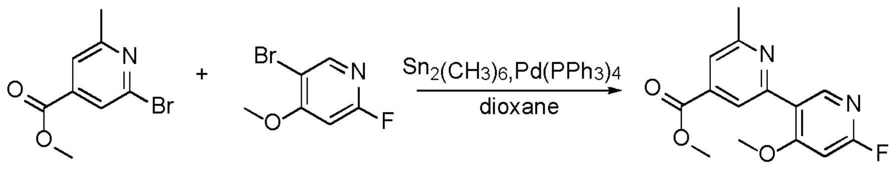


9-1

2-Bromo-6-methylisonicotinic acid methyl ester (3.50 g, 15.21 mmol), hexamethyltin (5.48 g, 16.73 mg), tetrakistriphenylphosphine palladium (1.76 g, 1.52 mmol) and dioxane (20 mL) were mixed evenly, nitrogen protection, reacted at 100 ° C for 40 min, 5-bromo-2-fluoro-4-methoxypyridine (2.82 g, 13.69 mmol) were added, kept warm overnight, LCMS monitored the reaction to be complete. Cooled to room temperature, spin dried, column chromatography PE/EA = 2/1 to obtain the product compound 9-1 (1.70 g, yield: 40%).MS:277.09[M+H]$^+$

Step 2: Synthesis of compound 9-2

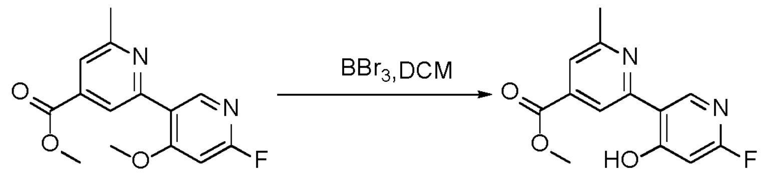


9-1 9-2

Compound 9-1 (1.70 g, 6.15 mmol) was added to a DCM solution of $BBr_3$ (30 mL, 1M) and reacted for 10 min in an ice bath. 30 mL water was added, 3 x 20 mL of DCM was extracted, dried over anhydrous sodium sulfate, filtered, and dried to obtain a crude product, compound 9-2 (2.0 g). MS of compound 9-2: 263.08 [M+H] $^+$

Step 3: Synthesis of compound 9-3

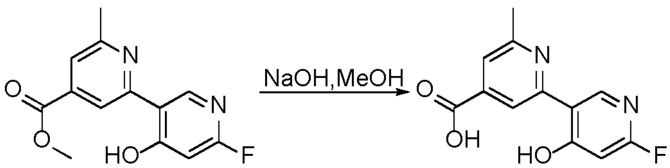


9-2 9-3

Compound 9-2 (1.60 g, 6.10 mmol) was dissolved in MeOH (30 mL), and a solution of NaOH (1.22 g, 30.51 mmol) in water (20 mL) was added at room temperature. The mixture was kept warm for 30 min and the reaction was completed under the monitoring of LCMS. The organic solvent was dried by spin drying, and the aqueous phase was extracted with 20 mL DCM. The pH of the aqueous phase was adjusted to 4, filtered, and dried under vacuum to obtain the product compound 9-3 (1.00 g, 66%). MS of compound 9-3:249.06[M+H]$^+$

Step 4: Synthesis of compound 9-7

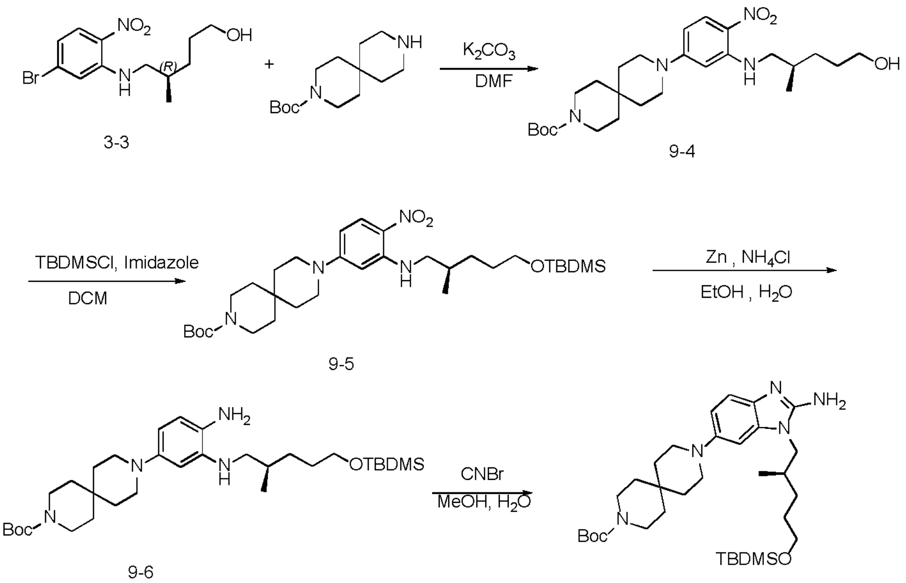


3-3 9-4 9-5 9-6 9-7

The preparation of compound 9-7 adopts the same synthesis method as compound 8-5. MS of compound 9-7:600.42 [M+H]$^+$

Step 5: Synthesis of compound 9-8

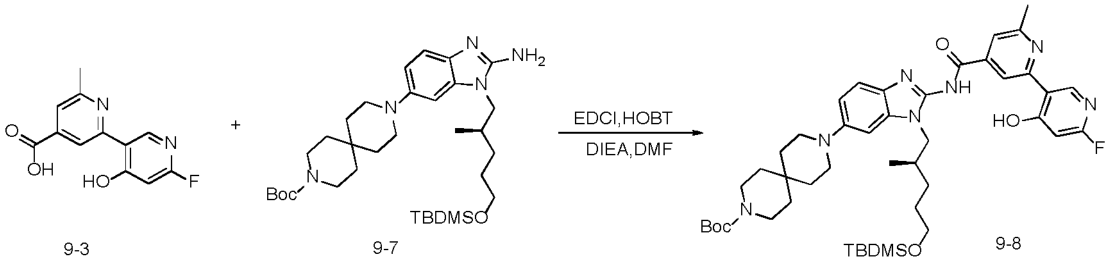


9-3 9-7 9-8

Compound 9-3 (0.52 g, 2.10 mmol), compound 9-7 (1.20 g, 2.00 mmol), EDCI (0.96 g, 5.00 mmol), HOBT (0.68 g, 5.00 mmol), DIEA (1.29 g, 10.00 mmol) and DMF (15 mL) were mixed evenly and reacted at 70 °C for 30 min. LCMS monitored the reaction to be complete, 30 mL ethyl acetate was added, the mixture was washed with saturated brine, dried over anhydrous sodium sulfate, filtered, and dried to obtain the crude product compound 9-8. MS of compound 9-8: 830.47 [M+H]$^+$

Step 6: Synthesis of compound 9-9

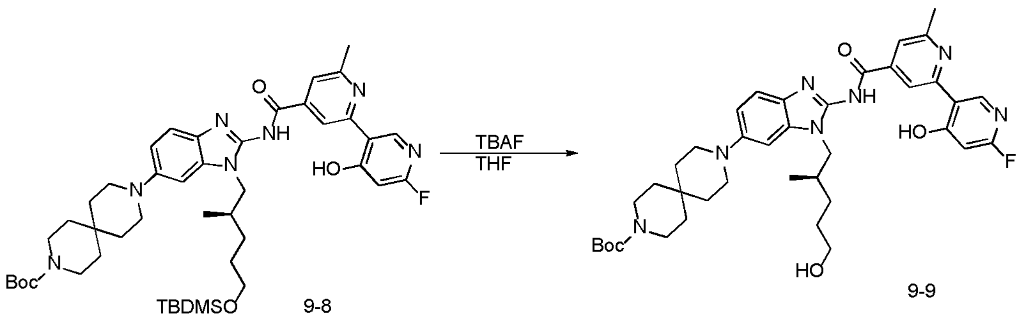


9-8 9-9

The preparation of compound 9-9 adopts the same synthesis method as compound 5-7, except that the raw material compound 5-6 is replaced by compound 9-8. MS of compound 9-9: 716.39 [M+H] $^+$

Step 7: Synthesis of Compound 9-10

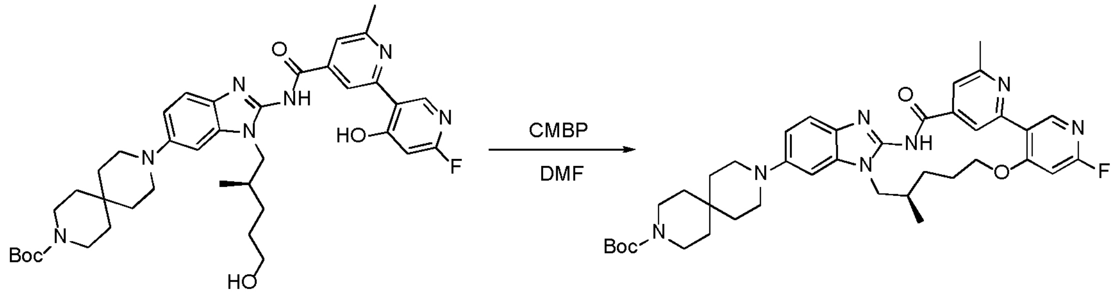


9-9 9-10

The preparation of compound 9-10 adopts the same synthesis method as compound 5-8, except that the raw material compound 5-7 is replaced by compound 9-9. MS of compound 9-10: 698.38 [M+H] $^+$

Step 8: Synthesis of compound 9

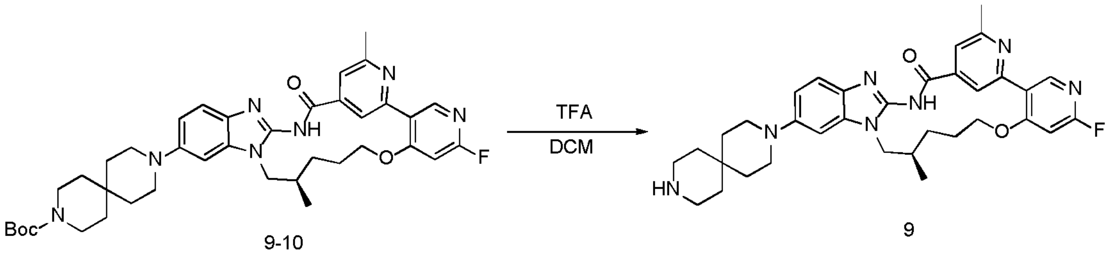


9-10 9

Compound 9-10 (1.20 g, 1.72 mmol) was dissolved in DCM (10 mL), TFA (3 mL) was added at room temperature, and the reaction was carried out at room temperature for 1 h. The reaction was completed after monitoring by LCMS. Saturated aqueous sodium carbonate solution was added to adjust the pH to 8, and the mixture was extracted with a mixed solvent of 3 x 20 mL DCM/MeOH (10/1), dried over anhydrous sodium sulfate, filtered, and dried to obtain the product compound 9.
MS: 598.32[M+H] $^+$

<u>Example 10 Synthesis of Compound 10</u>

*(R)-1$^6$-fluoro-2$^6$,7-dimethyl-5$^6$-(9-methyl-3,9-diazaspiro[5.5]undecan-3-yl)-5$^1$H-11-oxa-4-aza-5(2,1)-benzo[d]imidazola-1(3,4),2(2,4)-dipyridinacycloundecaphan-3-o ne*

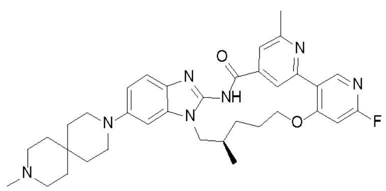

Step 1: Synthesis of compound 10

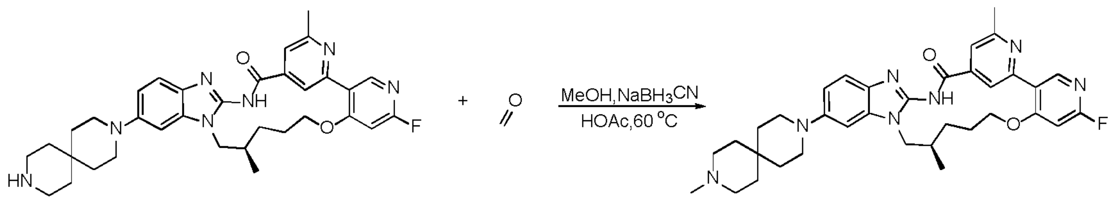


9

10

Compound 9 (20 mg, 0.03 mmol), 0.1 mL formaldehyde aqueous solution, 1 drop of HOAc and MeOH (2 mL) were mixed evenly, $NaBH_3CN$ (4 mg, 0.06 mmol) was added at room temperature, and reacted at 60°C for 1 h. The reaction solution was directly prepared by Prep-HPLC, chromatographic column luna C18, 30*250 mm, mobile phase (A: 0.1% formic acid aqueous solution, B: acetonitrile; acetonitrile from 17-50%, 20 mins), to obtain the product. MS of compound 10: 612.34 [M+H]+

Example 11 Synthesis of Compound 11

*(R)-2$^6$,5$^5$,7-trimethyl-5$^6$-(9-methyl-3,9-diazaspiro[5.5]undecan-3-yl)-5$^1$H-11-oxa -4-aza-5(2,1)-benzo[d]imidazola-1(3,4),2(2,4)-dipyridinacycloundecaphan-3-one*

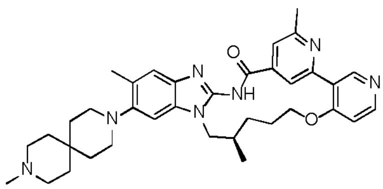

The synthesis method of compound 11 is the same as that of compound 5, except that 4-bromo-2,5-difluoronitrobenzene is replaced by 2-bromo-4-fluoro-5-nitrotoluene. The synthesis route is as follows. The MS of compound 11 is 608.36 [M+H] $^+$

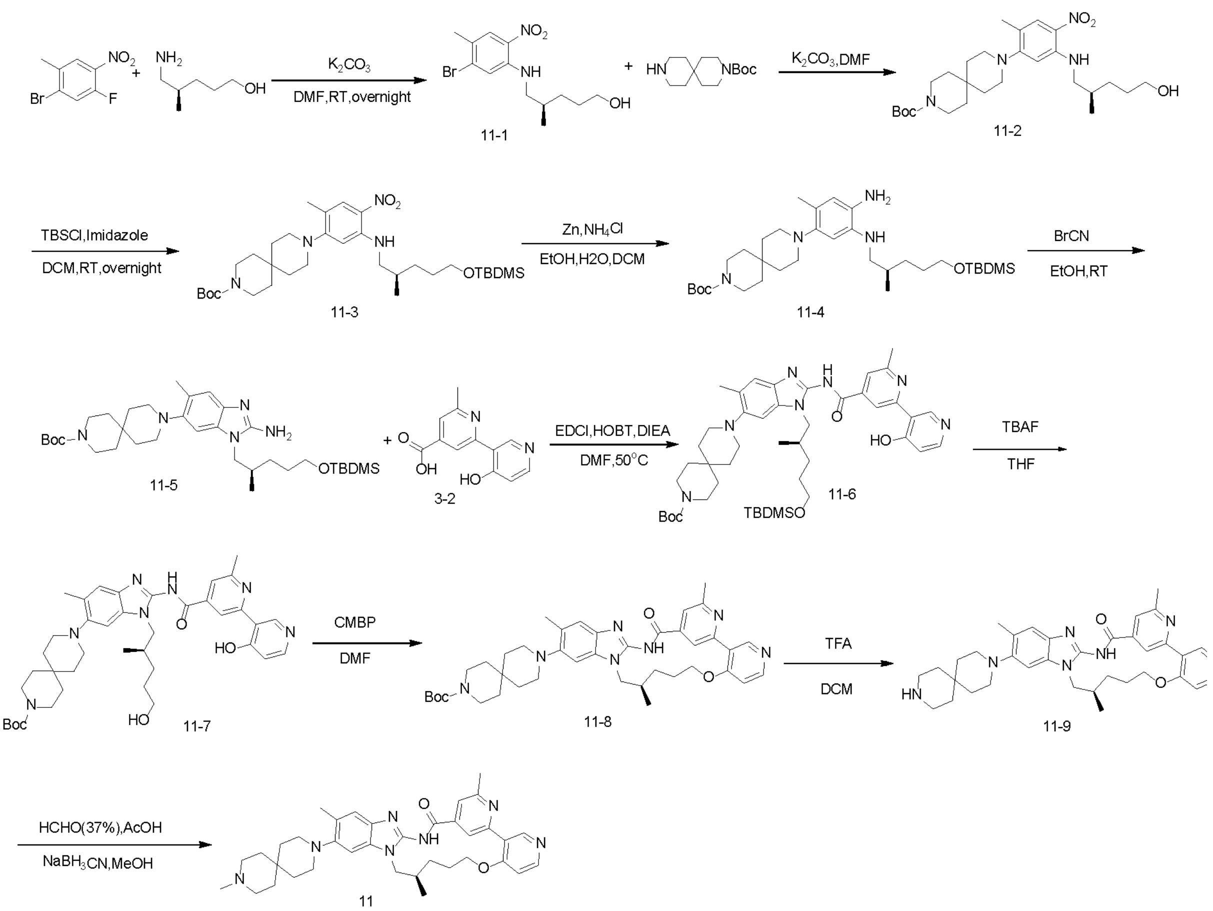


Example 12 Synthesis of Compound 12

*(R)-$2^6$,$5^5$,7-trimethyl-$5^6$-(9-(oxetan-3-yl)-3,9-diazaspiro[5.5]undecan-3-yl)-$5^1$H-1 1-oxa-4-aza-5(2,1)-benzo[d]imidazola-1(3,4),2(2,4)-dipyridinacycloundecaphan-3-on e*

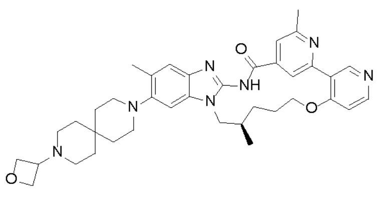

Step 1: Synthesis of compound 12

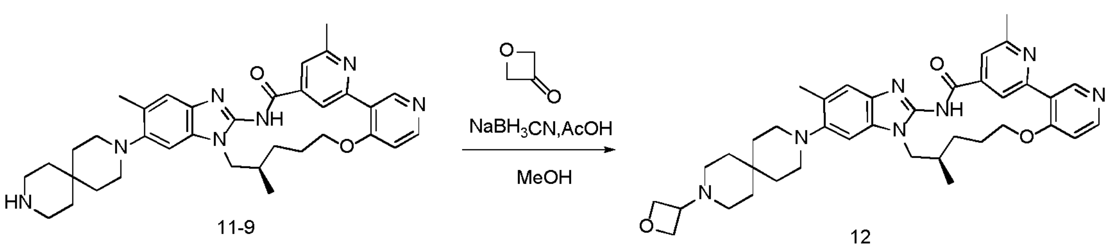


The synthesis method of compound 12 is the same as compound 6, except that compound 5-9 is replaced by

compound 11-9. MS of compound 12: 650.37 [M+H] +

Example 13 Synthesis of Compound 13

*2⁶,5⁵-dimethyl-5⁶-(9-methyl-3,9-diazaspiro[5.5]undecan-3-yl)-5¹H-11-oxa-4-aza -5(2,1)-benzo[d]imidazola-1(3,4),2(2,4)-dipyridinacycloundecaphan-3-one*

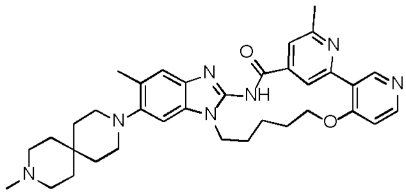

Step 1: Synthesis of compound 13-1

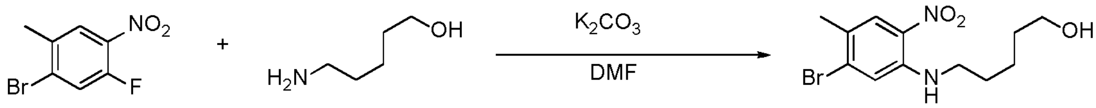


13-1

In a 250 mL single-necked flask, 2-bromo-4-fluoro-5-nitrotoluene (5 g, 21.0 mmol) was dissolved in DMF (50 mL). Potassium carbonate (5.81 g, 42.0 mmol) and 5-aminopentane-1-ol (2.38 g, 23.10 mmol) were added, and the reaction was allowed to proceed overnight at room temperature. After the disappearance of the starting material as monitored by TLC, the reaction solution was poured into 300 mL water, and a large amount of orange-red solid precipitated. The solid was filtered, rinsed with 100 mL x2 of water, and the filter cake was dried under vacuum to obtain the product compound 13-1 (6.1 g, yield: 92%).MS:317.04, 319.03[M+H] +

Step 2: Synthesis of compound 13

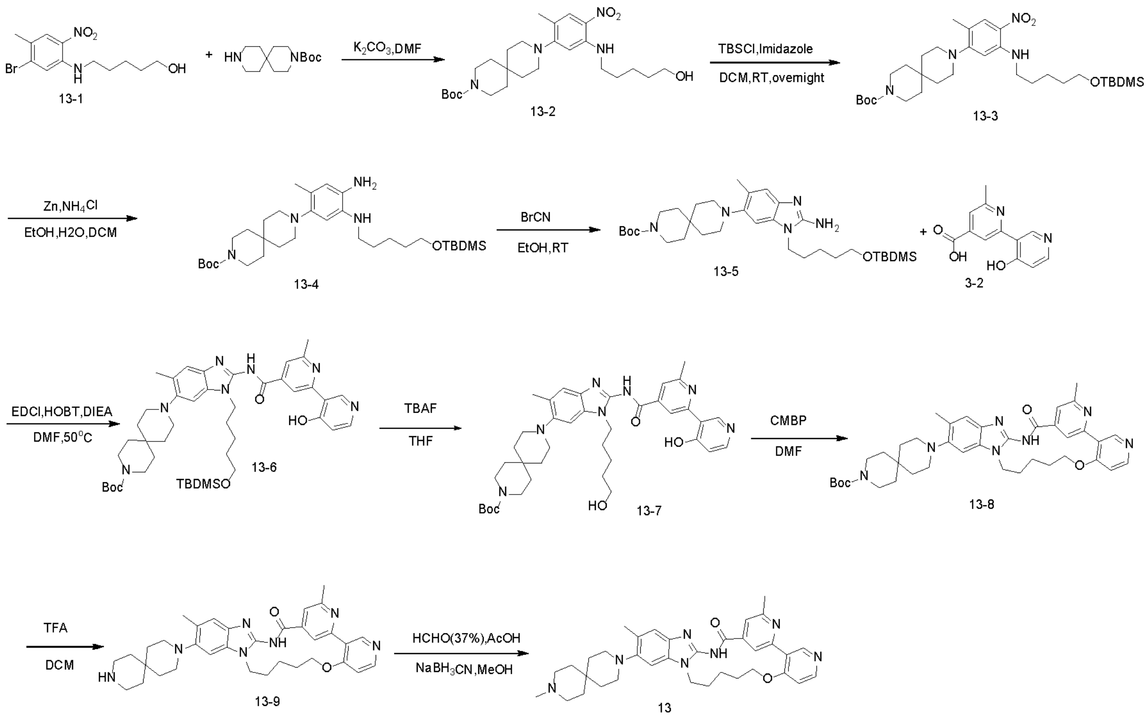


The other synthesis steps of compound 13 are the same as those of compound 11, except that compound 11-1 is replaced by compound 13-1. MS of compound 13: 594.35 [M+H] $^+$.

Example 14 Synthesis of Compound 14

*5⁵-fluoro-2⁶-methyl-5⁶-(9-methyl-3,9-diazaspiro[5.5]undecan-3-yl)-5¹H-11-oxa-4-aza-5(2,1)-benzo[d]imidazola-1(3,4),2(2,4)-dipyridinacycloundecaphan-3-one*

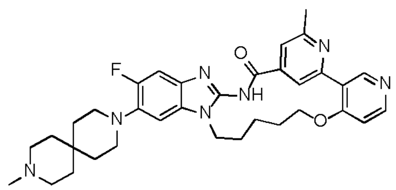

The synthesis steps of compound 14 are the same as those of compound 13, except that 2-bromo-4-fluoro-5-nitrotoluene is replaced by 4-bromo-2,5-difluoronitrobenzene. MS of compound 14: 598.32 [M+H] $^+$.

$^1$H NMR (500 MHz, DMSO-$d_6$) δ 12.62 (s, 1H), 9.24 (s, 1H), 9.04 (s, 1H), 8.46 (d, *J* = 5.6 Hz, 1H), 7.71 (s, 1H), 7.28 (t, *J* = 9.1 Hz, 2H), 7.20 (d, *J* = 5.8 Hz, 1H), 4.28 (s, 2H), 4.20 (s, 2H), 2.98 (t, *J* = 5.3 Hz, 4H), 2.64 (s, 3H), 2.56 (s, 4H), 2.34 (s, 3H), 2.01 (s, 2H), 1.91 (s, 4H), 1.59 (dt, *J* = 19.8, 5.4 Hz, 8H).

Example 15 Synthesis of Compound 15

*5⁶-(9-((3S,4R)-3-fluoro-4-methoxypiperidin-1-yl)-3-azaspiro[5.5]undecan-3-yl)-2⁶-methyl-5¹H-11-oxa-4-aza-5(2,1)-benzo[d]imidazola-1(3,4),2(2,4)-dipyridinacyclou ndecaphan-3-one*

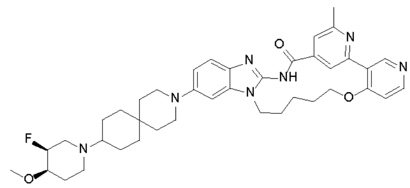

Step 1: Synthesis of compound 15-1

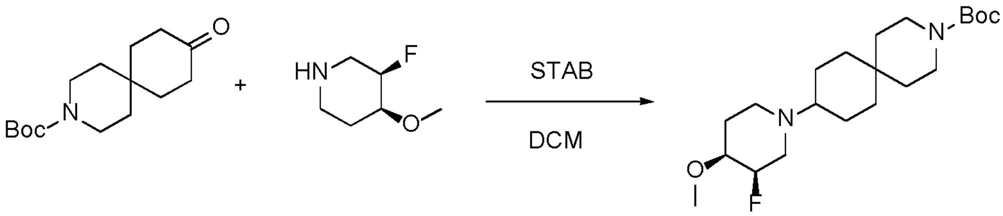


15-1

In a 50 mL single-necked flask, 3-Boc-9-oxo-3-azaspiro[5.5]undecane (0.50 g, 1.87 mmol) was dissolved in DCM (10 mL). (3R, 4S)-3-fluoro-4-methoxypiperidine (0.25 g, 1.87 mmol) and sodium triacetylborohydride (1.19 g, 5.61 mmol) were added. The reaction was allowed to proceed overnight at room temperature. The reaction of the raw materials was completed by LCMS monitoring. The reaction solution was poured into 20 mL sodium bicarbonate aqueous solution, the liquids were separated, the organic phase was concentrated, and column chromatography with DCM:MeOH=30:1-10:1 was performed to obtain the product 15-1 (0.62 g, yield: 86%).MS:385.28[M+H] $^+$

Step 2: Synthesis of compound 15-2

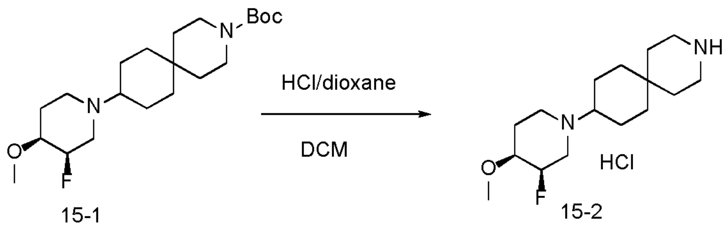


15-1 15-2

The synthesis method of compound 15-2 is the same as that of compound 4-2, except that compound 4-1 is replaced by compound 15-1. MS of compound 15-2: 285.23 [M+H] $^+$

Step 3: Synthesis of compound 15

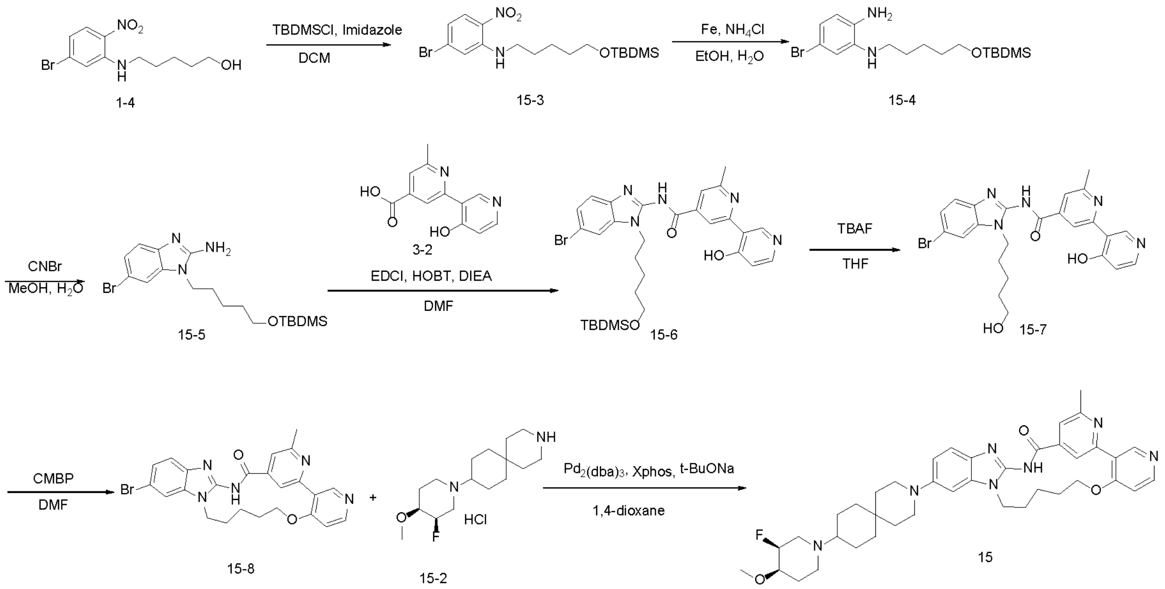


The synthesis method of compound 15 is the same as that of compound 3, except that compound 3-3 is replaced by compound 1-4. MS of compound 15: 696.40 [M+H] $^+$

Example 16 Synthesis of Compound 16

*(R)-1$^6$,5$^5$-difluoro-5$^6$-(9-(3-methoxypropyl)-3,9-diazaspiro[5.5]undecan-3-yl)-26, 7-dimethyl-5$^1$H-11-oxa-4-aza-5(2,1)-benzo[d]imidazola-1(3,4),2(2,4)-dipyridinacyclo undecaphan-3-one*

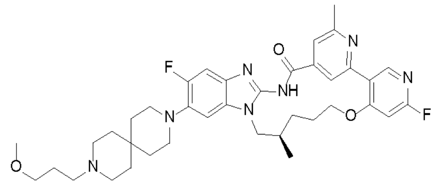

Step 1: Synthesis of compound 16

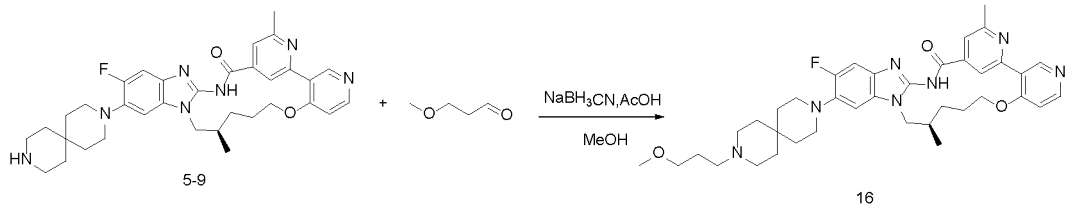


The synthesis method of compound 16 is the same as compound 6, except that 3-oxetanone is replaced by 3-methoxypropanal. MS of compound 16: 688.37 [M+H] $^+$

Example 17 Synthesis of Compound 17

*1$^6$-fluoro-5$^6$-(9-(3-methoxypropyl)-3,9-diazaspiro[5.5]undecan-3-yl)-2$^6$-methyl-5 $^1$H-11-oxa-4-aza-5(2,1)-benzo[d]imidazola-1(3,4),2(2,4)-dipyridinacycloundecaphan-3-one*

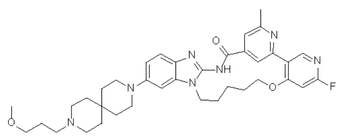

The synthesis method of compound 17 is the same as that of compound 16, except that compound 5-1 is replaced by compound 1-4. The synthesis route is as follows. The MS of compound 17 is 656.36 [M+H] +

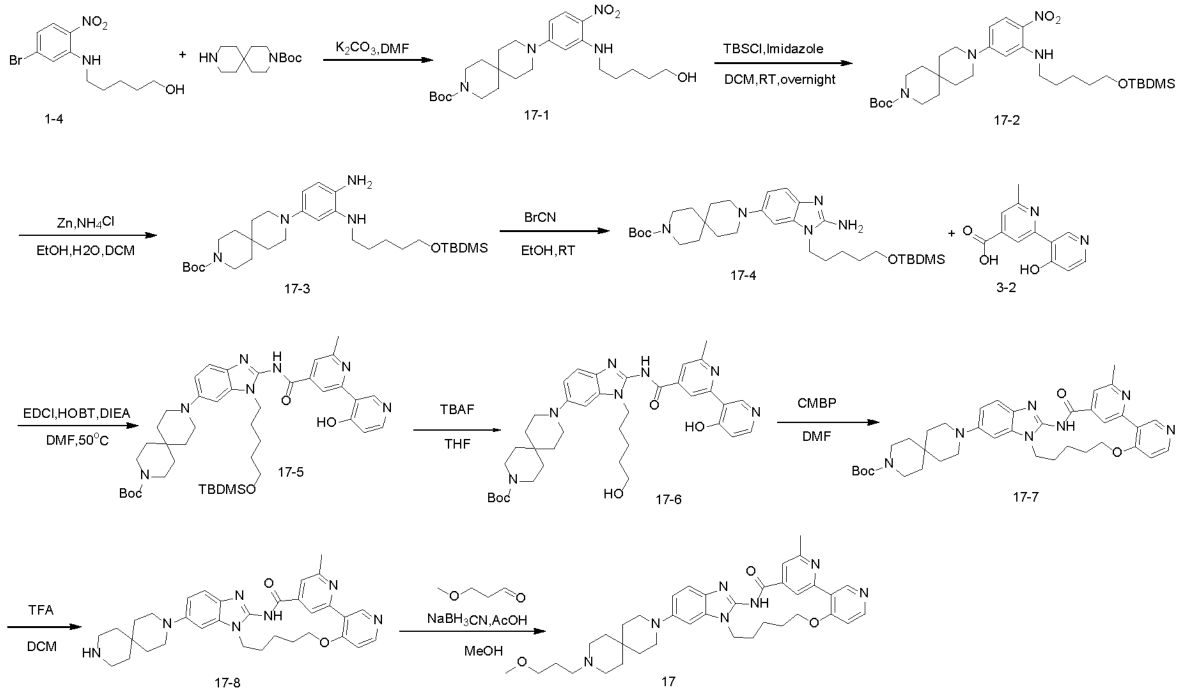


Example 18 Synthesis of Compound 18

*3-(9-(2⁶-methyl-3-oxo-5¹H-11-oxa-4-aza-5(2,1)-benzo[d]imidazola-1(3,4),2(2,4) -dipyridinacycloundecaphane-5⁶-yl)-3,9-diazaspiro[5.5]undecan-3-yl)propanamide*

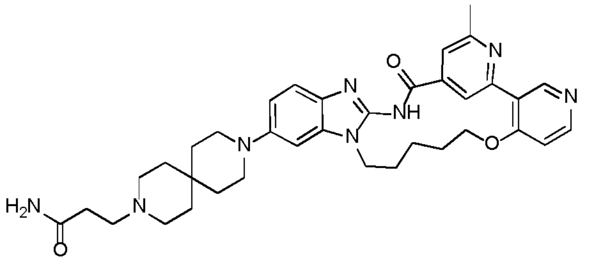

Step 1: Synthesis of compound 18

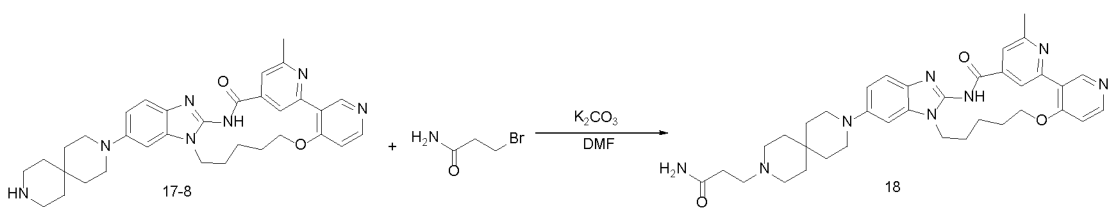

In a 50 mL single-necked flask, compound 17-8 (50 mg, 0.09 mmol) was dissolved in DMF (2 mL). 3-Bromopropionamide (27 mg, 0.18 mmol) and potassium carbonate (40 mg, 0.27 mmol) were added, and the mixture was reacted at 70°C for 2 h under nitrogen protection. The raw material disappeared after LCMS monitoring, and the filtrate was concentrated and prepared by Prep-HPLC, using a chromatographic column luna C18, 30*250 mm, and a mobile phase (A: 0.1% formic acid aqueous solution, B: acetonitrile; acetonitrile is 15-40%, 20 mins), to obtain the product compound 18 (30.8 mg, yield: 55%). MS: 637.35 [M+H] +

Example 19 Synthesis of Compound 19

*(R)-$2^6$,7-dimethyl-$5^6$-(9-methyl-3,9-diazaspiro[5.5]undecan-3-yl)-$5^5$-((2-(methyls ulfonyl)ethyl)amino)-$5^1$H-11-oxa-4-aza-5(2,1)-benzo[d]imidazola-1(3,4),2(2,4)-dipyri dinacycloundecaphan-3-one*

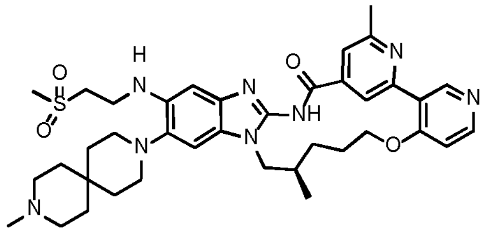

Step 1: Synthesis of compound 19-1

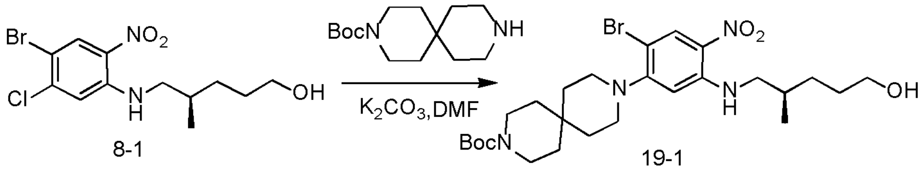


8-1

19-1

In a 100 mL reaction bottle, compound 8-1 (5.60 g, 16.00 mmol), tert-butyl 3,9-diazaspiro[5.5]undecane-3-carboxylate (4.88 g, 19.19 mmol), potassium carbonate (4.42 g, 31.99 mmol) and DMF (25 mL) were mixed evenly, heated to 100 °C for 8 h, cooled, 100 mL EA was added to the reaction solution, 50 mL water was used for extraction, and the organic layer was separated. The organic layer was washed with saturated brine (50 mL*3), the organic layer was dried, filtered, and the filtrate was concentrated. The residue was separated and purified by column chromatography. The eluent was DCM:MeOH=20:1. The eluate was concentrated to obtain the product compound 19-1 (9.11 g, yield: 99%).MS:569.23,571.23 $[M+H]^+$

Step 2: Synthesis of compound 19-2

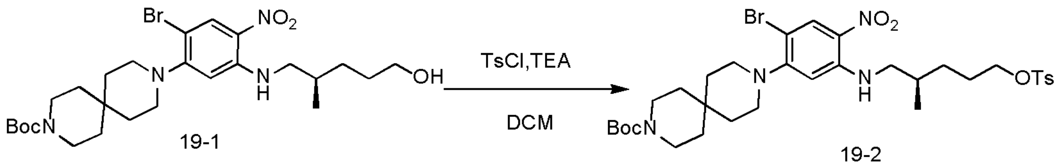


19-1

19-2

Compound 19-1 (9.11 g, 16 mmol), triethylamine (4.45 mL, 32.01 mmol) and DCM (100 mL) were added to the reaction flask. P-Toluenesulfonyl chloride (3.97 g, 20.80 mmol) was added in batches under an ice bath. After the addition, the temperature was raised to room temperature for reaction for 22 h. The reaction was basically complete under LCMS monitoring. The reaction solution was extracted with 100 mL water, separated, and the organic layer was washed with saturated brine (50 mL*2). The organic layer was dried, filtered, and the filtrate was concentrated. The residue was separated and purified by column chromatography. The eluent was PE:DCM=3:1. The eluent was concentrated to obtain

the product compound 19-2 (10.19 g, yield: 88.05%).MS:723.22,723.22 [M+H] +

Step 3: Synthesis of compound 19-3

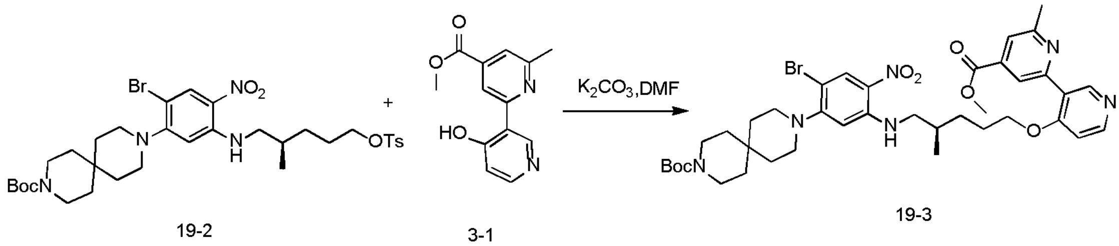


19-2 3-1 19-3

In a 100 mL reaction bottle, compound 19-2 (4.55 g, 6.30 mmol), compound 3-1 (2.00 g, 8.19 mmol), potassium carbonate (1.74 g, 12.60 mmol) and DMF (20 mL) were mixed evenly, heated to 100 °C for 2 h, cooled, 100 mL EA was added to the reaction solution, 50 mL water was extracted, the organic layer was separated, the organic layer was washed with saturated brine (50 mL * 3), the organic layer was dried, filtered, the filtrate was concentrated, and the residue was separated and purified by column chromatography. The eluent was DCM: MeOH = 25: 1. The eluate was concentrated to obtain the product compound 19-3 (3.88 g, yield: 77%). MS:795.30,797.30 [M+H] +

Step 4: Synthesis of compound 19-4

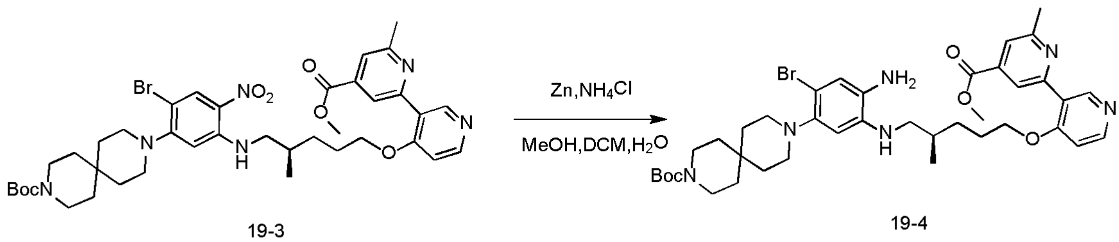


19-3 19-4

In a 100 mL single-necked flask, compound 19-3 (3.76 g, 4.73 mmol) was dissolved in DCM/MeOH/$H_2O$ (20 mL/10 mL/20 mL). Zinc powder (2.47 g, 37.84 mmol) and ammonium chloride (5.06 g, 94.6 mmol) were added under ice bath, and the mixture was heated to room temperature for 3 h. LCMS monitoring showed that the reaction of the raw materials was complete and the reaction was stopped. Post-treatment: Filtered through diatomaceous earth, the filter cake was rinsed with dichloromethane, the filtrate was collected, the layers were separated, the dichloromethane phase was collected, washed twice with water, washed once with saturated brine, dried, filtered, and concentrated to obtain compound 19-4 (3.58 g, yield: 99%). MS: 765.32, 767.32 [M+H]+

Step 5: Synthesis of compound 19-5

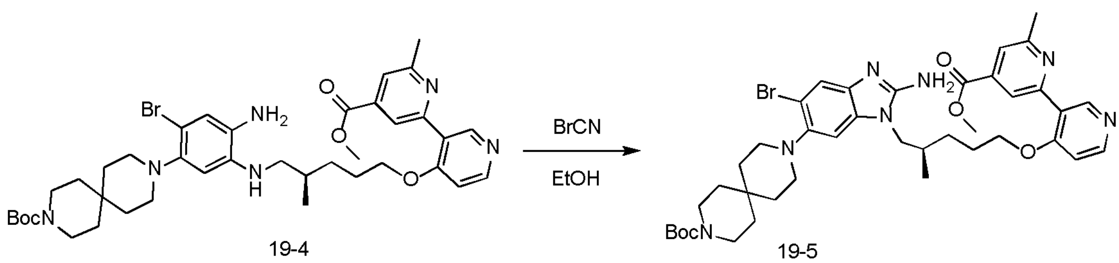


19-4 19-5

In a 100 mL single-necked flask, compound 19-4 (2.15 g, 2.82 mmol) was dissolved in anhydrous ethanol (30 mL), and solid cyanogen bromide (0.31 g, 2.96 mmol) was added under ice bath. The mixture was stirred for 2 h. LCMS

monitored the complete reaction of the raw materials. Aqueous potassium carbonate solution (0.5 N) and DCM were added to the reaction solution, and stirred and separated into layers. The dichloromethane phase was collected, washed twice with water, washed once with saturated brine, dried, filtered, and concentrated. The residue was mixed with silica gel and passed through Flash column chromatography (eluent: DCM: MeOH = 13:1) to obtain compound 19-5 (1.74 g, yield: 79%).MS:790.32, 792.32[M+H]+

Step 6: Synthesis of compound 19-6

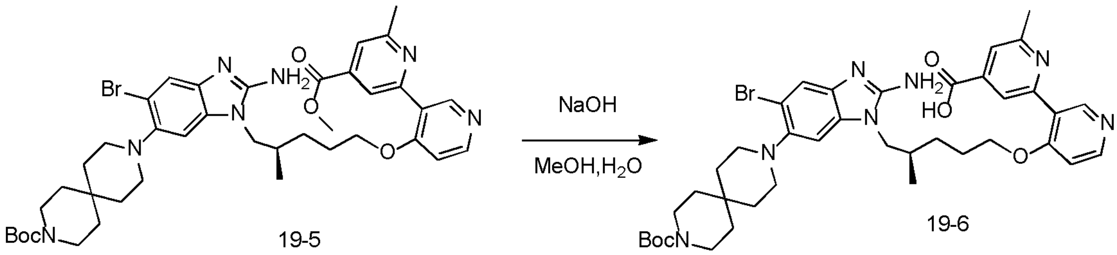


19-5 19-6

Compound 19-5 (1.74 g, 2.21 mmol) was dissolved in methanol (8 mL). Sodium hydroxide (0.18 g, 4.42 mmol) and water (8 mL) were added. The reaction was allowed to react at room temperature for 1 hour. The reaction was completed by LCMS monitoring. The methanol was removed by concentration. The pH was adjusted to 7 with 2 mol/L dilute hydrochloric acid. The system was concentrated. Purification was performed by Flash C18 reverse phase column chromatography (A: 0.1% formic acid aqueous solution, B: acetonitrile; acetonitrile from 0-100%, 20 mins). The product compound 19-6 (1.68 g, yield: 98%) was obtained.MS:776.31, 778.31[M+H] +.

Step 7: Synthesis of compound 19-7

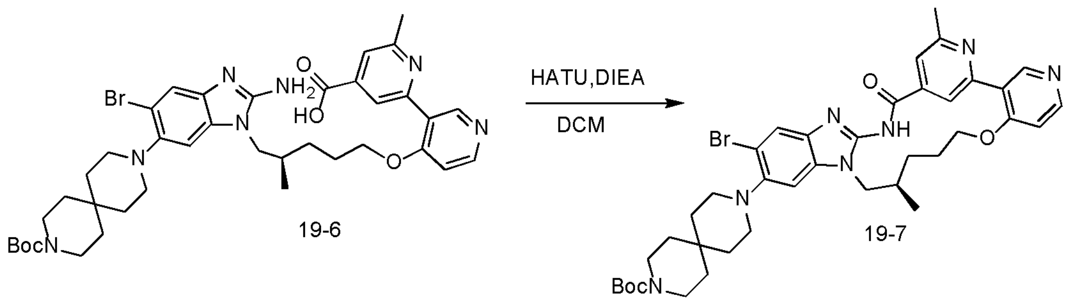


19-6 19-7

Compound 19-6 (1.64 g, 2.11 mmol) was dissolved in DCM (17 mL). Stirred and cooled. HATU (0.84 g, 2.21 mmol) and DIEA (0.70 mL, 4.21 mmol) were added at 0°C. After the addition was completed, the temperature was naturally raised to room temperature for 2 hours. The reaction was completed by LCMS monitoring. 30 mL DCM was added and washed with 20 mL saturated brine. The organic phase was dried, filtered and concentrated. The residue was mixed with silica gel and passed through Flash column chromatography (eluent: DCM: MeOH = 25: 1) to obtain compound 19-7 (1.57 g, yield: 98%). MS: 758.29, 760.30 [M+H]+

Step 8: Synthesis of compound 19-8

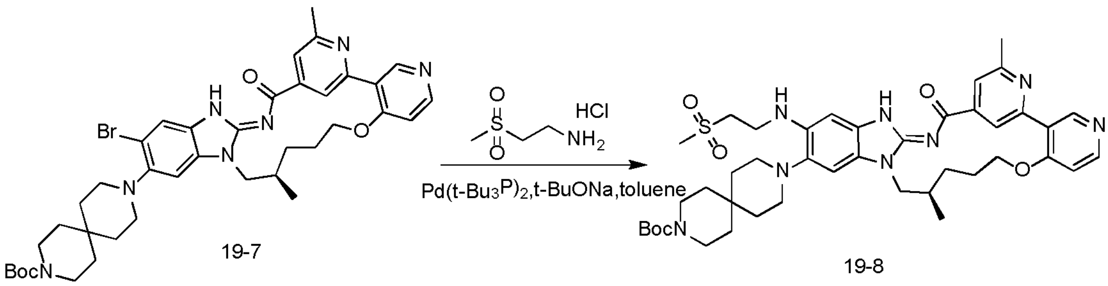


Compound 19-7 (257 mg, 0.34 mmol), 2-methylsulfonylethylamine hydrochloride (108 mg, 0.68 mmol), sodium tert-butoxide (98 mg, 1.02 mmol), di(tri-tert-butylphosphine)palladium (36 mg, 0.07 mmol) and toluene (10 mL) were added to a single-necked flask in sequence, and the atmosphere was replaced with nitrogen three times. Microwave, react at 100 ° C for 2 h. LCMS monitored the reaction. The solvent was evaporated under reduced pressure, and the residue was mixed with silica gel and passed through Flash column chromatography (eluent: DCM: MeOH = 40: 1) to obtain compound 19-8 (166 mg, yield: 61%).MS:801.40[M+H]$^+$

Step 9: Synthesis of compound 19-9

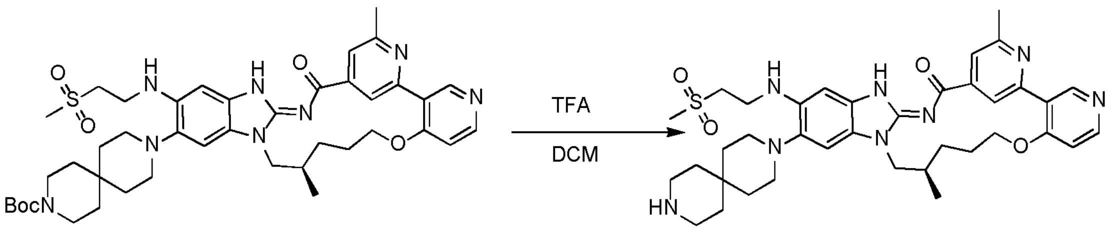


19-8 19-9

Compound 19-8 (128 mg, 0.16 mmol) was added to DCM (4 mL) and stirred to dissolve. Trifluoroacetic acid (2 mL) was added dropwise. After the addition was completed, the mixture was stirred at room temperature for 2 hours. LCMS was used to monitor the reaction until it was complete, and the mixture was concentrated to obtain the crude product 19-9 (124 mg, 111%), which was directly used in the next step.MS: 701.35 [M+H]$^+$

Step 10: Synthesis of compound 19

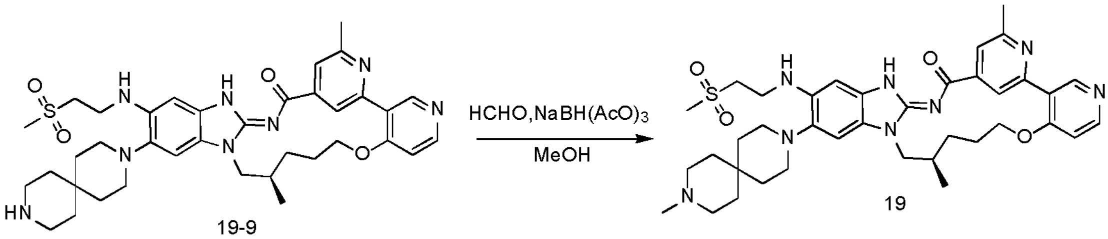


The crude compound 19-9 (100 mg, 0.14 mmol) from the previous step was dissolved in MeOH (5 mL). Formaldehyde (5.09 mg, 0.18 mmol) and sodium triacetoxyborohydride (36 mg, 0.18 mmol) were added in sequence and reacted at room temperature for 1 h. The reaction was monitored by LCMS, and the product compound 19 (21 mg, 20.6%) was obtained using a luna C18 column, 30*250 mm, and a mobile phase (A: 0.1% formic acid aqueous solution, B: acetonitrile; acetonitrile from 17-50%, 20 mins).MS:715.37[M+H]$^+$

<u>Example 20 Synthesis of Compound 20</u>

*(R)-$2^{6}$,7-dimethyl-$5^{6}$-(9-methyl-3,9-diazaspiro[5.5]undecan-3-yl)-$5^{5}$-(methylamin o)-$5^{1}$H-11-oxa-4-aza-5(2,1)-benzo[d] imidazola-1(3,4),2(2,4)-dipyridinacycloundecap han-3-one*

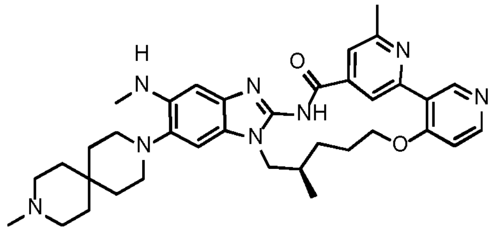

The synthesis method of compound 20 is the same as that of compound 19, except that 2-(methylsulfonyl) ethylamine hydrochloride is replaced by methylamine hydrochloride. The synthesis route is as follows. MS of compound 20: 623.37 [M+H]

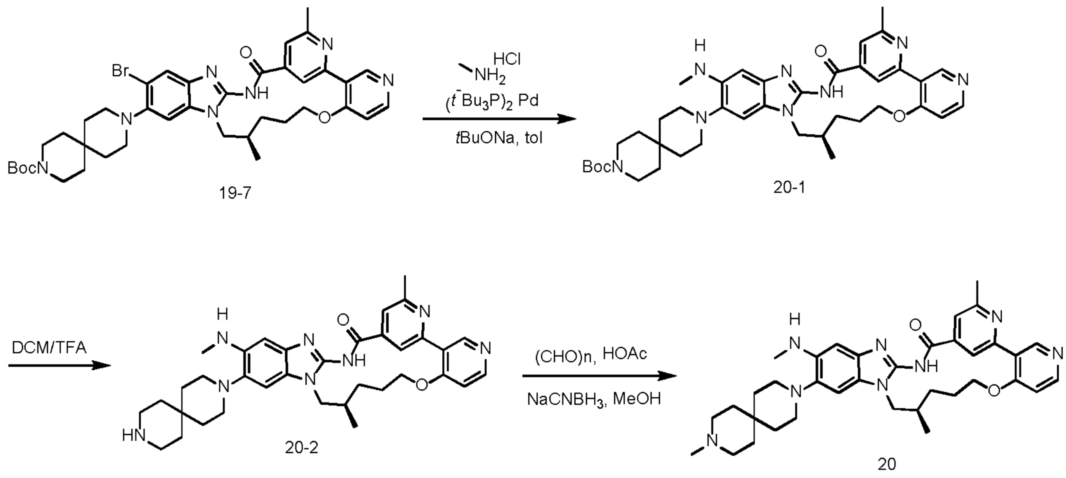


19-7 20-1 20-2 20

Example 21 Synthesis of Compound 21

*(R)-7-methyl-$2^{6}$-(methyl-$d_3$)-$5^{6}$-(9-(oxetan-3-yl)-3,9-diazaspiro[5.5]undecan-3-yl)-$5^{1}$ H-11-oxa-4-aza-5(2,1)-benzo[d] imidazola-1(3,4),2(2,4)-dipyridinacycloundecaphan-3-one*

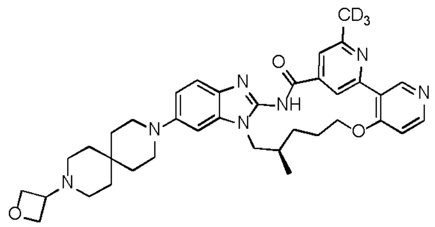

Step 1: Synthesis of compound 21-1

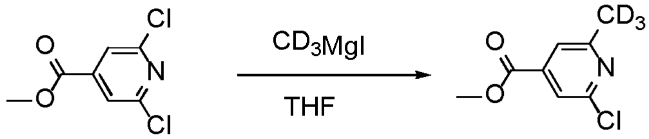


21-1

In a 50 mL single-necked flask, methyl 2,6-dichloroisonicotinate (2.06 g, 10 mmol) was dissolved in THF (10 mL), and deuterated methylmagnesium iodide (1 mol/L) (30 mL, 30 mmol) was added dropwise at -78°C. The mixture was reacted for 2 hours at -20°C. The reaction was completed by LCMS monitoring. The system was quenched by ammonium chloride solution and extracted with ethyl acetate. The mixture was concentrated and purified by column chromatography (PE/EA=5/1). The product compound 21-1 (0.45 g, yield 24%) was obtained. MS: 189.04[M+H] +.

Step 2: Synthesis of compound 21-2

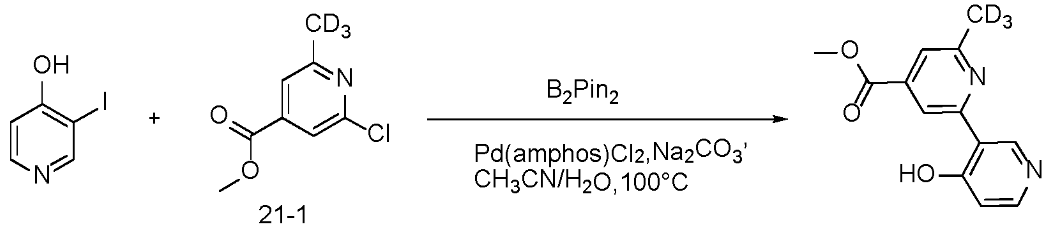


21-1

21-2

4-Hydroxy-3-iodopyridine (15 g, 67.87 mmol), compound 21-1 (25 g, 135.74 mmol), biboronic acid pinacol ester (52 g, 203.61 mmol), Pd(amphos)$Cl_2$ (4.7 g, 6.8 mmol) and sodium carbonate (28 g, 203 mmol) were dispersed in acetonitrile (900 mL) and water (90 mL). Under nitrogen protection, the oil bath was heated to 100 °C for 2 hours. The reaction was completed by LCMS monitoring. Cooled to room temperature. 1 L water was added, the pH was adjusted to neutral with 1N dilute hydrochloric acid, and extracte with 500 mL ethyl acetate three times. Combine the organic phases, dry and concentrate. Purify by column chromatography (DCM/MeOH=10/1). The product compound 21-2 (10 g, yield 62%) was obtained. MS:248.10[M+H] +

Step 3: Synthesis of compound 21-3

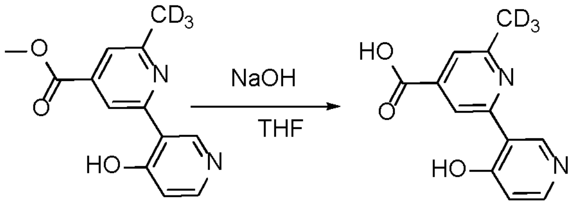


21-2

21-3

The synthesis method of compound 21-3 is the same as that of compound 3-2, except that the raw material is replaced by compound 21-2 from compound 3-1. The MS of compound 21-3 is: 234.09 [M+H] +

Step 4: Synthesis of compound 21-4

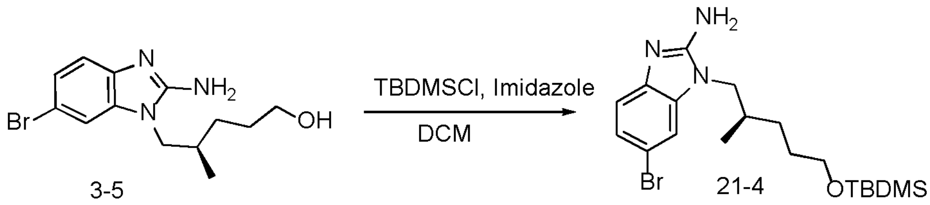


3-5 21-4

The synthesis method of compound 21-4 is the same as that of compound 1-6, except that the raw material is replaced by compound 3-5 from compound 1-5. MS of compound 21-4: 426.15, 428.15 [M+H] $^+$

Step 5: Synthesis of compound 21-5

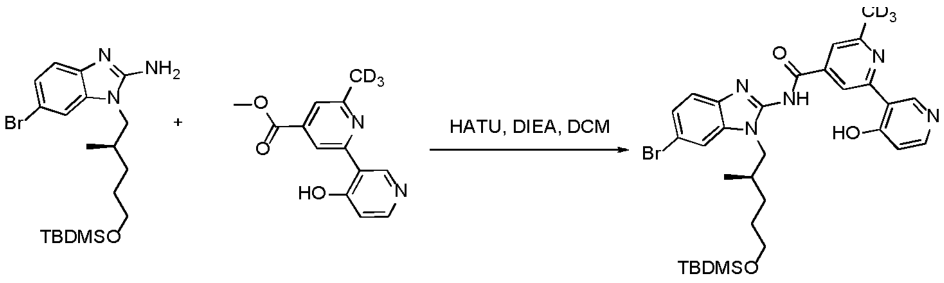


21-4 21-3 21-5

The synthesis method of compound 21-5 is the same as that of compound 1-9, except that the raw materials are replaced by compound 21-3 and compound 21-4 from compound 1-3 and compound 1-8. MS of compound 21-5: 641.23, 643.23 [M+H] $^+$

Step 6: Synthesis of compound 21-6

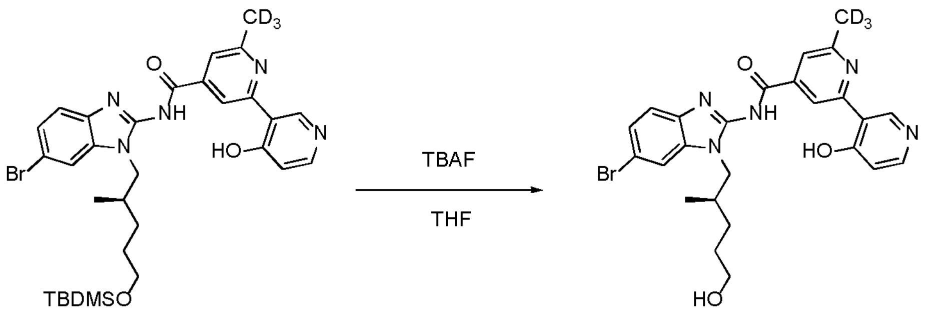


21-5 21-6

The synthesis method of compound 21-6 is the same as that of compound 1-10, except that the raw material is replaced by compound 21-5 from compound 1-9. MS of compound 21-6: 527.14, 529.14 [M+H] $^+$

Step 7: Synthesis of compound 21-7

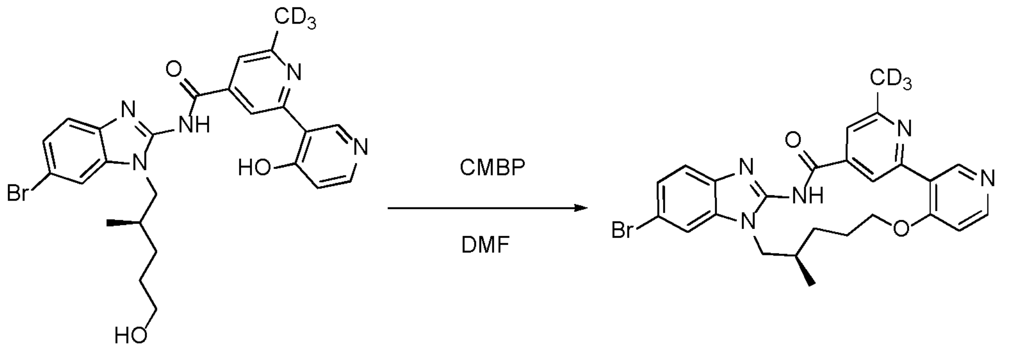


21-6 21-7

The synthesis method of compound 21-7 is the same as that of compound 5-8, except that the raw material 5-7 is replaced by 21-6. The MS of compound 21-7 is 509.13, 511.13 $[M+H]^+$

Step 8: Synthesis of compound 21

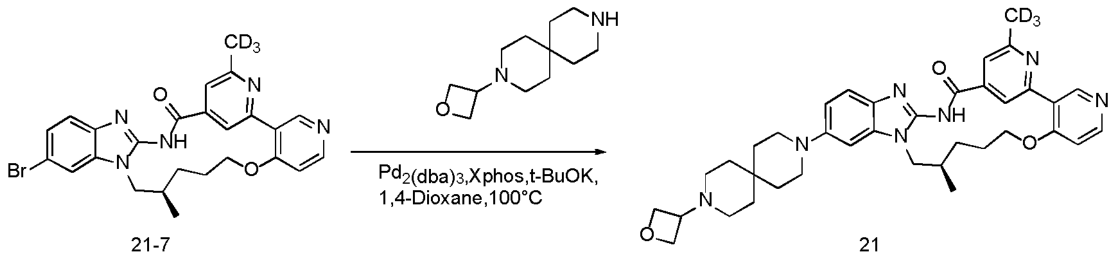


21-7 21

The synthesis method of compound 21 is the same as that of compound 3, except that the raw material is replaced by compound 21-7 from compound 3-7, and the raw material is replaced by 3-(oxetan-3-yl)-3,9-diazaspiro[5.5]undecane from 3-methyl-3,9-diazaspiro[5.5]undecane. The MS of compound 21 is 639.38 $[M+H]^+$

Example 22 Synthesis of Compound 22

*(R)-$1^6$-fluoro-$5^6$-(9-(2-methoxyethyl)-3,9-diazaspiro[5.5]undecan-3-yl)-$2^6$,7-dime thyl-$5^1$H-11-oxa-4-aza-5(2,1)-benzo [d]imidazola-1(3,4),2(2,4)-dipyridinacycloundeca phan-3-one*

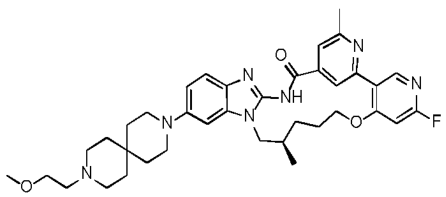

Step 1: Synthesis of compound 22

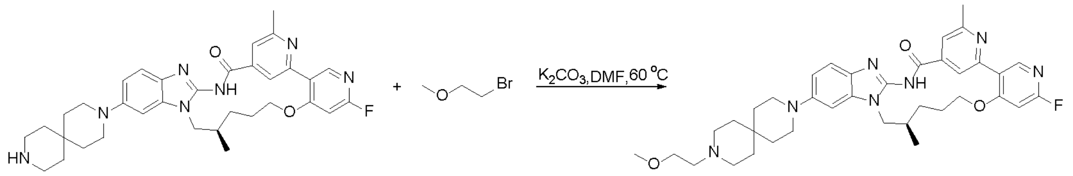


9

22

Compound 9 (50 mg, 0.08 mmol), 1-bromo-2-methoxyethane (14. mg, 0.10 mmol), $K_2CO_3$ (35 mg, 0.25 mmol) and DMF (2 mL) were mixed evenly and reacted at 60°C for 3 h. The reaction was monitored by LCMS. The reaction solution was directly prepared by Prep-HPLC, using a chromatographic column luna C18, 30*250 mm, and a mobile phase (A: 0.1% formic acid aqueous solution, B: acetonitrile; acetonitrile from 17-50%, 20 mins) to obtain the product. MS of compound 22: 656.36 $[M+H]^+$

The following examples shown in Table 1 were prepared in a similar manner to the above compounds.

Table 1

| Example | Structure | Compound name | MS: [M+H]+ |
|---|---|---|---|
| 23 | 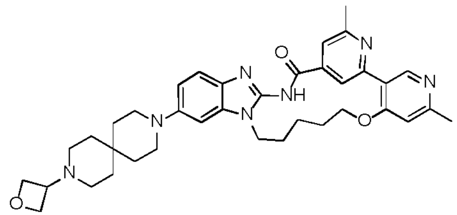 | $1^6$,$2^6$-dimethyl-$5^6$-(9-(oxetan-3-yl)-3,9-diazaspiro[5.5]unde can-3-yl)-$5^1$H-11-oxa-4-aza-5 (2,1)-benzo[d]imidazola-1(3, 4),2(2,4)-dipyridinacyclounde caphan-3-one | 636.36 |
| 24 | 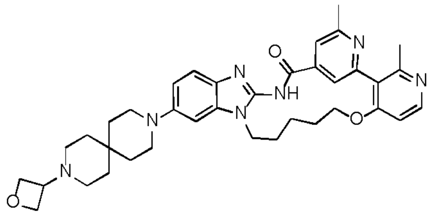 | $1^2$,$2^6$-dimethyl-$5^6$-(9-(oxetan-3-yl)-3,9-diazaspiro[5.5]unde can-3-yl)-$5^1$H-11-oxa-4-aza-5 (2,1)-benzo[d]imidazola-1(3, 4),2(2,4)-dipyridinacyclounde caphan-3-one | 636.36 |
| 25 | 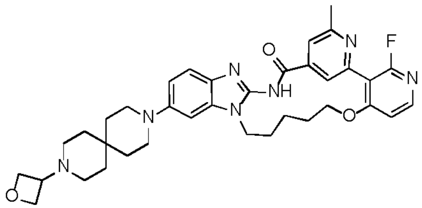 | $1^2$-fluoro-$2^6$-methyl-$5^6$-(9-(ox etan-3-yl)-3,9-diazaspiro[5.5] undecan-3-yl)-$5^1$H-11-oxa-4-aza-5(2,1)-benzo[d]imidazola -1(3,4),2(2,4)-dipyridinacyclo undecaphan-3-one | 640.33 |
| 26 | 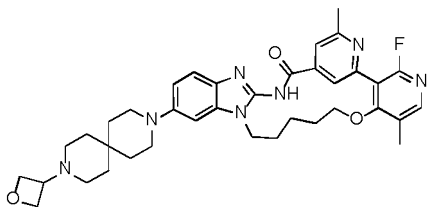 | $1^2$-fluoro-$1^5$,$2^6$-dimethyl-$5^6$-(9 -(oxetan-3-yl)-3,9-diazaspiro[ 5.5]undecan-3-yl)-$5^1$H-11-ox a-4-aza-5(2,1)-benzo[d]imida zola-1(3,4),2(2,4)-dipyridinac ycloundecaphan-3-one | 654.35 |

(continued)

| Example | Structure | Compound name | MS: [M+H]$^+$ |
|---|---|---|---|
| 27 | 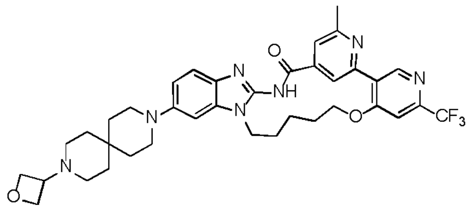 | $2^6$-methyl-$5^6$-(9-(oxetan-3-yl) -3,9-diazaspiro[5.5]undecan-3-yl)-$1^6$-(trifluoromethyl)-$5^1$ H-11-oxa-4-aza-5(2,1)-benzo[ d]imidazola-1 (3,4),2(2,4)-dip yridinacycloundecaphan-3-on e | 690.33 |
| 28 | 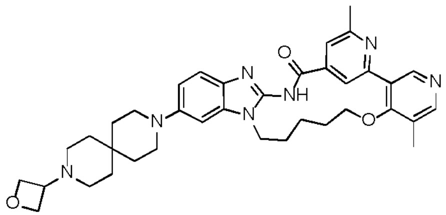 | $1^5$,$2^6$ -dimethyl-$5^6$ -(9-(oxetan-3-yl)-3,9-diazaspiro[5.5]unde can-3-yl)-$5^1$H-11-oxa-4-aza-5 (2,1)-benzo[d]imidazola-1(3, 4),2(2,4)-dipyridinacycloun de caphan-3-one | 636.36 |
| 29 | 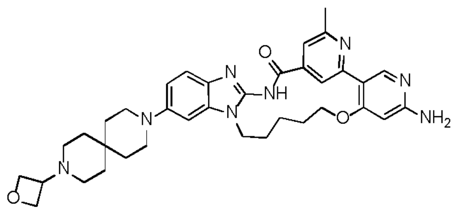 | $1^6$-amino-$2^6$-methyl-$5^6$-(9-(ox etan-3-yl)-3,9-diazaspiro[5.5] undecan-3-yl)-$5^1$H-11-oxa-4-aza-5(2,1)-benzo[d]imidazola -1(3,4),2(2,4)-dipyridinacyclo undecaphan-3-one | 637.35 |
| 30 | 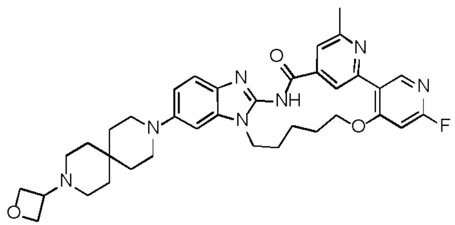 | $1^6$-fluoro-$2^6$-methyl-$5^6$-(9-(ox etan-3-yl)-3,9-diazaspiro[5.5] undecan-3-yl)-$5^1$H-11-oxa-4-aza-5(2,1)-benzo[d]imidazola -1(3,4),2(2,4)-dipyridinacyclo undecaphan-3-one | 640.33 |

(continued)

| Example | Structure | Compound name | MS: $[M+H]^+$ |
|---|---|---|---|
| 31 | 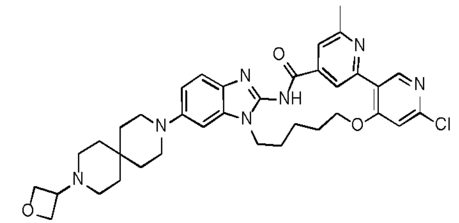 | $1^6$-chloro-$2^6$-methyl-$5^6$-(9-(ox etan-3-yl)-3,9-diazaspiro[5.5] undecan-3-yl)-$5^1$H-11-oxa-4-aza-5(2,1)-benzo[d]imidazola -1(3,4),2(2,4)-dipyridinacyclo undecaphan-3-one | 656.30 |
| 32 | 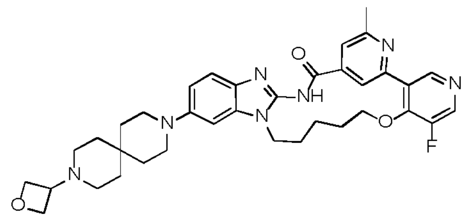 | $1^5$-fluoro-$2^6$-methyl-$5^6$-(9-(ox etan-3-yl)-3,9-diazaspiro[5.5] undecan-3-yl)-$5^1$H-11-oxa-4-aza-5(2,1)-benzo[d]imidazola -1(3,4),2(2,4)-dipyridinacyclo undecaphan-3-one | 640.33 |
| 33 | 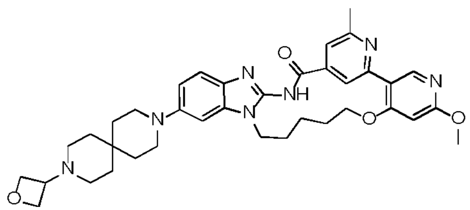 | $1^6$-methoxy-$2^6$-methyl-$5^6$-(9-( oxetan-3-yl)-3,9-diazaspiro[5. 5]undecan-3-yl)-$5^1$H-11-oxa-4-aza-5(2,1)-benzo[d]imidazo la-1(3,4),2(2,4)-dipyridinacyc loundecaphan-3-one | 652.35 |
| 34 | 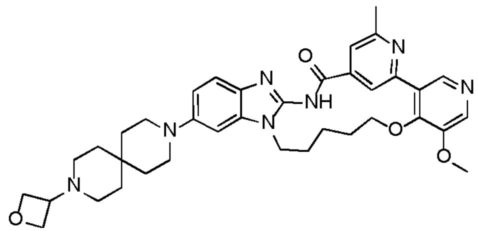 | $1^5$-methoxy-$2^6$-methyl-$5^6$-(9-( oxetan-3-yl)-3,9-diazaspiro[5. 5]undecan-3-yl)-$5^1$H-11-oxa-4-aza-5(2,1)-benzo[d]imidazo la-1 (3,4),2(2,4)-dipyridinacyc loundecaphan-3-one | 652.35 |

(continued)

| Example | Structure | Compound name | MS: $[M+H]^+$ |
|---|---|---|---|
| 35 | 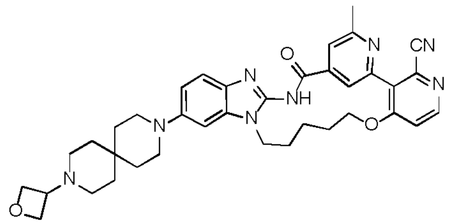 | $2^6$-methyl-$5^6$-(9-(oxetan-3-yl) -3,9-diazaspiro[5.5]undecan-3-yl)-3-oxo-$5^1$H-11-oxa-4-aza -5(2,1)-benzo[d]imidazola-1( 3,4),2(2,4)-dipyridinacycloun decaphane-$1^2$-carbonitrile | 647.34 |
| 36 | 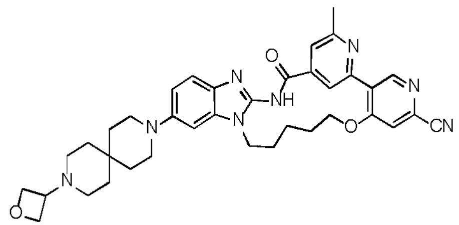 | $2^6$-methyl-$5^6$-(9-(oxetan-3-yl) -3,9-diazaspiro[5.5]undecan-3-yl)-3-oxo-$5^1$H-11-oxa-4-aza -5(2,1)-benzo[d]imidazola-1( 3,4),2(2,4)-dipyridinacycloun decaphane-$1^6$-carbonitrile | 647.34 |
| 37 | 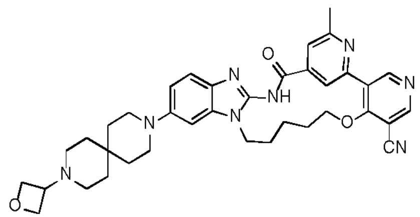 | $2^6$-methyl-$5^6$-(9-(oxetan-3-yl) -3,9-diazaspiro[5.5]undecan-3-yl)-3-oxo-$5^1$H-11-oxa-4-aza -5(2,1)-benzo[d]imidazola-1( 3,4),2(2,4)-dipyridinacycloun decaphane-$1^5$ -carbonitrile | 647.34 |
| 38 | 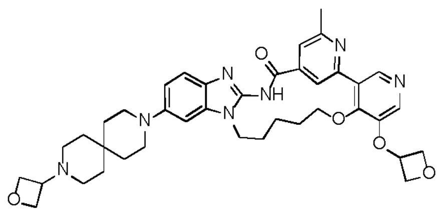 | $2^6$-methyl-$5^6$-(9-(oxetan-3-yl) -3,9-diazaspiro[5.5]undecan-3-yl)-$1^5$-(oxetan-3-yloxy)-$5^1$ H-11-oxa-4-aza-5(2,1)-benzo[ d]imidazola-1 (3,4),2(2,4)-dip yridinacycloundecaphan-3-on e | 694.36 |

(continued)

| Example | Structure | Compound name | MS: $[M+H]^+$ |
|---|---|---|---|
| 39 | 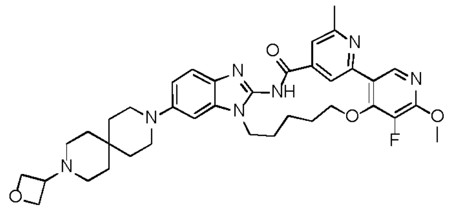 | $1^5$-fluoro-$1^6$-methoxy-$2^6$-met hyl-56-(9-(oxetan-3-yl)-3,9-di azaspiro[5.5]undecan-3-yl)-$5^1$ H-11-oxa-4-aza-5(2,1)-benzo[ d]imidazola-1 (3,4),2(2,4)-dip yridinacycloundecaphan-3-on e | 670.34 |
| 40 | 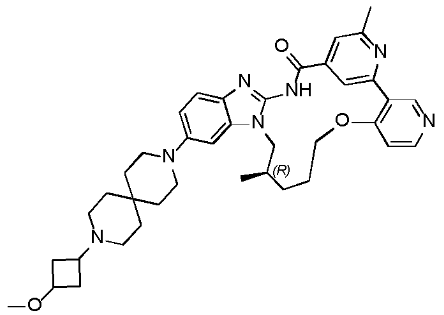 | (R)-$5^6$-(9-(3-methoxycyclobu tyl)-3,9-diazaspiro[5.5]undec an-3-yl)-$2^6$,7-dimethyl-$5^1$H-1 1-oxa-4-aza-5(2,1)-benzo[d]i midazola-1(3,4),2(2,4)-dipyri dinacycloundecaphan-3-one | 664.39 |
| 41 | 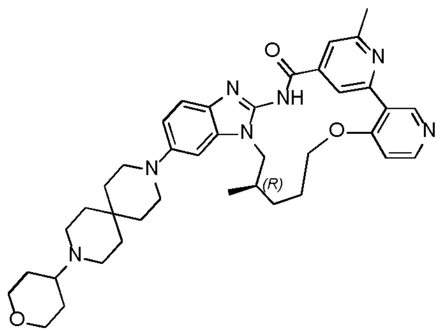 | (R)-$2^6$,7-dimethyl-$5^6$-(9-(tetra hydro-2H-pyran-4-yl)-3,9-dia zaspiro[5.5]undecan-3-yl)-$5^1$ H-11-oxa-4-aza-5(2,1)-benzo [d]imidazola-1(3,4),2(2,4)-di pyridinacycloundecaphan-3-o ne | 664.39 |

(continued)

| Example | Structure | Compound name | MS: $[M+H]^+$ |
|---|---|---|---|
| 42 | 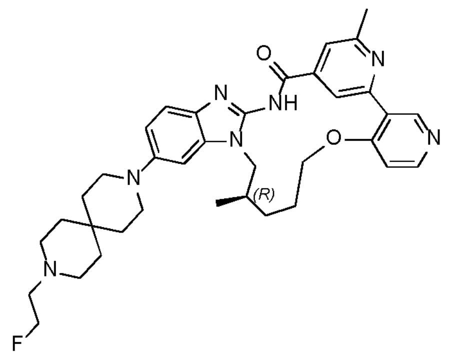 | (R)-$5^6$ -(9-(2-fluoroethyl)-3,9-diazaspiro[5.5]undecan-3-yl)-$2^6$,7-dimethyl-51H-11-oxa-4-aza-5(2,1)-benzo[d]imidazola -1(3,4),2(2,4)-dipyridinacyclo undecaphan-3-one | 626.35 |
| 43 | 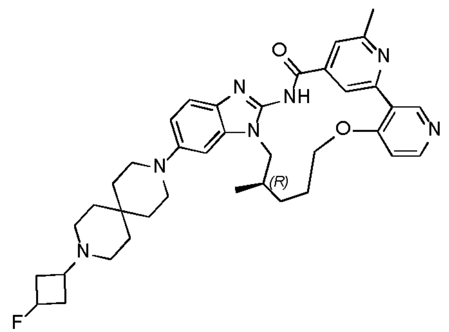 | (R)-$5^6$-(9-(3-fluorocyclobutyl )-3,9-diazaspiro[5.5]undecan-3-yl)-$2^6$,7-dimethyl-$5^1$H-11-o xa-4-aza-5(2,1)-benzo[d]imid azola-1(3,4),2(2,4)-dipyridina cycloundecaphan-3-one | 652.37 |
| 44 | 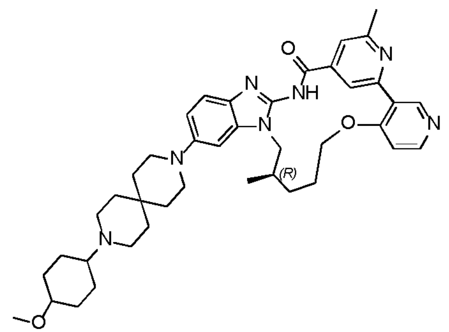 | (R)-$5^6$-(9-(4-methoxycyclohe xyl)-3,9-diazaspiro[5.5]undec an-3-yl)-$2^6$,7-dimethyl-$5^1$H-1 1-oxa-4-aza-5(2,1)-benzo[d]i midazola-1 (3,4),2(2,4)-dipyri dinacycloundecaphan-3-one | 692.42 |

(continued)

| Example | Structure | Compound name | MS: [M+H]+ |
|---|---|---|---|
| 45 | 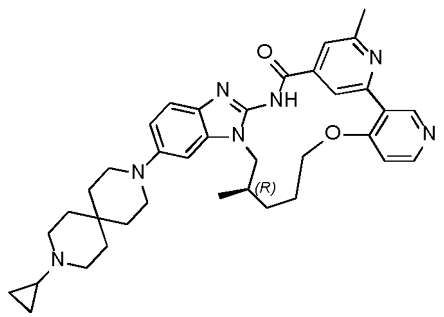 | (R)-5$^6$-(9-cyclopropyl-3,9-dia zaspiro[5.5]undecan-3-yl)-2$^6$, 7-dimethyl-5$^1$H-11-oxa-4-aza -5(2,1)-benzo[d]imidazola-1( 3,4),2(2,4)-dipyridinacycloun decaphan-3-one | 620.36 |
| 46 | 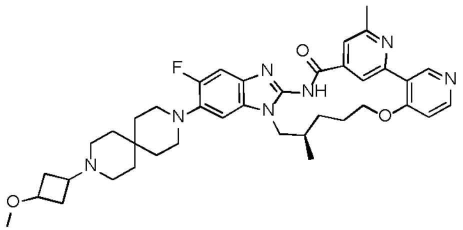 | (R)-5$^5$-fluoro-5$^6$-(9-(3-metho xycyclobutyl)-3,9-diazaspiro[ 5.5]undecan-3-yl)-2$^6$,7-dimet hyl-5$^1$H-11-oxa-4-aza-5(2,1)-benzo[d]imidazola-1(3,4),2(2, 4)-dipyridinacycloundecapha n-3-one | 682.38 |
| 47 | 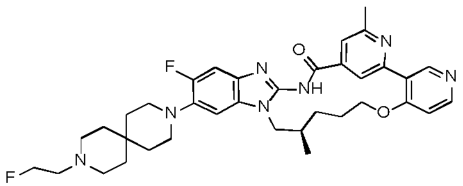 | (R)-5$^5$-fluoro-5$^6$-(9-(2-fluoroe thyl)-3,9-diazaspiro[5.5]unde can-3-yl)-2$^6$,7-dimethyl-5$^1$H-11-oxa-4-aza-5(2,1)-benzo[d] imidazola-1 (3,4),2(2,4)-dipyri dinacycloundecaphan-3-one | 644.34 |
| 48 | 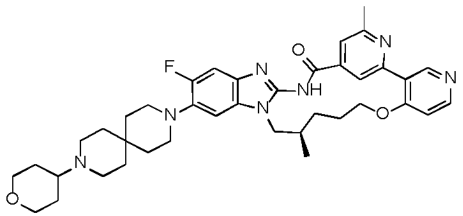 | (R)-5$^5$-fluoro-2$^6$,7-dimethyl-5 $^6$-(9-(tetrahydro-2H-pyran-4-yl)-3,9-diazaspiro[5.5]undeca n-3-yl)-5$^1$H-11-oxa-4-aza-5(2 ,1)-benzo[d]imidazola-1(3,4), 2(2,4)-dipyridinacycloundeca phan-3-one | 682.38 |

(continued)

| Example | Structure | Compound name | MS: [M+H]+ |
|---|---|---|---|
| 49 | 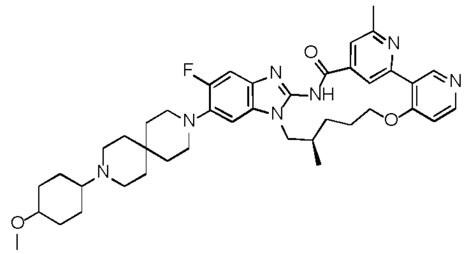 | (R)-$5^5$-fluoro-$5^6$-(9-(4-metho xycyclohexyl)-3,9-diazaspiro[ 5.5]undecan-3-yl)-$2^6$,7-dimet hyl-$5^1$H-11-oxa-4-aza-5(2,1)-benzo[d]imidazola-1(3,4),2(2, 4)-dipyridinacycloundecapha n-3-one | 710.41 |
| 50 | 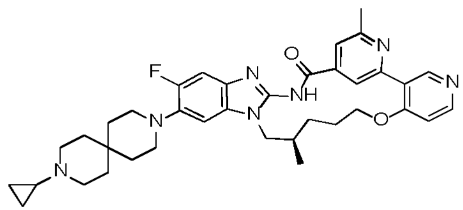 | (R)-$5^6$-(9-cyclopropyl-3,9-dia zaspiro[5.5]undecan-3-yl)-$5^5$-fluoro-$2^6$,7-dimethyl-$5^1$H-11-oxa-4-aza-5(2,1)-benzo[d]imi dazola-1(3,4),2(2,4)-dipyridin acycloundecaphan-3-one | 638.35 |
| 51 | 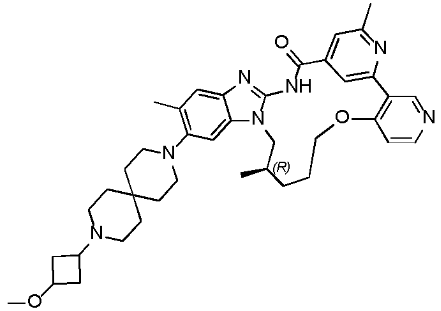 | (R)-$5^6$-(9-(3-methoxycyclobu tyl)-3,9-diazaspiro[5.5]undec an-3-yl)-$2^6$,$5^5$,7-trimethyl-$5^1$ H-11-oxa-4-aza-5(2,1)-benzo [d]imidazola-1 (3,4),2(2,4)-di pyridinacycloundecaphan-3-o ne | 678.41 |
| 52 | 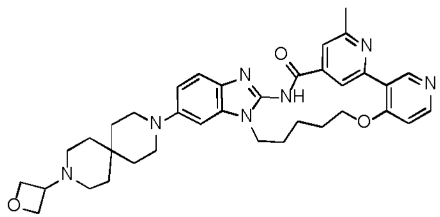 | $2^6$-methyl-$5^6$-(9-(oxetan-3-yl) -3,9-diazaspiro[5.5]undecan-3-yl)-$5^1$H-11-oxa-4-aza-5(2,1 )-benzo[d]imidazola-1(3,4),2( 2,4)-dipyridinacycloundecaph an-3-one | 622.34 |

(continued)

| Example | Structure | Compound name | MS: [M+H]$^+$ |
| --- | --- | --- | --- |
| 53 | 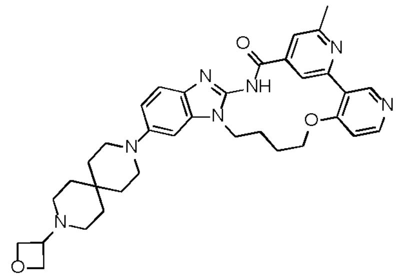 | $2^6$-methyl-$5^6$-(9-(oxetan-3-yl) -3,9-diazaspiro[5.5]undecan-3-yl)-$5^1$H-10-oxa-4-aza-5(2,1 )-benzo[d]imidazola-1(3,4),2( 2,4)-dipyridinacyclodecaphan -3-one | 608.33 |
| 54 | 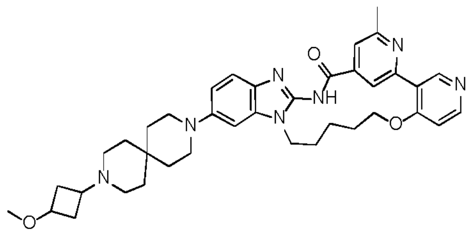 | $5^6$-(9-(3-methoxycyclobutyl)-3,9-diazaspiro[5.5]undecan-3 -yl)-$2^6$-methyl-$5^1$H-11-oxa-4-aza-5(2,1)-benzo[d]imidazola -1(3,4),2(2,4)-dipyridinacyclo undecaphan-3-one | 650.37 |
| 55 | 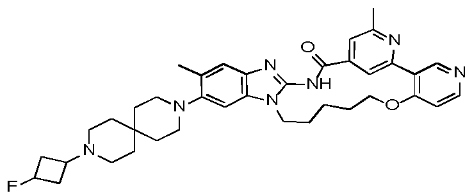 | $5^6$-(9-(3-fluorocyclobutyl)-3,9 -diazaspiro[5.5]undecan-3-yl) -$2^6$,$5^5$-dimethyl-$5^1$H-11-oxa-4 -aza-5(2,1)-benzo[d]imidazol a-1(3,4),2(2,4)-dipyridinacycl oundecaphan-3-one | 652.37 |
| 56 | 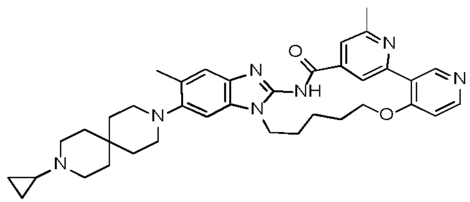 | $5^6$-(9-cyclopropyl-3,9-diazasp iro[5.5]undecan-3-yl)-$2^6$,$5^5$-di methyl-$5^1$H-11-oxa-4-aza-5(2 ,1)-benzo[d]imidazola-1(3,4), 2(2,4)-dipyridinacycloundeca phan-3-one | 620.36 |

(continued)

| Example | Structure | Compound name | MS: [M+H]+ |
|---|---|---|---|
| 57 | 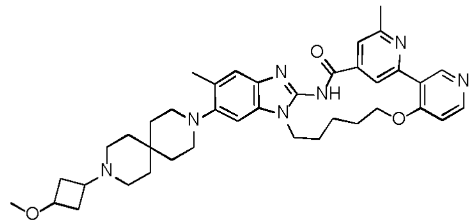 | $5^6$-(9-(3-methoxycyclobutyl)-3,9-diazaspiro[5.5]undecan-3 -yl)-$2^6$,$5^5$ -dimethyl-$5^1$H-11-o xa-4-aza-5(2,1)-benzo[d]imid azola-1(3,4),2(2,4)-dipyridina cycloundecaphan-3-one | 664.39 |
| 58 | 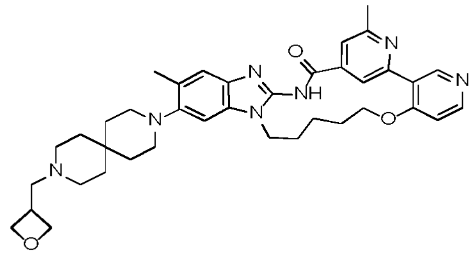 | $2^6$,$5^5$-dimethyl-$5^6$-(9-(oxetan-3-ylmethyl)-3,9-diazaspiro[5. 5]undecan-3-yl)-$5^1$H-11-oxa-4-aza-5(2,1)-benzo[d]imidazo la-1 (3,4),2(2,4)-dipyridinacyc loundecaphan-3-one | 650.37 |
| 59 | 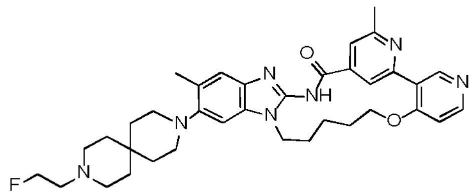 | $5^6$-(9-(2-fluoroethyl)-3,9-diaz aspiro[5.5]undecan-3-yl)-$2^6$,5 $^5$-dimethyl-$5^1$H-11-oxa-4-aza-5(2,1)-benzo[d]imidazola-1(3 ,4),2(2,4)-dipyridinacyclound ecaphan-3-one | 626.35 |
| 60 | 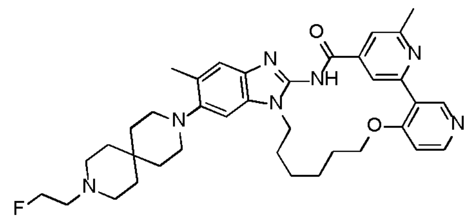 | $5^6$-(9-(2-fluoroethyl)-3,9-diaz aspiro[5.5]undecan-3-yl)-$2^6$,5 $^5$ -dimethyl-$5^1$H-12-oxa-4-aza-5(2,1)-benzo[d]imidazola-1(3 ,4),2(2,4)-dipyridinacyclodod ecaphan-3-one | 640.37 |

(continued)

| Example | Structure | Compound name | MS: [M+H]+ |
|---|---|---|---|
| 61 | 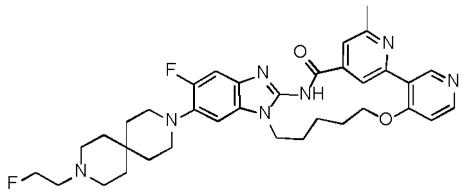 | $5^5$-fluoro-$5^6$-(9-(2-fluoroethyl )-3,9-diazaspiro[5.5]undecan-3-yl)-$2^6$-methyl-$5^1$H-11-oxa-4 -aza-5(2,1)-benzo[d]imidazol a-1(3,4),2(2,4)-dipyridinacycl oundecaphan-3-one | 630.33 |
| 62 | 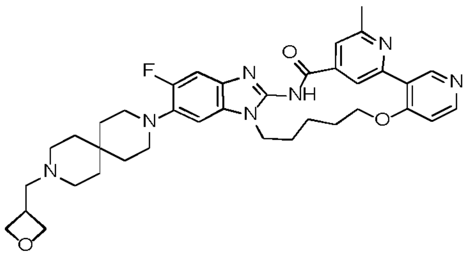 | $5^5$-fluoro-$2^6$-methyl-$5^6$-(9-(ox etan-3-ylmethyl)-3,9-diazaspi ro[5.5]undecan-3-yl)-$5^1$H-11-oxa-4-aza-5(2,1)-benzo[d]imi dazola-1(3,4),2(2,4)-dipyridin acycloundecaphan-3-one | 654.35 |
| 63 | 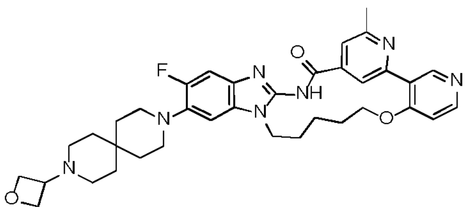 | $5^5$-fluoro-$2^6$-methyl-$5^6$-(9-(ox etan-3-yl)-3,9-diazaspiro[5.5] undecan-3-yl)-$5^1$H-11-oxa-4-aza-5(2,1)-benzo[d]imidazola -1(3,4),2(2,4)-dipyridinacyclo undecaphan-3-one | 640.33 |
| 64 | 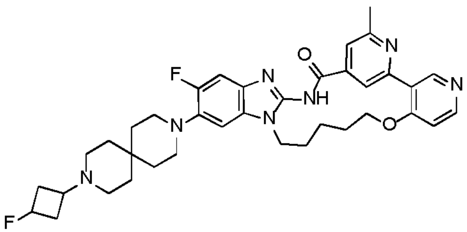 | $5^5$-fluoro-$5^6$-(9-(3-fluorocyclo butyl)-3,9-diazaspiro[5.5]und ecan-3-yl)-$2^6$-methyl-$5^1$H-11-oxa-4-aza-5(2,1)-benzo[d]imi dazola-1(3,4),2(2,4)-dipyridin acycloundecaphan-3-one | 656.34 |

(continued)

| Example | Structure | Compound name | MS: [M+H]+ |
|---|---|---|---|
| 65 | 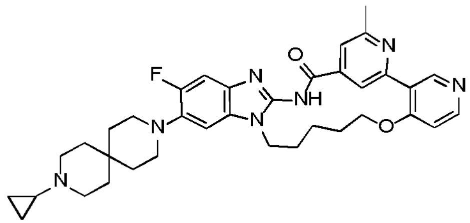 | $5^6$-(9-cyclopropyl-3,9-diazasp iro[5.5]undecan-3-yl)-$5^5$-fluor o-$2^6$-methyl-$5^1$H-11-oxa-4-az a-5(2,1)-benzo[d]imidazola-1 (3,4),2(2,4)-dipyridinacyclou ndecaphan-3-one | 624.34 |
| 66 | 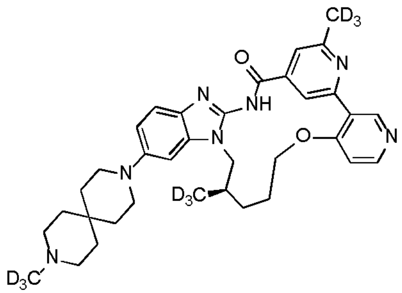 | (R)-$2^6$,7-bis(methyl-$d_3$)-$5^6$-(9-(methyl-$d_3$)-3,9-diazaspiro[5. 5]undecan-3-yl)-$5^1$H-11-oxa-4-aza-5(2,1)-benzo[d]imidazo la-1(3,4),2(2,4)-dipyridinacyc loundecaphan-3-one | 603.40 |
| 67 | 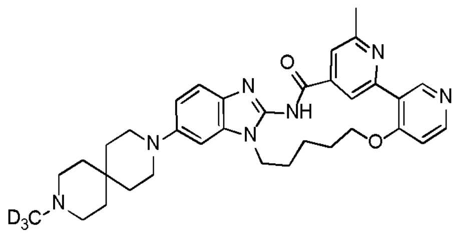 | $2^6$-methyl-$5^6$-(9-(methyl-$d_3$)-3 ,9-diazaspiro[5.5]undecan-3-yl)-$5^1$H-11-oxa-4-aza-5(2,1)-benzo [d]imidazola-1(3,4),2(2, 4)-dipyridinacycloundecapha n-3-one | 583.35 |
| 68 | 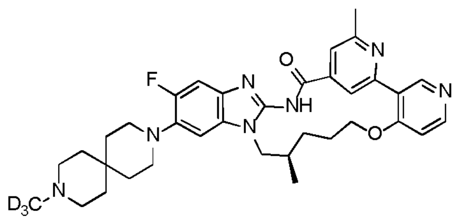 | (R)-$5^5$-fluoro-$2^6$,7-dimethyl-5 $^6$-(9-(methyl-$d_3$)-3,9-diazaspir o[5.5]undecan-3-yl)-$5^1$H-11-o xa-4-aza-5(2,1)-benzo[d]imid azola-1(3,4),2(2,4)-dipyridina cycloundecaphan-3-one | 615.36 |

(continued)

| Example | Structure | Compound name | MS: $[M+H]^+$ |
|---|---|---|---|
| 69 | 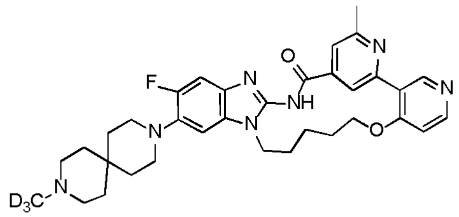 | $5^5$-fluoro-$2^6$-methyl-$5^6$-(9-(m ethyl-$d_3$)-3,9-diazaspiro[5.5]u ndecan-3-yl)-$5^1$H-11-oxa-4-a za-5(2,1)-benzo[d]imidazola-1(3,4),2(2,4)-dipyridinacyclo undecaphan-3-one | 601.34 |
| 70 | 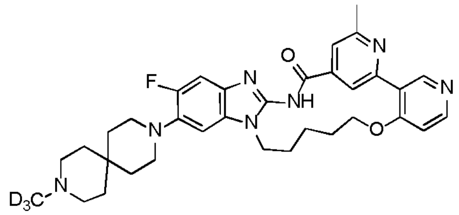 | **$2^6$,$5^5$-dimethyl-$5^6$-(9-(meth yl-*$d_3$*)-3,9-diazaspiro[5.5]un decan-3-yl)-$5^1$*H*-11-oxa-4-a za-5(2,1)-benzo[*d*]imidazola -1(3,4),2(2,4)-dipyridinacycl oundecaphan-3-one** | 601.34 |
| 71 | 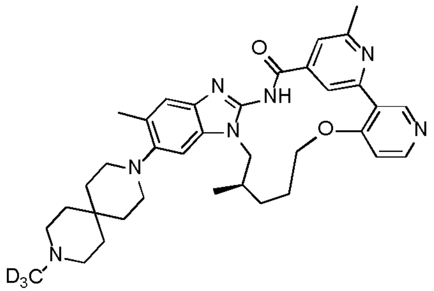 | (R)-$2^6$,$5^5$,7-trimethyl-$5^6$-(9-( methyl-$d_3$)-3,9-diazaspiro[5.5 ]undecan-3-yl)-$5^1$H-11-oxa-4 -aza-5(2,1)-benzo[d]imidazol a-1(3,4),2(2,4)-dipyridinacycl oundecaphan-3-one | 611.38 |
| 72 | 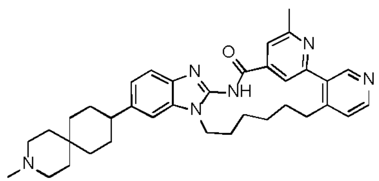 | $2^6$-methyl-$5^6$-(3-methyl-3-aza spiro[5.5]undecan-9-yl)-$5^1$H-4-aza-5(2,1)-benzo[d]imidazo la-1(3,4),2(2,4)-dipyridinacyc loundecaphan-3-one | 577.36 |

(continued)

| Example | Structure | Compound name | MS: [M+H]+ |
|---|---|---|---|
| 73 | 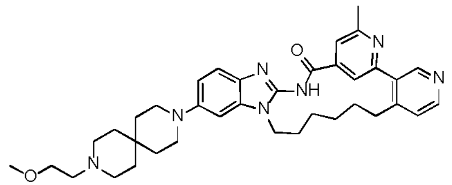 | 5⁶-(9-(2-methoxyethyl)-3,9-di azaspiro[5.5]undecan-3-yl)-2⁶ -methyl-5¹H-4-aza-5(2,1)-ben zo [d]imidazola-1(3,4),2(2,4)-dipyridinacycloundecaphan-3 -one | 622.38 |
| 74 | 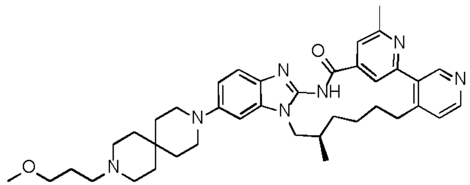 | (R)-5⁶-(9-(3-methoxypropyl)-3,9-diazaspiro[5.5]undecan-3 -yl)-2⁶,7-dimethyl-5¹H-4-aza-5(2,1)-benzo[d]imidazola-1(3 ,4),2(2,4)-dipyridinacyclound ecaphan-3-one | 650.41 |
| 75 | 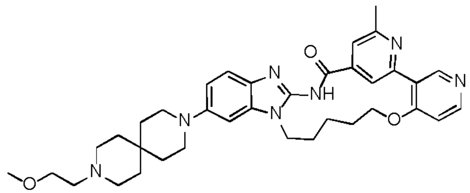 | 5⁶-(9-(2-methoxyethyl)-3,9-di azaspiro[5.5]undecan-3-yl)-2⁶ -methyl-5¹H-11-oxa-4-aza-5( 2,1)-benzo[d]imidazola-1(3,4 ),2(2,4)-dipyridinacycloundec aphan-3-one | 624.36 |
| 76 | 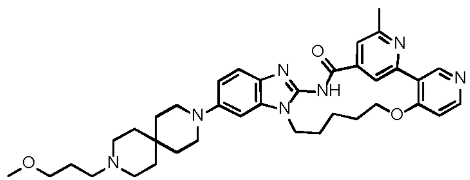 | 5⁶-(9-(3-methoxypropyl)-3,9-diazaspiro[5.5]undecan-3-yl)-2⁶-methyl-5¹H-11-oxa-4-aza-5(2,1)-benzo[d]imidazola-1(3 ,4),2(2,4)-dipyridinacyclound ecaphan-3-one | 637.37 |
| 77 | 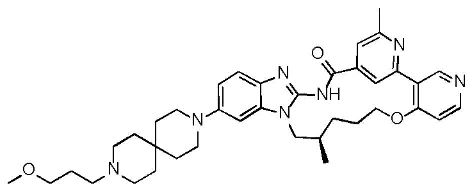 | (R)-5⁶-(9-(3-methoxypropyl)-3,9-diazaspiro[5.5]undecan-3 -yl)-2⁶,7-dimethyl-5¹H-11-ox a-4-aza-5(2,1)-benzo[d]imida zola-1(3,4),2(2,4)-dipyridinac ycloundecaphan-3-one | 652.39 |

(continued)

| Example | Structure | Compound name | MS: $[M+H]^+$ |
|---|---|---|---|
| 78 | 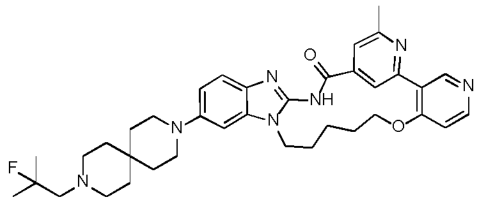 | $5^6$ -(9-(2-fluoro-2-methylprop yl)-3,9-diazaspiro[5.5]undeca n-3-yl)-$2^6$-methyl-$5^1$H-11-oxa -4-aza-5(2,1)-benzo[d]imidaz ola-1(3,4),2(2,4)-dipyridinacy cloundecaphan-3-one | 640.37 |
| 79 | 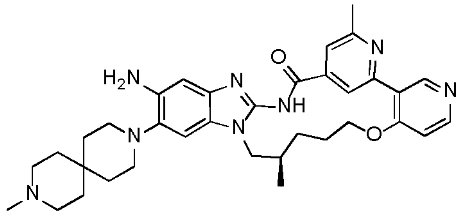 | (R)-$5^5$-amino-$2^6$,7-dimethyl-5 $^6$-(9-methyl-3,9-diazaspiro[5. 5]undecan-3-yl)-$5^1$H-11-oxa-4-aza-5(2,1)-benzo[d]imidazo la-1(3,4),2(2,4)-dipyridinacyc loundecaphan-3-one | 609.36 |
| 80 | 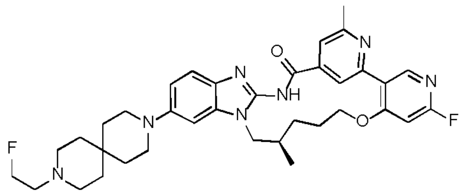 | (R)-$1^6$-fluoro-$5^6$-(9-(2-fluoroe thyl)-3,9-diazaspiro[5.5]unde can-3-yl)-$2^6$,7-dimethyl-$5^1$H-11-oxa-4-aza-5(2,1)-benzo[d] imidazola-1 (3,4),2(2,4)-dipyri dinacycloundecaphan-3-one | 644.34 |
| 81 | 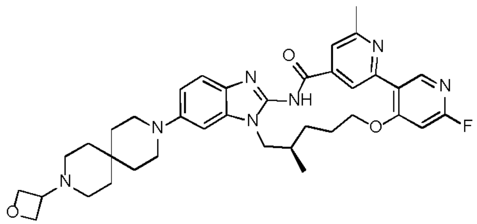 | (R)-$1^6$-fluoro-$2^6$,7-dimethyl-5 $^6$-(9-(oxetan-3-yl)-3,9-diazasp iro[5.5]undecan-3-yl)-$5^1$H-11 -oxa-4-aza-5(2,1)-benzo[d]im idazola-1 (3,4),2(2,4)-dipyridi nacycloundecaphan-3-one | 654.35 |

(continued)

| Example | Structure | Compound name | MS: [M+H]+ |
|---|---|---|---|
| 82 | 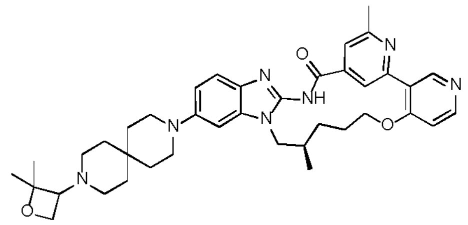 | (7R)-5$^{6}$-(9-(2,2-dimethyloxet an-3-yl)-3,9-diazaspiro[5.5]u ndecan-3-yl)-2$^{6}$,7-dimethyl-5$^{1}$ H-11-oxa-4-aza-5(2,1)-benzo [d]imidazola-1(3,4),2(2,4)-di pyridinacycloundecaphan-3-o ne | 664.39 |
| 83 | 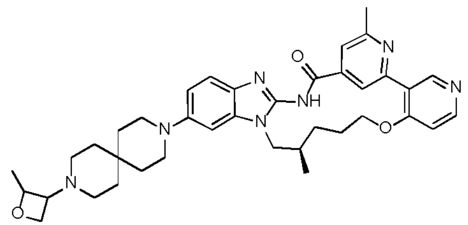 | (7R)-2$^{6}$,7-dimethyl-5$^{6}$-(9-(2-methyloxetan-3-yl)-3,9-diaza spiro[5.5]undecan-3-yl)-5$^{1}$H-11-oxa-4-aza-5(2,1)-benzo[d] imidazola-1 (3,4),2(2,4)-dipyri dinacycloundecaphan-3-one | 650.37 |
| 84 | 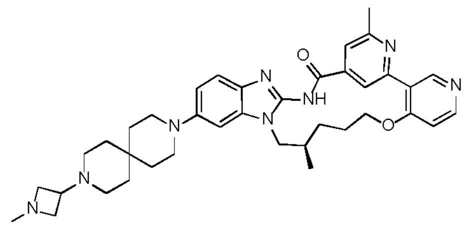 | (R)-2$^{6}$,7-dimethyl-5$^{6}$-(9-(1-m ethylazetidin-3-yl)-3,9-diazas piro[5.5]undecan-3-yl)-5$^{1}$H-1 1-oxa-4-aza-5(2,1)-benzo[d]i midazola-1 (3,4),2(2,4)-dipyri dinacycloundecaphan-3-one | 649.39 |
| 85 | 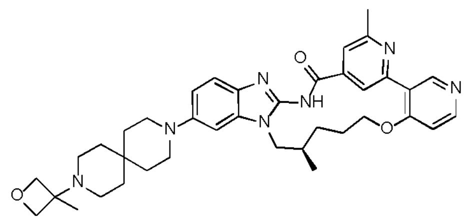 | (R)-2$^{6}$,7-dimethyl-5$^{6}$-(9-(3-m ethyloxetan-3-yl)-3,9-diazasp iro[5.5]undecan-3-yl)-5$^{1}$H-11 -oxa-4-aza-5(2,1)-benzo[d]im idazola-1 (3,4),2(2,4)-dipyridi nacycloundecaphan-3-one | 650.37 |

(continued)

| Example | Structure | Compound name | MS: [M+H]+ |
|---|---|---|---|
| 86 | 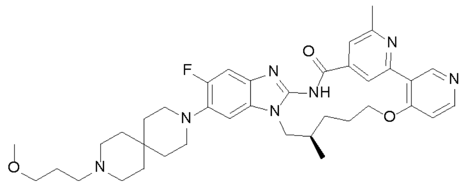 | (R)-$5^5$-fluoro-$5^6$-(9-(3-metho xypropyl)-3,9-diazaspiro[5.5] undecan-3-yl)-$2^6$,7-dimethyl-$5^1$H-11-oxa-4-aza-5(2,1)-ben zo[d]imidazola-1(3,4),2(2,4)-dipyridinacycloundecaphan-3 -one | 670.38 |
| 87 | 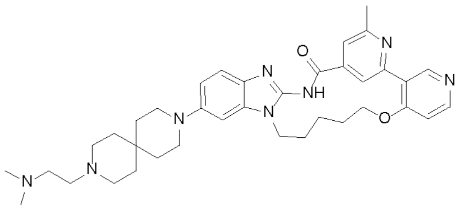 | $5^6$-(9-(2-(dimethylamino)ethy 1)-3,9-diazaspiro[5.5]undecan -3-yl)-$2^6$-methyl-$5^1$H-11-oxa-4-aza-5(2,1)-benzo[d]imidazo la-1(3,4),2(2,4)-dipyridinacyc loundecaphan-3-one | 637.39 |
| 88 | 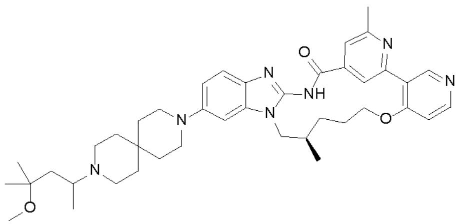 | (7R)-$5^6$-(9-(4-methoxy-4-met hylpentan-2-yl)-3,9-diazaspir o[5.5]undecan-3-yl)-$2^6$,7-dimethyl-$5^1$H-11-oxa-4-aza-5(2,1 )-benzo[d]imidazola-1(3,4),2( 2,4)-dipyridinacycloundecaphan-3-one | 694.44 |
| 89 | 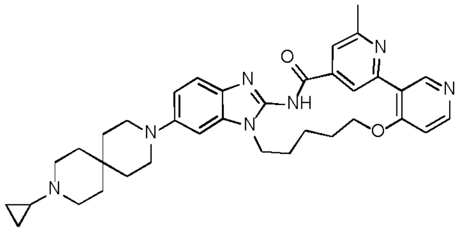 | $5^6$-(9-cyclopropyl-3,9-diazasp iro[5.5]undecan-3-yl)-$2^6$-met hyl-$5^1$H-11-oxa-4-aza-5(2,1)-benzo[d]imidazola-1(3,4),2(2, 4)-dipyridinacycloundecapha n-3-one | 606.35 |

(continued)

| Example | Structure | Compound name | MS: $[M+H]^+$ |
|---|---|---|---|
| 90 | 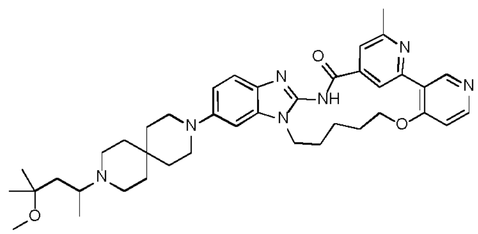 | $5^6$-(9-(4-methoxy-4-methylpe ntan-2-yl)-3,9-diazaspiro[5.5] undecan-3-yl)-$2^6$ -methyl-$5^1$H -11-oxa-4-aza-5(2,1)-benzo[d ]imidazola-1(3,4),2(2,4)-dipy ridinacycloundecaphan-3-one | 680.42 |
| 91 | 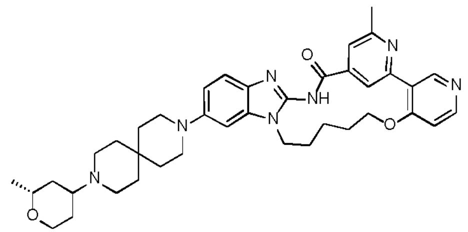 | $5^6$-(9-((2R,6S)-2,6-dimethylte trahydro-2H-pyran-4-yl)-3,9-diazaspiro[5.5]undecan-3-yl)-$2^6$-methyl-$5^1$H-11-oxa-4-aza-5(2,1)-benzo[d]imidazola-1(3 ,4),2(2,4)-dipyridinacyclound ecaphan-3-one | 678.41 |
| 92 | 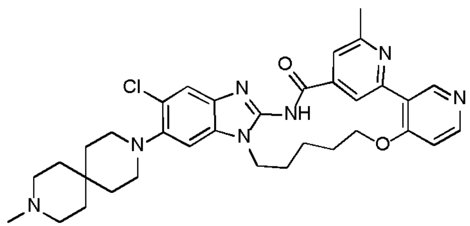 | $5^5$-chloro-$2^6$-methyl-$5^6$-(9-me thyl-3,9-diazaspiro[5.5]undec an-3-yl)-$5^1$H-11-oxa-4-aza-5( 2,1)-benzo[d]imidazola-1(3,4 ),2(2,4)-dipyridinacycloundec aphan-3-one | 614.29 |
| 93 | 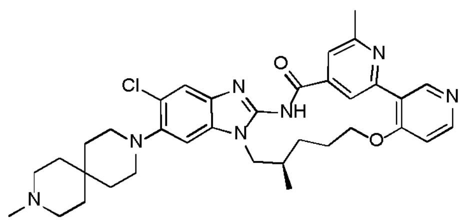 | (R)-$5^5$-chloro-$2^6$,7-dimethyl-5 $^6$-(9-methyl-3,9-diazaspiro[5. 5]undecan-3-yl)-$5^1$H-11-oxa-4-aza-5(2,1)-benzo[d]imidazo la-1(3,4),2(2,4)-dipyridinacyc loundecaphan-3-one | 628.31 |

(continued)

| Example | Structure | Compound name | MS: $[M+H]^+$ |
|---|---|---|---|
| 94 | 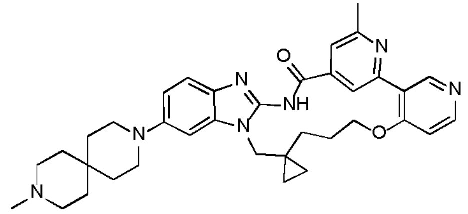 | 6'-methyl-6'-(9-methyl-3,9-di azaspiro[5.5]undecan-3-yl)spi ro[cyclopropane-1,7'-11-oxa-4-aza-5(2,1)-benzo[d]imidazo la-1(3,4),2(2,4)-dipyridinacyc loundecaphan]-3'-one | 606.35 |
| 95 | 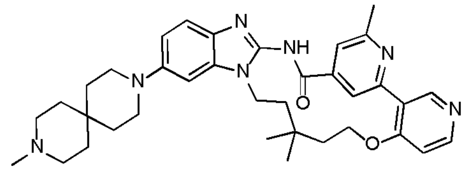 | $2^{6}$,8,8-trimethyl-$5^{6}$-(9-methyl -3,9-diazaspiro[5.5]undecan-3-yl)-$5^{1}$H-11-oxa-4-aza-5(2,1 )-benzo[d]imidazola-1(3,4),2( 2,4)-dipyridinacycloundecaph an-3-one | 608.36 |
| 96 | 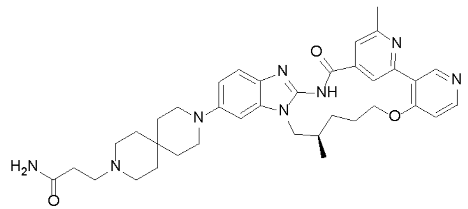 | (R)-3-(9-($2^{6}$,7-dimethyl-3-ox o-$5^{1}$H-11-oxa-4-aza-5(2,1)-be nzo[d]imidazola-1(3,4),2(2,4) -dipyridinacycloundecaphane -56-yl)-3,9-diazaspiro[5.5]un decan-3-yl)propanamide | 651.37 |
| 97 | 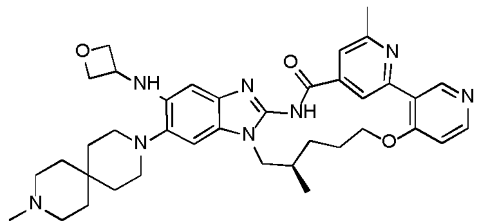 | (R)-$2^{6}$,7-dimethyl-$5^{6}$-(9-meth yl-3,9-diazaspiro[5.5]undecan -3-yl)-$5^{5}$-(oxetan-3-ylamino)-$5^{1}$H-11-oxa-4-aza-5(2,1)-ben zo[d]imidazola-1(3,4),2(2,4)-dipyridinacycloundecaphan-3 -one | 665.38 |
| 98 | 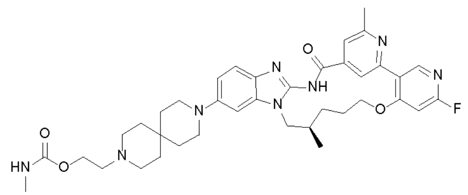 | (R)-2-(9-($1^{6}$-fluoro-$2^{6}$,7-dime thyl-3-oxo-$5^{1}$H-11-oxa-4-aza-5(2,1)-benzo[d]imidazola-1(3 ,4),2(2,4)-dipyridinacyclound ecaphane-$5^{6}$-yl)-3,9-diazaspir o[5.5]undecan-3-yl)ethyl methylcarbamate | 699.37 |

(continued)

| Example | Structure | Compound name | MS: [M+H]+ |
|---|---|---|---|
| 99 | 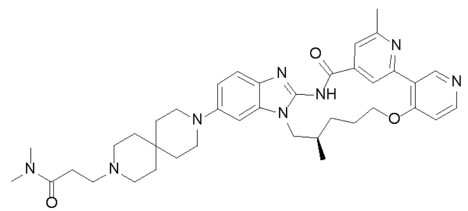 | (R)-3-(9-($2^6$,7-dimethyl-3-ox o-$5^1$H-11-oxa-4-aza-5(2,1)-be nzo[d]imidazola-1(3,4),2(2,4) -di-pyridinacycloundecaphane -$5^6$-yl)-3,9-diazaspiro[5.5]und ecan-3-yl)-N,N-dimethylprop ana-mide | 679.40 |
| 100 | 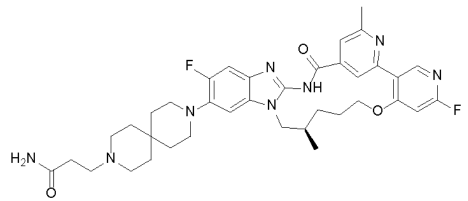 | (R)-3-(9-($1^6$,$5^5$-difluoro-$2^6$,7-dimethyl-3-oxo-$5^1$H-11-oxa-4 -aza-5(2,1)-benzo[d]imidazol a-1(3,4),2(2,4)-dipyridinacycl oundecaphane-$5^6$-yl)-3,9-diaz aspiro[5.5]undecan-3-yl)prop anamide | 687.35 |
| 101 | 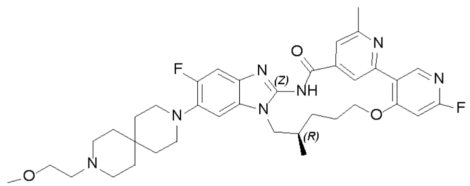 | (R)-$1^6$,$5^5$-difluoro-$5^6$-(9-(2-m ethoxyethyl)-3,9-diazaspiro[5 .5]undecan-3-yl)-$2^6$,7-dimeth yl-$5^1$H-11-oxa-4-aza-5(2,1)-b enzo[d]imidazola-1(3,4),2(2,4 )-dipyridinacycloundecaphan-3-one | 674.36 |
| 102 | 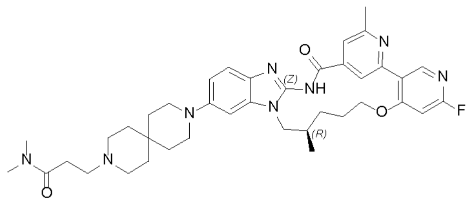 | (R)-3-(9-($1^6$ -fluoro-$2^6$,7-dime thyl-3-oxo-$5^1$H-11-oxa-4-aza-5(2,1)-benzo[d]imidazola-1(3 ,4),2(2,4)-dipyridinacyclound ecaphane-56-yl)-3,9-diazaspir o[5.5]undecan-3-yl)-N,N-dim ethylpropanamide | 697.39 |
| 103 | 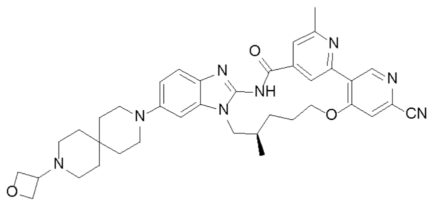 | (R)-$2^6$,7-dimethyl-$5^6$-(9-(oxet an-3-yl)-3,9-diazaspiro[5.5]u ndecan-3-yl)-3-oxo-$5^1$H-11-o xa-4-aza-5(2,1)-benzo[d]imid azola-1(3,4),2(2,4)-dipyridina | 661.35 |

(continued)

| Example | Structure | Compound name | MS: $[M+H]^+$ |
|---|---|---|---|
| | | cycloundecaphane-$1^6$-carboni trile | |
| 104 | 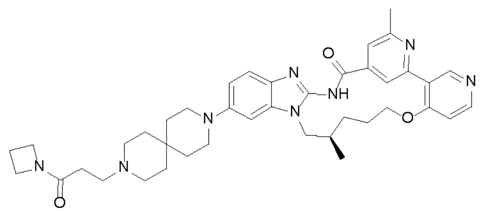 | (R)-$5^6$-(9-(3-(azetidin-1-yl)-3 -oxopropyl)-3,9-diazaspiro[5. 5]undecan-3-yl)-$2^6$,7-dimethy 1-$5^1$H-11-oxa-4-aza-5(2,1)-be nzo[d]imidazola-1(3,4),2(2,4) -dipyridinacycloundecaphan-3-one | 691.40 |
| 105 | 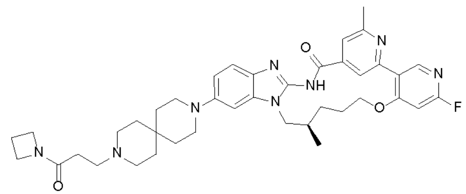 | (R)-$5^6$-(9-(3-(azetidin-1-yl)-3 -oxopropyl)-3,9-diazaspiro[5. 5]undecan-3-yl)-$1^6$-fluoro-$2^6$, 7-di-methyl-51H-11-oxa-4-aza -5(2,1)-benzo[d]imidazola-1( 3,4),2(2,4)-dipyridinacycloun decaphan-3-one | 709.39 |
| 106 | 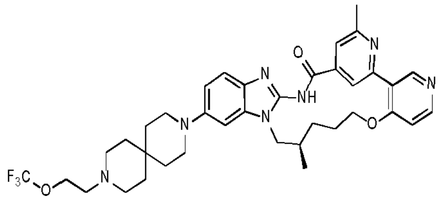 | (R)-$2^6$,7-dimethyl-$5^6$-(9-(2-(tr ifluoromethoxy)ethyl)-3,9-dia zaspiro[5.5]undecan-3-yl)-$5^1$ H-11-oxa-4-aza-5(2,1)-benzo [d]imidazola-1 (3,4),2(2,4)-di pyridinacycloundecaphan-3-o ne | 692.35 |
| 107 | 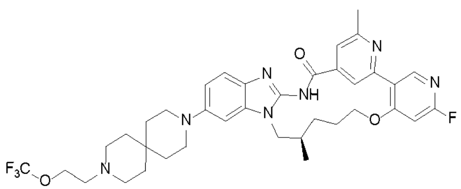 | (R)-$1^6$ -fluoro-$2^6$,7-dimethyl-5 $^6$-(9-(2-(trifluoromethoxy)eth yl)-3,9-diazaspiro[5.5]undeca n-3-yl)-$5^1$H-11-oxa-4-aza-5(2 ,1)-benzo[d]imidazola-1(3,4), 2(2,4)-dipyridinacycloundeca phan-3-one | 710.34 |

(continued)

| Example | Structure | Compound name | MS: $[M+H]^+$ |
|---|---|---|---|
| 108 | 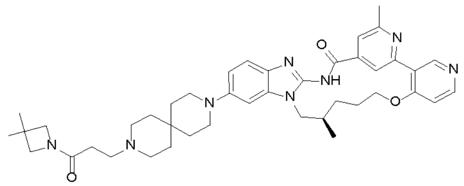 | (R)-$5^6$-(9-(3-(3,3-dimethylaze tidin-1-yl)-3-oxopropyl)-3,9-diazaspiro[5.5]undecan-3-yl)-$2^6$,7-dimethyl-$5^1$H-11-oxa-4-a za-5(2,1)-benzo[d]imidazola-1(3,4),2(2,4)-dipyridinacyclo undecaphan-3-one | 719.43 |
| 109 | 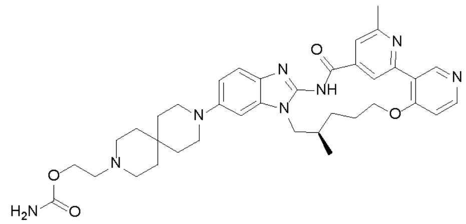 | (R)-2-(9-($2^6$,7-dimethyl-3-ox o-$5^1$H-11-oxa-4-aza-5(2,1)-be nzo[d]imidazola-1(3,4),2(2,4) -di-pyridinacycloundecaphane -$5^6$-yl)-3,9-diazaspiro[5.5]und ecan-3-yl)ethyl carbamate | 667.36 |

Example 77: $^1$H NMR (500 MHz, DMSO-$d_6$) δ 12.53 (s, 1H), 9.14 (s, 1H), 8.97

(s, 1H), 8.46 (d, *J* = 5.7 Hz, 1H), 7.77 - 7.73 (m, 1H), 7.36 (d, *J* = 8.7 Hz, 1H), 7.19 (d, *J* = 5.9 Hz, 1H), 7.07 (d, *J* = 2.1 Hz, 1H), 6.89 (dd, *J* = 8.9, 2.2 Hz, 1H), 4.25 (dt, *J* = 10.5, 7.2 Hz, 2H), 4.12 - 4.05 (m, 1H), 4.00 (dd, *J* = 13.9, 6.5 Hz, 1H), 3.34 (t, *J* = 6.3 Hz, 2H), 3.22 (s, 3H), 3.13 (t, *J* = 5.6 Hz, 4H), 2.90 (q, *J* = 7.2 Hz, 2H), 2.63 (s, 3H), 2.56 (dt, *J* = 22.9, 6.4 Hz, 6H), 2.29 - 2.23 (m, 1H), 2.03 (s, 1H), 1.72 (p, *J* = 6.4 Hz, 2H), 1.56 (dt, *J* = 22.6, 5.5 Hz, 9H), 0.93 (d, *J* = 6.5 Hz, 3H).

Example 87: $^1$H NMR (500 MHz, DMSO-$d_6$) δ 12.48 (s, 1H), 9.24 (s, 1H), 9.05

(s, 1H), 8.46 (d, *J* = 5.7 Hz, 1H), 7.71 (d, *J* = 1.2 Hz, 1H), 7.36 (d, *J* = 8.7 Hz, 1H), 7.20 (d, *J* = 5.8 Hz, 1H), 7.06 (d, *J* = 2.2 Hz, 1H), 6.91 (dd, *J* = 9.0, 2.1 Hz, 1H), 4.28 (t, *J* = 4.8 Hz, 2H), 4.19 (s, 2H), 3.14 (t, *J* = 5.6 Hz, 4H), 2.64 (s, 3H), 2.48 (s, 5H), 2.25 (s, 6H), 2.04 - 1.97 (m, 3H), 1.92 (s, 3H), 1.91 (t, *J* = 9.2 Hz, 1H), 1.58 (t, *J* = 5.6 Hz, 4H), 1.51 (t, *J* = 5.6 Hz, 4H), 1.23 (s, 2H).

Example 89: $^1$H NMR (500 MHz, DMSO-$d_6$) δ 12.48 (s, 1H), 9.24 (s, 1H), 9.05

(s, 1H), 8.46 (d, *J* = 5.8 Hz, 1H), 7.71 (d, *J* = 1.2 Hz, 1H), 7.36 (dd, *J* = 8.8, 5.3 Hz, 1H), 7.20 (d, *J* = 5.9 Hz, 1H), 7.06 (d, *J* = 2.3 Hz, 1H), 6.91 (dd, *J* = 8.7, 2.3 Hz, 1H), 4.28 (t, *J* = 4.9 Hz, 2H), 4.19 (t, *J* = 5.7 Hz, 2H), 3.14 (t, *J* = 5.9 Hz, 4H), 2.64 (s, 3H), 2.53 (d, *J* = 5.4 Hz, 2H), 2.04 - 1.97 (m, 3H), 1.95 - 1.87 (m, 5H), 1.60 (dt, *J* = 25.7, 6.3 Hz, 5H), 1.52 (s, 1H), 1.44 (t, *J* = 5.7 Hz, 3H), 1.23 (s, 2H), 0.39 (dt, *J* = 6.1, 3.0 Hz, 1H), 0.29 - 0.23 (m, 1H).

Example 90: $^1$H NMR (500 MHz, DMSO-$d_6$) δ 12.48 (s, 1H), 9.24 (s, 1H), 9.05

(s, 1H), 8.46 (d, *J* = 5.7 Hz, 1H), 7.71 (s, 1H), 7.36 (d, *J* = 8.7 Hz, 1H), 7.20 (d, *J* = 5.8 Hz, 1H), 7.05 (s, 1H), 6.91 (d, *J* = 8.8 Hz, 1H), 4.28 (d, *J* = 5.4 Hz, 2H), 4.19 (s, 2H), 3.14 (t, *J* = 5.5 Hz, 4H), 3.08 (s, 3H), 2.88 (d, *J* = 9.4 Hz, 1H), 2.64 (s, 5H), 2.54 (s, 1H), 2.01 (s, 2H), 1.92 (s, 4H), 1.74 (dd, *J* = 13.8, 4.8 Hz, 1H), 1.60 - 1.56 (m, 4H), 1.52 (s, 5H), 1.39 (dd, *J* = 14.3, 6.4 Hz, 1H), 1.13 (d, *J* = 4.5 Hz, 6H), 1.04 (d, *J* = 6.6 Hz, 3H).

Example 91: $^1$H NMR(500 MHz, DMSO-$d_6$) δ 12.48 (s, 1H), 9.24 (s, 1H), 9.05

(s, 1H), 8.46 (d, *J* = 5.7 Hz, 1H), 7.71 (s, 1H), 7.35 (d, *J* = 8.7 Hz, 1H), 7.20 (d, *J* = 5.9 Hz, 1H), 7.05 (d, *J* = 2.2 Hz, 1H), 6.90 (dd, *J* = 8.9, 2.1 Hz, 1H), 4.28 (t, *J* = 4.9 Hz, 2H), 4.22 - 4.15 (m, 2H), 3.39 (dt, *J* = 10.9, 6.3 Hz, 2H), 3.13 (t, *J* = 5.5 Hz, 3H), 2.64 (s, 3H), 2.55 (q, *J* = 9.2, 5.5 Hz, 4H), 2.05 - 1.85 (m, 6H), 1.79 - 1.72 (m, 2H), 1.57 (t, *J* = 5.6 Hz, 4H), 1.49 (d, *J* = 5.8 Hz, 4H), 1.23 (s, 1H), 1.13 - 1.05 (m, 7H), 1.06 - 0.98 (m, 2H).

**Comparative compound 1**

According to Example I-021 on page 129 of WO2020260252, the following comparative compound 1 was prepared.

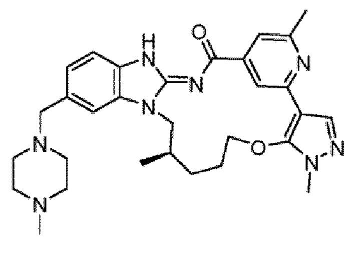

Comparative compound 1 (BI-4020)

**Comparative compound 2**

According to Example 51 on page 47 of CN114163454A, the following comparative compound 2 was prepared.

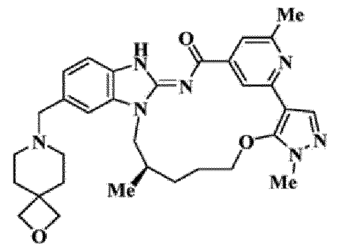

Comparative compound 2

**Pharmacological experiments**

**Example A: Cell proliferation inhibitory activity assay**

Suspension cells Ba/F3 (EGFR Del19/T790M/C797S), Ba/F3 (EGFR L858R/T790M/C797S), Ba/F3 (EGFR Del19/C797S), and Ba/F3 (EGFR L858R/C797S) overexpressing different EGFR mutations were cultured in PRMI 1640 containing 10% FBS and 1% PS, and adherent cells A431 carrying EGFR WT were cultured in DMEM containing 10% FBS and 1% PS. The experimental steps for compound activity detection are as follows:

(1) Take adherent cells in the logarithmic growth phase, digest them with trypsin, count them, and then inoculate them into a 96-well plate at a certain density. Incubate them overnight at 37°C and 5% $CO_2$ to allow them to adhere. Take suspension cells in the logarithmic growth phase, count them, and then inoculate them into a 96-well plate at a certain density;
(2) Compound preparation: Dilute the compound with DMSO in a 3-fold gradient, and then dilute it 100 times with culture medium to obtain a 10 * working solution;
(3) Add 10 * working solution containing compounds of different concentrations to each well, and incubate the cells in a 37 °C, 5% $CO_2$ incubator for 72 hours;
(4) Take out a 96 well cell culture plate, add 50 μ L CTG reagent to each well, shake for 2 minutes, react for 10 minutes, and use PerkinElmer reader to read the luminescence signal value Lum;
(5) Calculate the cell survival inhibition rate of each well, analyze the data using GraphPad Prism 6.0 software, fit the data using a nonlinear regression equation to obtain a dose-response curve, and calculate the $IC_{50}$ of the compound:

Cell survival inhibition rate (%)=(1-(Lum test compound-Lum culture medium control)/(Lum cell control-Lum culture medium control))×100%

$$Y= minimum + (minimum- minimum)/(1+10^((LogIC50-X)* slope));$$

X: logarithm of compound concentration; Y: cell survival inhibition rate.

The $IC_{50}$ results of cell proliferation inhibition activity are shown in Table 2.

Table 2

| Example | Ba/F3 cells overexpressing different EGFR mutations $IC_{50}$ (nM) | | | | A431 cell $IC_{50}$ (nM) |
|---|---|---|---|---|---|
| | Del19/ T790M/ C797S | L858R/ T790M/ C797S | Del19/ C797S | L858R/ C797S | Carrying EGFR WT |
| 1 | 0.24 | 0.62 | 0.08 | 0.23 | 1643 |
| 3 | 0.49 | 0.71 | 0.07 | 0.44 | 759 |
| 4 | 0.2 | 1.1 | 0.2 | 0.8 | 274 |
| 5 | 0.61 | 0.98 | 0.19 | 0.88 | 331 |
| 6 | 2.1 | 4.1 | 0.38 | 2.1 | 403 |

(continued)

| Example | Ba/F3 cells overexpressing different EGFR mutations $IC_{50}$ (nM) | | | | A431 cell $IC_{50}$ (nM) |
|---|---|---|---|---|---|
| | Del19/ T790M/ C797S | L858R/ T790M/ C797S | Del19/ C797S | L858R/ C797S | Carrying EGFR WT |
| 7 | 1.6 | 3.7 | 4.5 | 11 | 226 |
| 9 | 0.73 | 1.1 | 0.77 | 3.6 | 425 |
| 11 | 2.2 | 3.4 | 0.15 | 1.0 | 493 |
| 12 | 4.9 | 7.6 | 0.48 | 4.4 | 1004 |
| 13 | 0.96 | 2.0 | 0.20 | 1.3 | >3333 |
| 14 | 0.37 | 1.5 | 0.18 | 1.8 | 805 |
| 17 | 1.2 | 4.7 | 5.0 | 18 | 1468 |
| 18 | 0.77 | 2.1 | 0.5 | 3.9 | 631 |
| 45 | 1.6 | 3.7 | 0.27 | 2.1 | 701 |
| 52 | 0.8 | 1.6 | 0.5 | 4.7 | 700 |
| 59 | 2.4 | 3.7 | 0.45 | 5.7 | / |
| 61 | 0.91 | 1.8 | 0.29 | 3.0 | 1080 |
| 62 | 0.95 | 3 | 0.57 | 3.8 | 772 |
| 63 | 0.5 | 1.5 | 0.67 | 2.0 | 472 |
| 65 | 1.1 | 2.3 | 0.69 | 4.6 | 1250 |
| 75 | 0.07 | 0.08 | 0.11 | 0.07 | 646 |
| 76 | 0.14 | 0.2 | 0.09 | 0.34 | 586 |
| 77 | 0.36 | 0.36 | 0.23 | 0.18 | 426 |
| 78 | 5.9 | 12 | 3.3 | 21 | >3333 |
| 81 | 1.4 | 4.3 | 0.1 | 5.3 | 1747 |
| 82 | 4.7 | 12 | 2.9 | 17 | 770 |
| 83 | 1.3 | 2 | 0.27 | 2 | 294 |
| 86 | 0.41 | 0.18 | 0.15 | 0.38 | 299 |
| 87 | 5.4 | 11 | 5.8 | 21 | 1407 |
| 89 | 1.8 | 4.6 | 0.71 | 6.5 | 1206 |
| 90 | 4.2 | 12 | 4.0 | 31 | 733 |
| 91 | 1.7 | 3.4 | 0.64 | 5.2 | 759 |

The above experiments show that the compounds of the present invention have good proliferation inhibitory activity on Ba/F3 (EGFR Del 19/C797S), Ba/F3 (EGFR L858R/C797S), Ba/F3 (EGFR L858R/T790M/C797S) and Ba/F3 (EGFR Del19/T790M/C797S) double mutation or triple mutation cell lines, and have weaker inhibitory effect on EGFR wild-type cell line A431, indicating that the compounds of the present invention have good cell inhibitory activity and selectivity.

**Example B: Pharmacokinetics in mice**

Mouse PK studies were performed using female BALB/c mice (20-30 g). Compounds were prepared and prepared immediately before use. Mice were administered intravenously or orally by gavage. Blood was collected from the mouse orbital venous plexus, and 100 μL of whole blood was transferred to a K2-EDTA tube at each time point.

1 mg/kg was given intravenously, and whole blood was collected at 0.083, 0.25, 0.5, 1, 2, 4, 6 and 24 h after administration; 5 mg/kg were given orally by gavage, and whole blood was collected at 0.25, 0.5, 1, 2, 4, 6, 24 h after

administration in different batches, or the mice were killed at 2, 4 and 6 h to obtain brains. Whole blood samples were centrifuged and plasma was separated; brain tissue samples were homogenized by adding 4 times the volume of buffer, and the plasma and brain tissue samples were stored in a -80°C refrigerator for later use. After the above plasma and brain tissue samples were precipitated with acetonitrile for protein, the supernatant was taken and mixed with water in a 1:1 ratio, and 10 μL was taken for LC-MS/MS detection. The pharmacokinetic data of orally administered plasma are shown in Table 3.

Table 3

| Example | $T_{1/2}$ (h) | $C_{max}$ (ng/mL) | $AUC_{last}$(h*ng/mL) |
|---|---|---|---|
| Comparative compound 1 | 4.22 | 32.8 | 361 |
| 4 | 5.28 | 181 | 2092 |
| 6 | 21.5 | 92.4 | 1142 |
| 52 | 20.1 | 215 | 3457 |
| 91 | / | 41.9 | 1495 |

**Example C: Pharmacokinetics in rats**

Male SD rats (200-300 g) were used for rat PK studies. Compounds were prepared and prepared immediately before use. Rats were administered intravenously or orally by gavage. Blood was collected from the rats through the orbital venous plexus, and 100 μL whole blood was collected into K2-EDTA tubes at each time point.

1 mg/kg was given intravenously, and whole blood was collected at 0.083, 0.25, 0.5, 1, 2, 4, 7, 24, 48, and 72 h after administration;5 mg/kg were given orally by gavage, and whole blood was collected at 0.25, 0.5, 1, 2, 4, 7, 24, 48, and 72 h after administration in different batches, or the rats were killed at 4 and 24 h to obtain brains. The brain tissue samples were homogenized by adding 4 times the volume of buffer, and the whole blood and brain tissue samples were stored in a -80°C refrigerator for later use. After the above whole blood and brain tissue samples were precipitated with acetonitrile for protein, the supernatant was taken and mixed with water in a ratio of 1:2, and 10 μL was taken for LC-MS/MS detection. The pharmacokinetic data of orally administered plasma are shown in Table 4.

Table 4

| Example | $T_{1/2}$ (h) | $C_{max}$ (ng/mL) | $AUC_{last}$ (h*ng/mL) |
|---|---|---|---|
| Comparative compound 1 | 2.94 | 10.5 | 96 |
| Comparative compound 2 | / | 27.3 | 132 |
| 4 | 27.5 | 165 | 2592 |
| 6 | 17.2 | 106 | 1704 |
| 15 | / | 104 | 3387 |
| 52 | 12.4 | 57.5 | 917 |
| 81 | 18.8 | 116 | 1845 |
| 86 | 15.3 | 60.2 | 1405 |
| 91 | 26.4 | 42.5 | 1611 |

The experiments of Examples B and C show that the compounds of the present invention exhibit excellent pharmacokinetic properties and are significantly better than the comparative compounds 1 and 2.

**Example D: Determination of metabolic stability of liver microsomes**

The liver microsomes of human, rat, mouse and dog were used to study the metabolic stability of the compounds. The specific experimental steps are as follows:

(1) Add 40 μL $MgCl_2$ and 306 μL PBS to each well of a 96-well plate, and then add 4 μL compound, with a final DMSO concentration of ≤0.5%. Set up a blank well without compound;

(2) Add 10 μL of 20 mg/mL liver microsomes to each well, incubate at 37°C for 10 min;
(3) Add 40 μL NADPH working solution to each well to start the reaction, and set up a control well without NADPH;
(4) At 0, 5, 15, and 45 min after the start of the reaction, 50 μL of sample was taken out, the termination solution was added, and the mixture was shaken for 5 min.
(5) The samples were then centrifuged at 3,220 g for 10 min, and 50 μL of the supernatant was removed and was added 200 μL water for dilution and analysis by

LC-MS/MS.

The in vitro intrinsic clearance (in vitro $CL_{int}$, in μL/min/mg) was calculated from the in vitro half-life $t_{1/2}$ (minutes) using the following equation (mean of replicates):

In vitro clearance rate=0.693/ in vitro half-life $t_{12}$×[incubation volume (μL)/( protein amount (mg)]

The results are shown in Table 5.

Table 5

| Example | $CL_{int}$(μL/min/mg protein) | | | |
|---|---|---|---|---|
| | Human | Rat | Mouse | Dog |
| 1 | 7.2 | 14.2 | 32.7 | 8.9 |
| 3 | 6.6 | 8 | 23.1 | 7.9 |
| 4 | 26.3 | 5.6 | / | 24 |
| 5 | 9.1 | 8.9 | 22.8 | 2.2 |
| 6 | 41.5 | 2.9 | / | 20.6 |
| 12 | 24.9 | 0 | 5.5 | 10.4 |
| 13 | 4 | 7 | 6.2 | 4.2 |
| 59 | 4.82 | 23.3 | 8.93 | 0 |
| 76 | 8 | 15.6 | 23 | 0 |
| 77 | 17 | 6.7 | 25.1 | 0 |
| 78 | 4.3 | 12.2 | 69.5 | 22 |
| 86 | 11.4 | 4.5 | 21.7 | 11.5 |

Experiments show that the compounds of the present invention exhibit excellent metabolic stability in liver microsomes.

Although the present invention has been comprehensively described through its embodiments, it is worth noting that various changes and modifications are obvious to those skilled in the art. Such changes and modifications should be included in the scope of the appended claims of the present invention.

## Claims

1. A compound as shown in formula (I), or its stereoisomer, tautomer, solvate, pharmaceutically acceptable salt, or deuterated derivative:

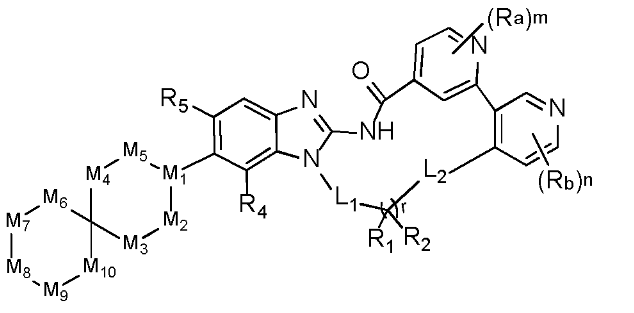

formula (I)

wherein,

$L_1$, $L_2$ are independently selected from the group consisting of bond, $-CR_hR_i-$, $-NR_h-$ and -O-;
each of $R_a$, $R_b$ is the same or different, independently selected from the group consisting of H, deuterium, halogen, CN, $NO_2$, $-OR_e$, $-SR_e$, $-S(O)R_e$, $-S(O)_2R_e$, $-NR_eR_f$, $C_{1-6}$ alkyl, $C_{2-6}$ alkenyl and $C_{2-6}$ alkynyl; wherein, the $C_{1-6}$ alkyl, $C_{2-6}$ alkenyl, $C_{2-6}$ alkynyl, $R_e$ and $R_f$ are optionally substituted with one or more deuterium, halogen, CN, oxo, $C_{1-6}$ alkyl, $C_{1-6}$ haloalkyl, $C_{2-6}$ alkenyl, $C_{2-6}$ alkynyl, $-OR_h$ or $-NR_hR_i$;
each of $R_1$ and $R_2$ is the same or different, independently selected from $R_h$; or
$R_1$ and $R_2$, together with the atoms to which they are attached, form a 3-14 membered heteroaryl group or a $C_{3-14}$ carbocyclic group; wherein the 3-14 membered heteroaryl group or $C_{3-14}$ carbocyclic group is optionally substituted with one or more $R_h$;
$M_1$ is selected from the group consisting of $CR_c$ and N;
$M_3$, $M_4$, $M_7$, $M_8$ and $M_9$ are independently selected from the group consisting of $CR_cR_c$ and $NR_c$;
$M_2$, $M_5$, $M_6$ and $M_{10}$ are independently selected from the group consisting of absent, $CR_cR_c$, $-CR_cR_c-CR_cR_c-$ and $NR_c$;
$R_4$ is H, deuterium, halogen, $-NH_2$, $C_{1-6}$ alkyl, $C_{1-6}$ haloalkyl, $C_{2-6}$ alkenyl, $C_{2-6}$ haloalkenyl, $C_{2-6}$ alkynyl, $C_{2-6}$ halogenated alkynyl, $C_{1-6}$ alkoxy, $C_{1-6}$ haloalkoxy, $C_{3-6}$ cycloalkyl, $C_{3-6}$ halocycloalkyl or cyano;
$R_5$ is H, deuterium, halogen, $-NH_2$, -N=S(O)ReR$_f$, -OH, -C(=O)OH, $-C(=O)NH_2$, $C_{1-6}$ alkyl, $C_{2-6}$ alkenyl, $C_{2-6}$ alkynyl, $C_{1-6}$ alkoxy, $-NH(C_{1-6}$ alkyl), $-N(C_{1-6}$ alkyl$)_2$, $C_{3-8}$ cycloalkyl, 3-8 membered heterocyclyl, $C_{6-10}$ aryl or 5-10 membered heteroaryl; wherein, the $-NH_2$, -N=S(O)ReR$_f$, -OH, -C(=O)OH, $-C(=O)NH_2$, $C_{1-6}$ alkyl, $C_{2-6}$ alkenyl, $C_{2-6}$ alkynyl, $C_{1-6}$ alkoxy, $-NH(C_{1-6}$ alkyl), $-N(C_{1-6}$ alkyl$)_2$, $C_{3-8}$ cycloalkyl, 3-8 membered heterocyclyl, $C_{6-10}$ aryl and 5-10 membered heteroaryl are optionally substituted with one or more $R_h$;
each of $R_c$ is the same or different, and is independently selected from the group consisting of H, deuterium, halogen, CN, $NO_2$, oxo, $C_{1-6}$ alkyl, $C_{2-6}$ alkenyl, $C_{2-6}$ alkynyl, $-C_{0-6}$ alkylene $-C_{6-14}$ aryl, $-C_{0-6}$ alkylene-5-14 membered heteroaryl, $-C_{0-6}$ alkylene-$C_{3-14}$ carbocyclyl, $-C_{0-6}$ alkylene-3-14 membered heterocyclyl, $-OR_e$, $-NR_eR_f$, $-SR_e$, $-C(O)R_e$, $-N(R_e)C(O)R_f$, $-C(O)NR_eR_f$, $-N(R_e)C(O)OR_f$, $-OC(O)NR_eR_f$, -N(Re)C(O)NR$_f$R$_g$, $-S(O)R_e$, $-S(O)(=NR_e)R_f$, $-S(O)_2R_e$, $-S(O)NR_eR_f$, $-S(O)_2NR_eR_f$, $-N(R_e)S(O)R_f$, $-N(R_e)S(O)_2R_f$, $-C(O)OR_e$, $-OC(O)R_f$ and $-OC(O)OR_g$; wherein, the $C_{1-6}$ alkyl, $C_{2-6}$ alkenyl, $C_{2-6}$ alkynyl, $-C_{0-6}$ alkylene-$C_{6-14}$ aryl, $-C_{0-6}$ alkylene-5-14 membered heteroaryl, $-C_{0-6}$ alkylene-$C_{3-14}$ carbocyclyl, $-C_{0-6}$ alkylene-3-14 membered heterocyclyl, $R_e$, $R_f$ and $R_g$ are optionally substituted with one or more $R_h$, $-OR_h$, $-NR_hR_i$, $-C(O)OR_h$, $-OC(O)R_h$, $-COR_h$, $-C(O)NR_hR_i$;
$R_e$, $R_f$, $R_g$, $R_h$, $R_i$ are independently selected from the group consisting of hydrogen, deuterium, halogen, CN, OH, $NH_2$, -COOH, $-NH(C_{1-6}$ alkyl), $-N(C_{1-6}$ alkyl$)_2$, oxo, $C_{1-6}$ alkyl, $C_{1-6}$ alkoxy, $C_{2-6}$ alkenyl, $C_{2-6}$ alkynyl, $-O-C_{3-14}$ cycloalkyl, $-O-C_{3-14}$ heterocyclyl, $-S-C_{3-14}$ heterocyclyl, $-S-C_{3-14}$ cycloalkyl, $-S-C_{1-6}$ alkyl, $-CONH_2$, $-CONH(C_{1-6}$ alkyl), $-CON(C_{1-6}$ alkyl$)_2$, $-CONH(C_{2-6}$ alkenyl), $-CON(C_{2-6}$ alkenyl$)_2$, $-CONH(C_{2-6}$ alkynyl), $-CON(C_{2-6}$ alkynyl$)_2$, $-S(O)-C_{1-6}$ alkyl, $-S(O)_2-C_{1-6}$ alkyl, $-S(O)-C_{2-6}$ alkynyl, $-S(O)_2-C_{2-6}$ alkynyl, $-S(O)-C_{2-6}$ alkynyl, $-S(O)_2-C_{2-6}$ alkynyl, $-S(O)-C_{3-14}$ cycloalkyl, $-S(O)_2-C_{3-14}$ heterocyclyl, $-S(O)NH_2$, $-S(O)NHC_{1-6}$ alkyl, $-S(O)N(C_{1-6}$ alkyl$)_2$, -CHO, $-C(O)-C_{1-6}$ alkyl, $-C(O)-C_{2-6}$ alkenyl, $-C_{0-6}$ alkylene $-C_{6-14}$ aryl, $-C_{0-6}$ alkylene -5-14 membered heteroaryl, $-C_{0-6}$ alkylene $-C_{3-14}$ carbocyclyl and $-C_{0-6}$ alkylene -3-14 membered heterocyclyl; wherein, the $-NH(C_{1-6}$ alkyl), $-N(C_{1-6}$ alkyl$)_2$, $C_{1-6}$ alkyl, $C_{1-6}$ alkoxy, $C_{2-6}$ alkenyl, $C_{2-6}$ alkynyl, $-O-C_{3-14}$ cycloalkyl, $-O-C_{3-14}$ heterocyclyl, $-S-C_{3-14}$ heterocyclyl, $-S-C_{3-14}$ cycloalkyl, $-S-C_{1-6}$ alkyl, $-CONH(C_{1-6}$ alkyl), $-CON(C_{1-6}$ alkyl$)_2$, $-CONH(C_{2-6}$ alkenyl), $-CON(C_{2-6}$ alkenyl$)_2$, $-CONH(C_{2-6}$ alkenyl), $-CON(C_{2-6}$ alkenyl$)_2$, $-S(O)-C_{1-6}$ alkyl, $-S(O)_2-C_{1-6}$ alkyl, $-S(O)-C_{2-6}$ alkenyl, $-S(O)_2-C_{2-6}$ alkenyl, $-S(O)-C_{2-6}$ alkynyl, $-S(O)_2-C_{2-6}$ alkynyl, $-S(O)-C_{3-14}$ cycloalkyl, $-S(O)_2-C_{3-14}$ heterocyclyl, $-S(O)NHC_{1-6}$ alkyl, $-S(O)N(C_{1-6}$ alkyl$)_2$, $-C(O)-C_{1-6}$ alkyl, $-C(O)-C_{2-6}$ alkenyl, $-C_{0-6}$ alkylene $-C_{6-14}$ aryl, $-C_{0-6}$ alkylene -5-14 membered heteroaryl, $-C_{0-6}$ alkylene-$C_{3-14}$ cycloalkyl and $-C_{0-6}$ alkylene-3-14 membered heterocyclyl are substituted with one or more of hydrogen, deuterium, halogen, CN, OH, $NH_2$, -COOH, oxo, $C_{1-6}$

alkyl, $C_{1-6}$ haloalkyl, $C_{2-6}$ alkenyl, $C_{2-6}$ haloalkenyl, $C_{2-6}$ alkynyl, $C_{2-6}$ haloalkynyl, $C_{1-6}$ alkoxy, $C_{1-6}$ haloalkoxy, -NH($C_{1-6}$alkyl), -NH($C_{1-6}$ haloalkyl), -N($C_{1-6}$alkyl)$_2$, -N($C_{1-6}$ haloalkyl)$_2$, -N($C_{1-6}$ alkyl)($C_{1-6}$ haloalkyl), -S(O)-$C_{1-6}$ alkyl, -S(O)$_2$-$C_{1-6}$ alkyl, -S(O)-$C_{2-6}$ alkenyl, -S(O)$_2$-$C_{2-6}$ alkenyl, -S(O)-$C_{2-6}$ alkynyl, -S(O)$_2$-$C_{2-6}$ alkynyl, -S(O)-$C_{3-14}$ cycloalkyl, -S(O)$_2$-$C_{3-14}$ heterocyclyl, -S(O)NH$C_{1-6}$ alkyl, -S(O)N($C_{1-6}$ alkyl)$_2$, -COO($C_{1-6}$ alkyl), -COO($C_{1-6}$ haloalkyl), -CONH($C_{1-6}$ alkyl), -CONH($C_{1-6}$ haloalkyl), -CON($C_{1-6}$ alkyl)$_2$, -CON($C_{1-6}$ haloalkyl)$_2$, -CON($C_{1-6}$ alkyl)($C_{1-6}$ haloalkyl), -$C_{0-6}$ alkylene-NHC(=O) $C_{1-6}$ alkyl, -$C_{0-6}$ alkylene-NHC(=O) $C_{1-6}$ haloalkyl, -$C_{0-6}$ alkylene -NHC(=O) $C_{2-6}$ alkenyl, substituted or unsubstituted -$C_{0-6}$ alkylene-$C_{3-14}$ carbocyclyl, substituted or substituted -$C_{0-6}$ alkylene -3-14 membered heterocyclyl;
m is 0, 1, 2 or 3;
n is 0, 1, 2 or 3; or
r is 0, 1, 2, 3, 4 or 5.

**2.** The compound according to claim 1, or its stereoisomer, tautomer, solvate, pharmaceutically acceptable salt or deuterated derivative, wherein, $L_1$ is -$CR_hR_i$-.

**3.** The compound according to claim 1 or 2, or its stereoisomer, tautomer, solvate, pharmaceutically acceptable salt or deuterated derivative, wherein, $L_1$ is -$CH_2$-.

**4.** The compound according to any one of claims 1-3, or its stereoisomer, tautomer, solvate, pharmaceutically acceptable salt or deuterated derivative, wherein, $L_2$ is bond or -O-.

**5.** The compound according to any one of claims 1-4, or its stereoisomer, tautomer, solvate, pharmaceutically acceptable salt or deuterated derivative, wherein, $R_1$ or $R_2$ is independently selected from the group consisting of H, $C_{1-6}$ alkyl, deuterated $C_{1-6}$ alkyl, hydroxy, $C_{1-6}$ haloalkyl, halogen, -$NH_2$, $C_{1-6}$ alkoxy and $C_{1-6}$ haloalkoxy.

**6.** The compound according to any one of claims 1-5, or its stereoisomer, tautomer, solvate, pharmaceutically acceptable salt or deuterated derivative, wherein, $R_1$ or $R_2$ is H or $C_{1-3}$ alkyl.

**7.** The compound according to any one of claims 1-4, or its stereoisomer, tautomer, solvate, pharmaceutically acceptable salt or deuterated derivative, wherein, $R_1$ and $R_2$, together with the atoms to which they are attached, form

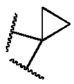, 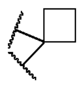,

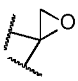, 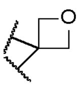, 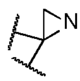 or 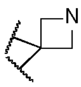.

**8.** The compound according to any one of claims 1-4, or its stereoisomer, tautomer, solvate, pharmaceutically acceptable salt or deuterated derivative, wherein,

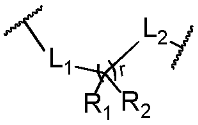 is 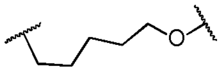, 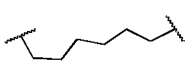, 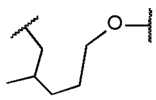, 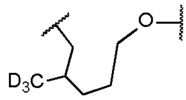,

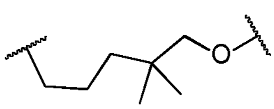, 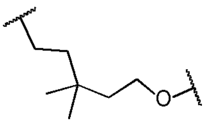 or 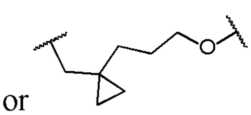.

**9.** The compound according to any one of claims 1-8, or its stereoisomer, tautomer, solvate, pharmaceutically acceptable salt or deuterated derivative, wherein, $R_a$ is H, deuterium, halogen, CN, OH, $NH_2$, $C_{1-6}$ alkyl, deuterated $C_{1-6}$ alkyl, $C_{2-6}$ alkenyl, $C_{2-6}$ alkynyl, $C_{1-6}$ alkoxy, $C_{1-6}$ haloalkyl or -NH-C(O)-$C_{1-6}$ alkyl.

**10.** The compound according to any one of claims 1-9, or its stereoisomer, tautomer, solvate, pharmaceutically acceptable salt or deuterated derivative, wherein, $R_a$ is H, deuterium, F, Cl, CN, $NH_2$, $C_{1-3}$ alkyl, deuterated $C_{1-3}$ alkyl, $C_{2-4}$ alkynyl, $C_{1-3}$ alkoxy or $C_{1-3}$ haloalkyl.

**11.** The compound according to any one of claims 1-10, or its stereoisomer, tautomer, solvate, pharmaceutically acceptable salt or deuterated derivative, wherein, $R_a$ is H or $CH_3$.

**12.** The compound according to any one of claims 1-11, or its stereoisomer, tautomer, solvate, pharmaceutically acceptable salt or deuterated derivative, wherein, $R_b$ is H, deuterium, halogen, CN, OH, $NH_2$, $C_{1-6}$ alkyl, deuterated $C_{1-6}$ alkyl, $C_{1-6}$ haloalkyl, $C_{1-6}$ alkoxy, $C_{1-6}$ haloalkoxy, -S-$C_{1-6}$ alkyl, -S(=O)-$CH_3$, -S(=O)$_2$-$C_{1-6}$ alkyl, -S(=O)$_2$-$C_{3-6}$ cycloalkyl or -O-3-6 membered heterocyclyl.

**13.** The compound according to any one of claims 1-12, or its stereoisomer, tautomer, solvate, pharmaceutically acceptable salt or deuterated derivative, wherein, $R_b$ is H, deuterium, F, Cl, -$OCH_3$, $C_{1-3}$ alkyl, $CD_3$, $CF_3$, CN, $NH_2$, -S-$CH_3$, -S(=O)-$CH_3$,

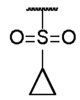 or 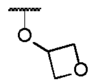.

**14.** The compound according to any one of claims 1-13, or its stereoisomer, tautomer, solvate, pharmaceutically acceptable salt or deuterated derivative, wherein, $R_b$ is H or $C_{1-3}$ alkyl; preferably, $R_b$ is H or $CH_3$.

**15.** The compound according to any one of claims 1-14, or its stereoisomer, tautomer, solvate, pharmaceutically acceptable salt or deuterated derivative, wherein, $R_4$ is H.

**16.** The compound according to any one of claims 1-15, or its stereoisomer, tautomer, solvate, pharmaceutically acceptable salt or deuterated derivative, wherein, $R_5$ is H, halogen, $C_{1-6}$ alkyl, -$NH_2$, -NH($C_{1-6}$ alkyl), -N($C_{1-6}$ alkyl)$_2$, $C_{3-6}$ cycloalkyl or 4-6 membered heterocyclyl; wherein, the $C_{1-6}$ alkyl, -$NH_2$, -NH($C_{1-6}$ alkyl), -N($C_{1-6}$ alkyl)$_2$, $C_{3-6}$ cycloalkyl and 4-6 membered heterocyclyl are optionally substituted with one or more OH, $NH_2$, oxo, $C_{1-6}$ alkyl, $C_{1-6}$ alkoxy, -S(O)$_2$-$C_{1-6}$ alkyl, -$C_{1-6}$ alkyl - S(O)$_2$-$C_{1-6}$ alkyl, -NH($C_{1-6}$ alkyl) or -N($C_{1-6}$ alkyl)$_2$.

**17.** The compound according to any one of claims 1-16, or its stereoisomer, tautomer, solvate, pharmaceutically acceptable salt or deuterated derivative, wherein, $R_5$ is H, F, Cl, $CH_3$, -$NH_2$,

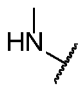, 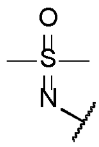, 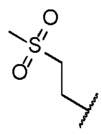, 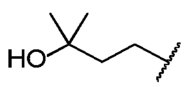, 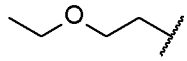,

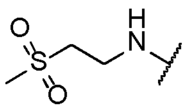, 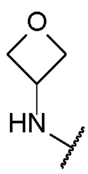, 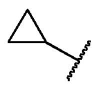, 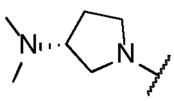, 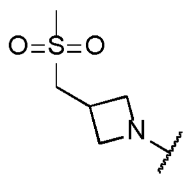, 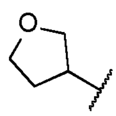,

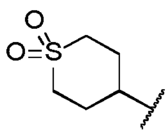, 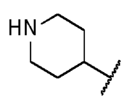, 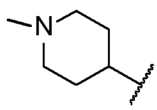 or 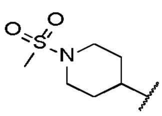.

**18.** The compound according to any one of claims 1-17, or its stereoisomer, tautomer, solvate, pharmaceutically acceptable salt or deuterated derivative, wherein, $R_5$ is H.

**19.** The compound according to any one of claims 1-18, or its stereoisomer, tautomer, solvate, pharmaceutically acceptable salt or deuterated derivative, wherein $R_c$ is H, deuterium, halogen, $NO_2$, CN, OH, $NH_2$, $C_{1-6}$ alkyl, $C_{2-6}$ alkenyl, $C_{2-6}$ alkynyl, $C_{1-6}$ alkoxy, $C_{1-6}$ haloalkyl, $C_{3-6}$ cycloalkyl or 3-6 membered heterocyclyl; wherein, the -OH, $NH_2$, $C_{1-6}$ alkyl, $C_{2-6}$ alkenyl, $C_{2-6}$ alkynyl, $C_{1-6}$ alkoxy, $C_{1-6}$ haloalkyl, $C_{3-6}$ cycloalkyl and 3-6 membered heterocyclyl are optionally substituted with one or more halogen, $C_{1-6}$ alkyl, $C_{1-6}$ alkoxy, $NH_2$, -COOH, -NH($C_{1-6}$ alkyl), -N($C_{1-6}$ alkyl)$_2$, $C_{3-6}$ cycloalkyl, 3-6 membered heterocyclyl, -$CONH_2$, -CONH($C_{1-6}$ alkyl), -CON($C_{1-6}$ alkyl)$_2$, -CONH($C_{2-6}$ alkenyl), -CON($C_{2-6}$ alkenyl)$_2$, -CONH($C_{2-6}$ alkynyl), -CON($C_{2-6}$ alkynyl)$_2$, substituted or unsubstituted CO-3-6 membered heterocyclyl, -O-$C_{1-6}$ haloalkyl, -OC(=O)$NH_2$, -OCONH($C_{1-6}$ alkyl) or -OCON($C_{1-6}$ alkyl)$_2$.

**20.** The compound according to any one of claims 1-19, or its stereoisomer, tautomer, solvate, pharmaceutically acceptable salt or deuterated derivative, wherein, $R_c$ is H, deuterium, $CH_3$, $CD_3$, $CH_2CH_3$, $CH(CH_3)_2$, -$C(CH_3)_3$, OH, F, Cl, $NH_2$,

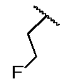,

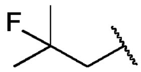, 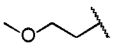, 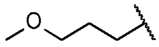, 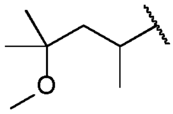, 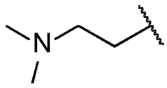, 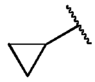,

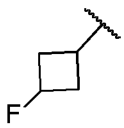, 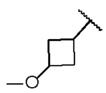, 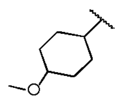, 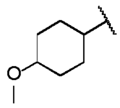, 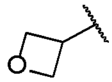, 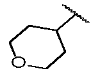, 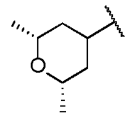,

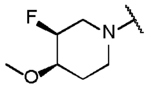, 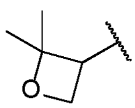, 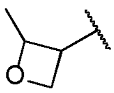, 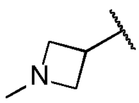, 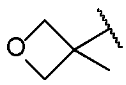, 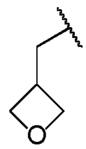, 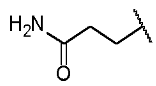,

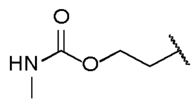, 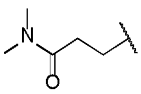, 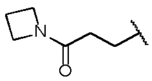, 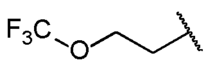, 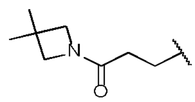

or

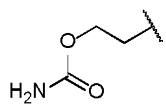.

**21.** The compound according to any one of claims 1-20, or its stereoisomer, tautomer, solvate, pharmaceutically acceptable salt or deuterated derivative, wherein,

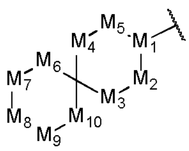 is 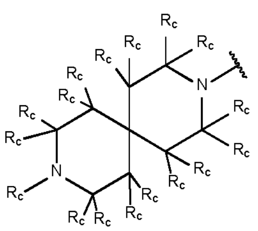, 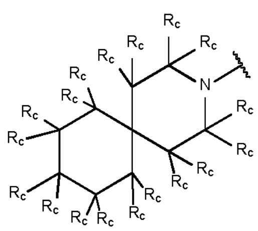 or 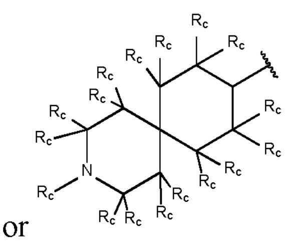.

**22.** The compound according to any one of claims 1-21, or its stereoisomer, tautomer, solvate, pharmaceutically acceptable salt or deuterated derivative, wherein,

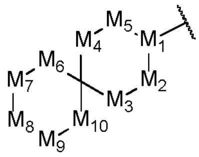 is 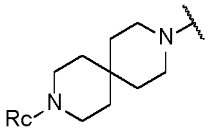, 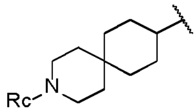 or 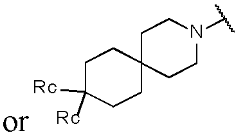.

**23.** The compound according to any one of claims 1-22, or its stereoisomer, tautomer, solvate, pharmaceutically acceptable salt or deuterated derivative, wherein,

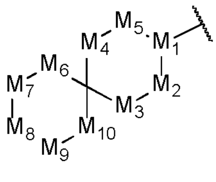 is 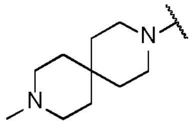, 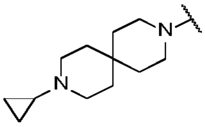, 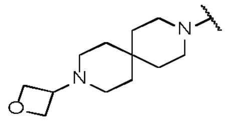,

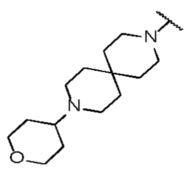, 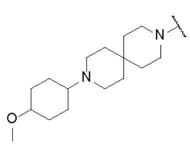, 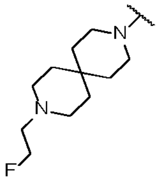, 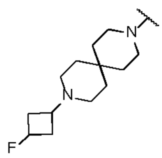, 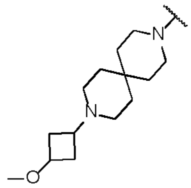,

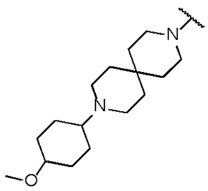, 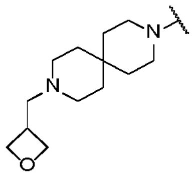, 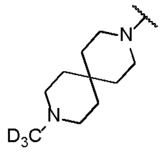, 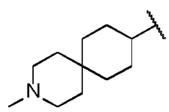, 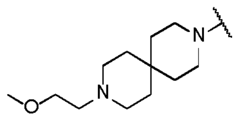,

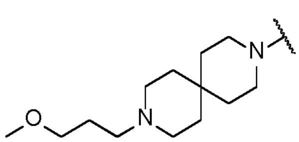, 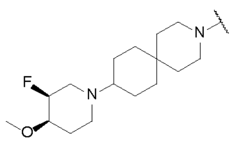, 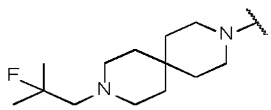, 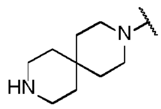,

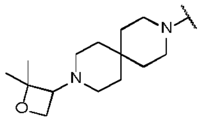, 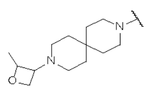, 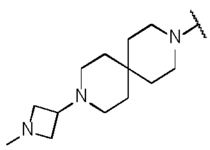, 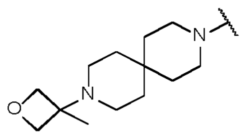,

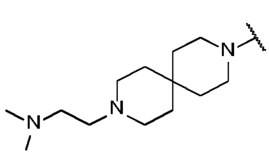, 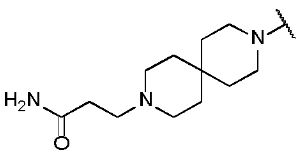, 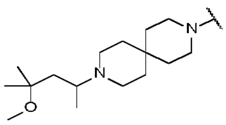,

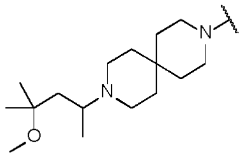, 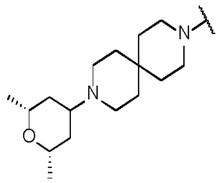, 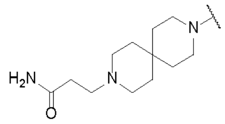, 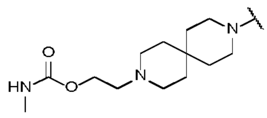,

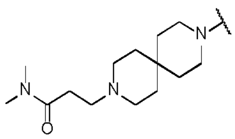, 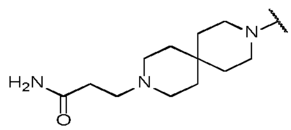, 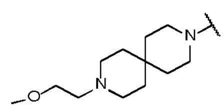, 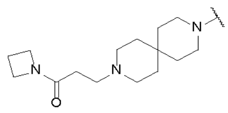,

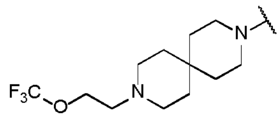, 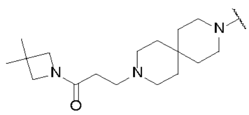 or 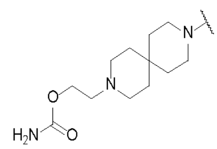.

**24.** The compound according to any one of claims 1-23, or its stereoisomer, tautomer, solvate, pharmaceutically acceptable salt or deuterated derivative, wherein, m is 0, 1 or 2.

**25.** The compound according to any one of claims 1-24, or its stereoisomer, tautomer, solvate, pharmaceutically acceptable salt or deuterated derivative, wherein, n is 0, 1 or 2.

**26.** The compound according to any one of claims 1-25, or its stereoisomer, tautomer, solvate, pharmaceutically acceptable salt or deuterated derivative, wherein, r is 2, 3 or 4.

**27.** The compound according to any one of claims 1-26, or its stereoisomer, tautomer, solvate, pharmaceutically acceptable salt or deuterated derivative, wherein, the compound of formula (I) is selected from a compound of formula (II) as shown below:

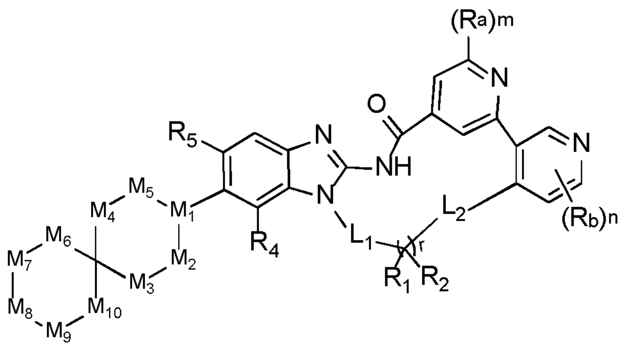

formula (II)

wherein, $L_1$, $L_2$, $R_a$, $R_b$, $R_1$, $R_2$, $R_4$, $R_3$, $M_1$, $M_2$, $M_3$, $M_4$, $M_5$, $M_6$, $M_7$, $M_8$, $M_9$, $M_{10}$, m, n and r in the compound of formula (II) are as defined in any one of claims 1-26.

**28.** The compound according to any one of claims 1-26, or its stereoisomer, tautomer, solvate, pharmaceutically acceptable salt or deuterated derivative, wherein, the compound of formula (I) is selected from a compound of formula (III) as shown below:

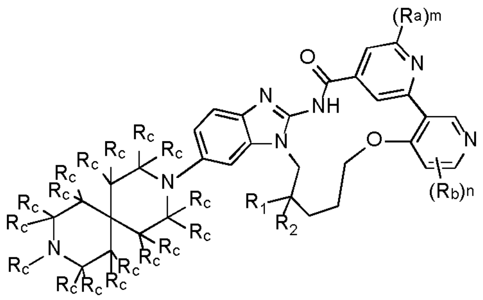

formula (III)

wherein, $R_a$, $R_b$, $R_c$, $R_1$, $R_2$, m and n in the compound of formula (III) are as defined in any one of claims 1-26.

**29.** The compound according to any one of claims 1-26, or its stereoisomer, tautomer, solvate, pharmaceutically acceptable salt or deuterated derivative, wherein, the compound of formula (I) is selected from a compound of formula (IV) as shown below:

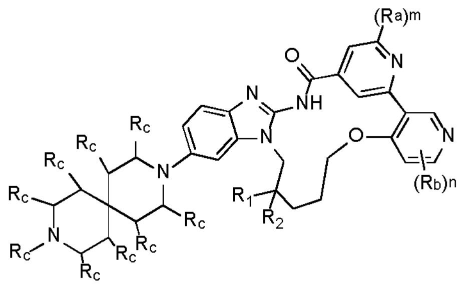

formula (IV)

wherein, $R_a$, $R_b$, $R_c$, $R_1$, $R_2$, m and n in the compound of formula (IV) are as defined in any one of claims 1-26.

**30.** The compound according to any one of claims 1-26, or its stereoisomer, tautomer, solvate, pharmaceutically acceptable salt or deuterated derivative, wherein, the compound of formula (I) is selected from a compound of formula (V) as shown below:

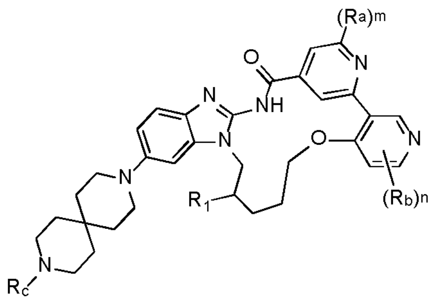

formula (V)

wherein, $R_a$, $R_b$, $R_c$, $R_1$, m and n in the compound of formula (V) are as defined in any one of claims 1-26.

**31.** The compound according to claim 1, or its stereoisomer, tautomer, solvate, pharmaceutically acceptable salt or deuterated derivative, wherein, the compound is selected from the group consisting of:

$2^6$-methyl-$5^6$-(9-methyl-3,9-diazaspiro[5.5]undecan-3-yl)-$5^1$H-11-oxa-4-aza-5(2, 1)-benzo[d]imidazola-1(3,4),2(2,4)-dipyridinacycloundecaphan-3-one;
$2^6$-methyl-$5^6$-(9-methyl-3,9-diazaspiro[5.5]undecan-3-yl)-$5^1$H-4-aza-5(2,1)-benz o[d]imidazola-1(3,4),2(2,4)-dipyridinacycloundecaphan-3-one;
(R)-$2^6$,7-dimethyl-$5^6$-(9-methyl-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-one;
(R)-$2^6$,7-dimethyl-$5^6$-(9-(oxetan-3-yl)-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-one;

(R)-$5^{5}$-fluoro-$2^{6}$,7-dimethyl-$5^{6}$-(9-methyl-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-o ne;

(R)-$5^{5}$-fluoro-$2^{6}$,7-dimethyl-$5^{6}$-(9-(oxetan-3-yl)-3,9-diazaspiro[5.5]undecan-3-yl) -$5^{1}$H-11-oxa-4-aza-5(2,1)-benzo[d]imidazola-1(3,4),2(2,4)-dipyridinacycloundecapha;

$2^{6}$,9,9-trimethyl-$5^{6}$-(9-methyl-3,9-diazaspiro[5.5]undecan-3-yl)-$5^{1}$H-11-oxa-4-az a-5(2,1)-benzo[d]imidazola-1(3,4),2(2,4)-dipyridinacycloundecaphan-3-one;

(R)-$5^{5}$-((dimethyl(oxo)-$1^{6}$-sulfanylidene)amino)-$2^{6}$,7-dimethyl-$5^{6}$-(9-methyl-3,9-diazaspiro[5.5]undecan-3-yl)-$5^{1}$H-11-oxa-4-aza-5(2,1)-benzo[d]imidazola-1(3,4),2(2, 4)-dipyridinacycloundecaphan-3-one;

(R)-$1^{6}$-fluoro-$2^{6}$,7-dimethyl-$5^{6}$-(3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

(R)-$1^{6}$-fluoro-$2^{6}$,7-dimethyl-$5^{6}$-(9-methyl-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-o ne;

(R)-$2^{6}$,$5^{5}$,7-trimethyl-$5^{6}$-(9-methyl-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

(R)-$2^{6}$,$5^{5}$,7-trimethyl-$5^{6}$-(9-(oxetan-3-yl)-3,9-diazaspiro[5.5]undecan-3-yl)-$5^{1}$H-1 1-oxa-4-aza-5(2,1)-benzo[d]imidazola-1(3,4),2(2,4)-dipyridinacycloundecaphan-3-on e;

$2^{6}$,$5^{5}$-dimethyl-$5^{6}$-(9-methyl-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

$5^{5}$-fluoro-$2^{6}$-methyl-$5^{6}$-(9-methyl-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

$5^{6}$-(9-((3S,4R)-3-fluoro-4-methoxypiperidin-1-yl)-3-azaspiro[5.5]undecan-3-yl)-$2^{6}$-methyl-$5^{1}$H-11-oxa-4-aza-5(2,1)-benzo[d]imidazola-1(3,4),2(2,4)-dipyridinacyclou ndecaphan-3-one;

(R)-$1^{6}$,$5^{5}$-difluoro-$5^{6}$-(9-(3-methoxypropyl)-3,9-diazaspiro[5.5]undecan-3-yl)-26, 7-dimethyl-$5^{1}$H-11-oxa-4-aza-5(2,1)-benzo[d]imidazola-1(3,4),2(2,4)-dipyridinacyclo undecaphan-3-one;

$1^{6}$-fluoro-$5^{6}$-(9-(3-methoxypropyl)-3,9-diazaspiro[5.5]undecan-3-yl)-$2^{6}$-methyl-5 $^{1}$H-11-oxa-4-aza-5(2,1)-benzo[d]imidazola-1(3,4),2(2,4)-dipyridinacycloundecaphan-3-one;

3-(9-($2^{6}$-methyl-3-oxo-$5^{1}$H-11-oxa-4-aza-5(2,1)-benzo[d]imidazola-1(3,4),2(2,4) -dipyridinacycloundecaphane-$5^{6}$-yl)-3,9-diazaspiro[5.5]undecan-3-yl)propanamide;

(R)-$2^{6}$,7-dimethyl-$5^{6}$-(9-methyl-3,9-diazaspiro[5.5]undecan-3-yl)-$5^{5}$-((2-(methyls ulfonyl)ethyl)amino)-$5^{1}$H-11-oxa-4-aza-5(2,1)-benzo[d]imidazola-1(3,4),2(2,4)-dipyri dinacycloundecaphan-3-one;

(R)-$2^{6}$,7-dimethyl-$5^{6}$-(9-methyl-3,9-diazaspiro[5.5]undecan-3-yl)-$5^{5}$-(methylami no)-$5^{1}$H-11-oxa-4-aza-5(2,1)-benzo[d]imidazola-1(3,4),2(2,4)-dipyridinacycloundeca phan-3-one;

(R)-7-methyl-$2^{6}$-(methyl-$d_3$)-$5^{6}$-(9-(oxetan-3-yl)-3,9-diazaspiro[5.5]undecan-3-yl )-$5^{1}$H-11-oxa-4-aza-5(2,1)-benzo[d]imidazola-1(3,4),2(2,4)-dipyridinacycloundecaph an-3-one;

(R)-$1^{6}$-fluoro-$5^{6}$-(9-(2-methoxyethyl)-3,9-diazaspiro[5.5]undecan-3-yl)-$2^{6}$,7-dim ethyl-$5^{1}$H-11-oxa-4-aza-5(2,1)-benzo[d]imidazola-1(3,4),2(2,4)-dipyridinacycloundec aphan-3-one;

$1^{6}$,$2^{6}$-dimethyl-$5^{6}$-(9-(oxetan-3-yl)-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

$1^{2}$,$2^{6}$-dimethyl-$5^{6}$-(9-(oxetan-3-yl)-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

$1^{2}$-fluoro-$2^{6}$-methyl-$5^{6}$-(9-(oxetan-3-yl)-3,9-diazaspiro[5.5]undecan-3-yl)-$5^{1}$H-11 -oxa-4-aza-5(2,1)-benzo[d]imidazola-1(3,4),2(2,4)-dipyridinacycloundecaphan-3-one;

$1^{2}$-fluoro-$1^{5}$,$2^{6}$-dimethyl-$5^{6}$-(9-(oxetan-3-yl)-3,9-diazaspiro[5.5]undecan-3-yl)-$5^{1}$ H-11-oxa-4-aza-5(2,1)-benzo[d]imidazola-1(3,4),2(2,4)-dipyridinacycloundecaphan-3 -one;

$2^{6}$-methyl-$5^{6}$-(9-(oxetan-3-yl)-3,9-diazaspiro[5.5]undecan-3-yl)-$1^{6}$-(trifluorometh yl)-$5^{1}$H-11-oxa-4-aza-5(2,1)-benzo[d]imidazola-1(3,4),2(2,4)-dipyridinacycloundecap han-3-one;

$1^{5}$,$2^{6}$-dimethyl-$5^{6}$-(9-(oxetan-3-yl)-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

$1^{6}$-amino-$2^{6}$-methyl-$5^{6}$-(9-(oxetan-3-yl)-3,9-diazaspiro[5.5]undecan-3-yl)-$5^{1}$H-11 -oxa-4-aza-5(2,1)-benzo[d]imidazola-1(3,4),2(2,4)-dipyridinacycloundecaphan-3-one;

$1^{6}$-fluoro-$2^{6}$-methyl-$5^{6}$-(9-(oxetan-3-yl)-3,9-diazaspiro[5.5]undecan-3-yl)-$5^{1}$H-11 -oxa-4-aza-5(2,1)-benzo[d]imidazola-1(3,4),2(2,4)-dipyridinacycloundecaphan-3-one;

$1^{6}$-chloro-$2^{6}$-methyl-$5^{6}$-(9-(oxetan-3-yl)-3,9-diazaspiro[5.5]undecan-3-yl)-$5^{1}$H-1 1-oxa-4-aza-5(2,1)-benzo[d]imidazola-1(3,4),2(2,4)-dipyridinacycloundecaphan-3-on e;

$1^{5}$-fluoro-$2^{6}$-methyl-$5^{6}$-(9-(oxetan-3-yl)-3,9-diazaspiro[5.5]undecan-3-yl)-$5^{1}$H-11 -oxa-4-aza-5(2,1)-benzo[d]imidazola-1(3,4),2(2,4)-dipyridinacycloundecaphan-3-one;

$1^{6}$-methoxy-$2^{6}$-methyl-$5^{6}$-(9-(oxetan-3-yl)-3,9-diazaspiro[5.5]undecan-3-yl)-$5^{1}$H -11-oxa-4-aza-5(2,1)-benzo

[d]imidazola-1(3,4),2(2,4)-dipyridinacycloundecaphan-3-o ne;

$1^5$-methoxy-$2^6$-methyl-$5^6$-(9-(oxetan-3-yl)-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-o ne;

$2^6$-methyl-$5^6$-(9-(oxetan-3-yl)-3,9-diazaspiro[5.5]undecan-3-yl)-3-oxo-$5^1$H-11-oxa-4-aza-5(2,1)-benzo[d]imidazola-1(3,4),2(2,4)-dipyridinacycloundecaphane-$1^2$-carbo nitrile;

$2^6$-methyl-$5^6$-(9-(oxetan-3-yl)-3,9-diazaspiro[5.5]undecan-3-yl)-3-oxo-$5^1$H-11-oxa-4-aza-5(2,1)-benzo[d]imidazola-1(3,4),2(2,4)-dipyridinacycloundecaphane-$1^6$-carbo nitrile;

$2^6$-methyl-$5^6$-(9-(oxetan-3-yl)-3,9-diazaspiro[5.5]undecan-3-yl)-3-oxo-$5^1$H-11-oxa-4-aza-5(2,1)-benzo[d]imidazola-1(3,4),2(2,4)-dipyridinacycloundecaphane-$1^5$-carbo nitrile;

$2^6$-methyl-$5^6$-(9-(oxetan-3-yl)-3,9-diazaspiro[5.5]undecan-3-yl)-$1^5$-(oxetan-3-yloxy)-$5^1$H-11-oxa-4-aza-5(2,1)-benzo[d]imidazola-1(3,4),2(2,4)-dipyridinacycloundeca phan-3-one;

$1^5$-fluoro-$1^6$-methoxy-$2^6$-methyl-$5^6$-(9-(oxetan-3-yl)-3,9-diazaspiro[5.5]undecan-3-yl)-$5^1$H-11-oxa-4-aza-5(2,1)-benzo[d]imidazola-1(3,4),2(2,4)-dipyridinacycloundec aphan-3-one;

(R)-$5^6$-(9-(3-methoxycyclobutyl)-3,9-diazaspiro[5.5]undecan-3-yl)-$2^6$,7-dimethyl-$5^1$H-11-oxa-4-aza-5(2,1)-benzo[d]imidazola-1(3,4),2(2,4)-dipyridinacycloundecapha n-3-one;

(R)-$2^6$,7-dimethyl-$5^6$-(9-(tetrahydro-2H-pyran-4-yl)-3,9-diazaspiro[5.5]undecan-3-yl)-$5^1$H-11-oxa-4-aza-5(2,1)-benzo[d]imidazola-1(3,4),2(2,4)-dipyridinacycloundec aphan-3-one;

(R)-$5^6$-(9-(2-fluoroethyl)-3,9-diazaspiro[5.5]undecan-3-yl)-$2^6$,7-dimethyl-51H-11-oxa-4-aza-5(2,1)-benzo[d]imidazola-1(3,4),2(2,4)-dipyridinacycloundecaphan-3-on e;

(R)-$5^6$-(9-(3-fluorocyclobutyl)-3,9-diazaspiro[5.5]undecan-3-yl)-$2^6$,7-dimethyl-5 $^1$H-11-oxa-4-aza-5(2,1)-benzo[d]imidazola-1(3,4),2(2,4)-dipyridinacycloundecaphan-3-one;

(R)-$5^6$-(9-(4-methoxycyclohexyl)-3,9-diazaspiro[5.5]undecan-3-yl)-$2^6$,7-dimethyl-$5^1$H-11-oxa-4-aza-5(2,1)-benzo[d]imidazola-1(3,4),2(2,4)-dipyridinacycloundecapha n-3-one;

(R)-$5^6$-(9-cyclopropyl-3,9-diazaspiro[5.5]undecan-3-yl)-$2^6$,7-dimethyl-$5^1$H-11-oxa-4-aza-5(2,1)-benzo[d]imidazola-1(3,4),2(2,4)-dipyridinacycloundecaphan-3-one;

(R)-$5^5$-fluoro-$5^6$-(9-(3-methoxycyclobutyl)-3,9-diazaspiro[5.5]undecan-3-yl)-$2^6$,7-dimethyl-$5^1$H-11-oxa-4-aza-5(2,1)-benzo[d]imidazola-1(3,4),2(2,4)-dipyridinacyclou ndecaphan-3-one;

(R)-$5^5$-fluoro-$5^6$-(9-(2-fluoroethyl)-3,9-diazaspiro[5.5]undecan-3-yl)-$2^6$,7-dimethyl-$5^1$H-11-oxa-4-aza-5(2,1)-benzo[d]imidazola-1(3,4),2(2,4)-dipyridinacycloundecap han-3-one;

(R)-$5^5$-fluoro-$2^6$,7-dimethyl-$5^6$-(9-(tetrahydro-2H-pyran-4-yl)-3,9-diazaspiro[5.5]undecan-3-yl)-$5^1$H-11-oxa-4-aza-5(2,1)-benzo[d]imidazola-1(3,4),2(2,4)-dipyridinacy cloundecaphan-3-one;

(R)-$5^5$-fluoro-$5^6$-(9-(4-methoxycyclohexyl)-3,9-diazaspiro[5.5]undecan-3-yl)-$2^6$,7-dimethyl-$5^1$H-11-oxa-4-aza-5(2,1)-benzo[d]imidazola-1(3,4),2(2,4)-dipyridinacyclo undecaphan-3-one;

(R)-$5^6$-(9-cyclopropyl-3,9-diazaspiro[5.5]undecan-3-yl)-$5^5$-fluoro-$2^6$,7-dimethyl-$5^1$H-11-oxa-4-aza-5(2,1)-benzo[d]imidazola-1(3,4),2(2,4)-dipyridinacycloundecaphan -3-one;

(R)-$5^6$-(9-(3-methoxycyclobutyl)-3,9-diazaspiro[5.5]undecan-3-yl)-$2^6$,$5^5$,7-trimethyl-$5^1$H-11-oxa-4-aza-5(2,1)-benzo[d]imidazola-1(3,4),2(2,4)-dipyridinacycloundeca phan-3-one;

$2^6$-methyl-$5^6$-(9-(oxetan-3-yl)-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-one;

$2^6$-methyl-$5^6$-(9-(oxetan-3-yl)-3,9-diazaspiro[5.5]undecan-3-yl)-$5^1$H-10-oxa-4-aza-5(2,1)-benzo[d]imidazola-1(3,4),2(2,4)-dipyridinacyclodecaphan-3-one;

$5^6$-(9-(3-methoxycyclobutyl)-3,9-diazaspiro[5.5]undecan-3-yl)-$2^6$-methyl-$5^1$H-11-oxa-4-aza-5(2,1)-benzo[d]imidazola-1(3,4),2(2,4)-dipyridinacycloundecaphan-3-one;

$5^6$-(9-(3-fluorocyclobutyl)-3,9-diazaspiro[5.5]undecan-3-yl)-$2^6$,$5^5$-dimethyl-$5^1$H-11-oxa-4-aza-5(2,1)-benzo[d]imidazola-1(3,4),2(2,4)-dipyridinacycloundecaphan-3-o ne;

$5^6$-(9-cyclopropyl-3,9-diazaspiro[5.5]undecan-3-yl)-$2^6$,$5^5$-dimethyl-$5^1$H-11-oxa-4 -aza-5(2,1)-benzo[d]imidazola-1(3,4),2(2,4)-dipyridinacycloundecaphan-3-one;

$5^6$-(9-(3-methoxycyclobutyl)-3,9-diazaspiro[5.5]undecan-3-yl)-$2^6$,$5^5$-dimethyl-$5^1$ H-11-oxa-4-aza-5(2,1)-benzo[d]imidazola-1(3,4),2(2,4)-dipyridinacycloundecaphan-3 -one;

$2^6$,$5^5$-dimethyl-$5^6$-(9-(oxetan-3-ylmethyl)-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-o ne;

$5^6$-(9-(2-fluoroethyl)-3,9-diazaspiro[5.5]undecan-3-yl)-$2^6$,$5^5$-dimethyl-$5^1$H-11-oxa-4-aza-5(2,1)-benzo[d]imidazola-1(3,4),2(2,4)-dipyridinacycloundecaphan-3-one;

$5^6$-(9-(2-fluoroethyl)-3,9-diazaspiro[5.5]undecan-3-yl)-$2^6$,$5^5$-dimethyl-$5^1$H-12-oxa-4-aza-5(2,1)-benzo[d]imidazola-1(3,4),2(2,4)-dipyridinacyclododecaphan-3-one;

$5^5$-fluoro-$5^6$-(9-(2-fluoroethyl)-3,9-diazaspiro[5.5]undecan-3-yl)-$2^6$-methyl-$5^1$H-11-oxa-4-aza-5(2,1)-benzo[d]imidazola-1(3,4),2(2,4)-dipyridinacycloundecaphan-3-o ne;

$5^5$-fluoro-$2^6$-methyl-$5^6$-(9-(oxetan-3-ylmethyl)-3,9-diazaspiro[5.5]undecan-3-yl)-$5^1$H-11-oxa-4-aza-5(2,1)-benzo[d]imidazola-1(3,4),2(2,4)-dipyridinacycloundecaphan -3-one;

$5^{5}$-fluoro-$2^{6}$-methyl-$5^{6}$-(9-(oxetan-3-yl)-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

$5^{5}$-fluoro-$5^{6}$-(9-(3-fluorocyclobutyl)-3,9-diazaspiro[5.5]undecan-3-yl)-$2^{6}$-methyl-$5^{1}$H-11-oxa-4-aza-5(2,1)-benzo[d]imidazola-1(3,4),2(2,4)-dipyridinacycloundecaphan -3-one;

$5^{6}$-(9-cyclopropyl-3,9-diazaspiro[5.5]undecan-3-yl)-$5^{5}$-fluoro-$2^{6}$-methyl-$5^{1}$H-11-oxa-4-aza-5(2,1)-benzo[d]imidazola-1(3,4),2(2,4)-dipyridinacycloundecaphan-3-one;

(R)-$2^{6}$,7-bis(methyl-$d_3$)-$5^{6}$-(9-(methyl-$d_3$)-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-o ne;

$2^{6}$-methyl-$5^{6}$-(9-(methyl-$d_3$)-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

(R)-$5^{5}$-fluoro-$2^{6}$,7-dimethyl-$5^{6}$-(9-(methyl-$d_3$)-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan -3-one;

$5^{5}$-fluoro-$2^{6}$-methyl-$5^{6}$-(9-(methyl-$d_3$)-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

$2^{6}$,$5^{5}$-dimethyl-$5^{6}$-(9-(methyl-$d_3$)-3,9-diazaspiro[5.5]undecan-3-yl)-$5^{1}$H-11-oxa-4 -aza-5(2,1)-benzo[d]imidazola-1(3,4),2(2,4)-dipyridinacycloundecaphan-3-one;

(R)-$2^{6}$,$5^{5}$,7-trimethyl-$5^{6}$-(9-(methyl-$d_3$)-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-one;

$2^{6}$-methyl-$5^{6}$-(3-methyl-3-azaspiro[5.5]undecan-9-yl)-$5^{1}$H-4-aza-5(2,1)-benzo[d] imidazola-1(3,4),2(2,4)-dipyridinacycloundecaphan-3-one;

$5^{6}$-(9-(2-methoxyethyl)-3,9-diazaspiro[5.5]undecan-3-yl)-$2^{6}$-methyl-$5^{1}$H-4-aza-5 (2,1)-benzo[d]imidazola-1(3,4),2(2,4)-dipyridinacycloundecaphan-3-one;

(R)-$5^{6}$-(9-(3-methoxypropyl)-3,9-diazaspiro[5.5]undecan-3-yl)-$2^{6}$,7-dimethyl-$5^{1}$ H-4-aza-5(2,1)-benzo[d]imidazola-1(3,4),2(2,4)-dipyridinacycloundecaphan-3-one;

$5^{6}$-(9-(2-methoxyethyl)-3,9-diazaspiro[5.5]undecan-3-yl)-$2^{6}$-methyl-$5^{1}$H-11-oxa-4-aza-5(2,1)-benzo[d]imidazola-1(3,4),2(2,4)-dipyridinacycloundecaphan-3-one;

$5^{6}$-(9-(3-methoxypropyl)-3,9-diazaspiro[5.5]undecan-3-yl)-$2^{6}$-methyl-$5^{1}$H-11-ox a-4-aza-5(2,1)-benzo[d]imidazola-1(3,4),2(2,4)-dipyridinacycloundecaphan-3-one;

(R)-$5^{6}$-(9-(3-methoxypropyl)-3,9-diazaspiro[5.5]undecan-3-yl)-$2^{6}$,7-dimethyl-$5^{1}$ H-11-oxa-4-aza-5(2,1)-benzo[d]imidazola-1(3,4),2(2,4)-dipyridinacycloundecaphan-3 -one;

$5^{6}$-(9-(2-fluoro-2-methylpropyl)-3,9-diazaspiro[5.5]undecan-3-yl)-$2^{6}$-methyl-$5^{1}$H-11-oxa-4-aza-5(2,1)-benzo[d]imidazola-1(3,4),2(2,4)-dipyridinacycloundecaphan-3-o ne;

(R)-$5^{5}$-amino-$2^{6}$,7-dimethyl-$5^{6}$-(9-methyl-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan-3-o ne;

(R)-$1^{6}$-fluoro-$5^{6}$-(9-(2-fluoroethyl)-3,9-diazaspiro[5.5]undecan-3-yl)-$2^{6}$,7-dimethyl-$5^{1}$H-11-oxa-4-aza-5(2,1)-benzo[d]imidazola-1(3,4),2(2,4)-dipyridinacycloundecap han-3-one;

(R)-$1^{6}$-fluoro-$2^{6}$,7-dimethyl-$5^{6}$-(9-(oxetan-3-yl)-3,9-diazaspiro[5.5]undecan-3-yl)-$5^{1}$H-11-oxa-4-aza-5(2,1)-benzo[d]imidazola-1(3,4),2(2,4)-dipyridinacycloundecapha n-3-one

(7R)-$5^{6}$-(9-(2,2-dimethyloxetan-3-yl)-3,9-diazaspiro[5.5]undecan-3-yl)-$2^{6}$,7-dimethyl-$5^{1}$H-11-oxa-4-aza-5(2,1)-benzo[d]imidazola-1(3,4),2(2,4)-dipyridinacycloundec aphan-3-one;

(7R)-$2^{6}$,7-dimethyl-$5^{6}$-(9-(2-methyloxetan-3-yl)-3,9-diazaspiro[5.5]undecan-3-yl)-$5^{1}$H-11-oxa-4-aza-5(2,1)-benzo[d]imidazola-1(3,4),2(2,4)-dipyridinacycloundecaph an-3-one;

(R)-$2^{6}$,7-dimethyl-$5^{6}$-(9-(1-methylazetidin-3-yl)-3,9-diazaspiro[5.5]undecan-3-yl)-$5^{1}$H-11-oxa-4-aza-5(2,1)-benzo[d]imidazola-1(3,4),2(2,4)-dipyridinacycloundecaph an-3-one;

(R)-$2^{6}$,7-dimethyl-$5^{6}$-(9-(3-methyloxetan-3-yl)-3,9-diazaspiro[5.5]undecan-3-yl)-$5^{1}$H-11-oxa-4-aza-5(2,1)-benzo[d]imidazola-1(3,4),2(2,4)-dipyridinacycloundecaphan -3-one;

(R)-$5^{5}$-fluoro-$5^{6}$-(9-(3-methoxypropyl)-3,9-diazaspiro[5.5]undecan-3-yl)-$2^{6}$,7-dimethyl-$5^{1}$H-11-oxa-4-aza-5(2,1)-benzo[d]imidazola-1(3,4),2(2,4)-dipyridinacyclound ecaphan-3-one;

$5^{6}$-(9-(2-(dimethylamino)ethyl)-3,9-diazaspiro[5.5]undecan-3-yl)-$2^{6}$-methyl-$5^{1}$H-11-oxa-4-aza-5(2,1)-benzo[d]imidazola-1(3,4),2(2,4)-dipyridinacycloundecaphan-3-o ne;

(7R)-$5^{6}$-(9-(4-methoxy-4-methylpentan-2-yl)-3,9-diazaspiro[5.5]undecan-3-yl)-$2^{6}$,7-dimethyl-$5^{1}$H-11-oxa-4-aza-5(2,1)-benzo[d]imidazola-1(3,4),2(2,4)-dipyridinacycl oundecaphan-3-one;

$5^{6}$-(9-cyclopropyl-3,9-diazaspiro[5.5]undecan-3-yl)-$2^{6}$-methyl-$5^{1}$H-11-oxa-4-aza-5(2,1)-benzo[d]imidazola-1(3,4),2(2,4)-dipyridinacycloundecaphan-3-one;

$5^{6}$-(9-(4-methoxy-4-methylpentan-2-yl)-3,9-diazaspiro[5.5]undecan-3-yl)-$2^{6}$-methyl-$5^{1}$H-11-oxa-4-aza-5(2,1)-benzo[d]imidazola-1(3,4),2(2,4)-dipyridinacycloundeca phan-3-one;

$5^{6}$-(9-((2R,6S)-2,6-dimethyltetrahydro-2H-pyran-4-yl)-3,9-diazaspiro[5.5]undecan-3-yl)-$2^{6}$-methyl-$5^{1}$H-11-

oxa-4-aza-5(2,1)-benzo[d]imidazola-1(3,4),2(2,4)-dipyridin acycloundecaphan-3-one;
$5^5$-chloro-$2^6$-methyl-$5^6$-(9-methyl-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-one;
(R)-$5^5$-chloro-$2^6$,7-dimethyl-$5^6$-(9-methyl-3,9-diazaspiro[5.5]undecan-3-yl)-$5^1$H-11-oxa-4-aza-5(2,1)-benzo[d]imidazola-1(3,4),2(2,4)-dipyridinacycloundecaphan-3-o ne;
6'-methyl-6'-(9-methyl-3,9-diazaspiro[5.5]undecan-3-yl)spiro[cyclopropane-1,7'-11-oxa-4-aza-5(2,1)-benzo[d]imidazola-1(3,4),2(2,4)-dipyridinacycloundecaphan]-3'-one;
$2^6$,8,8-trimethyl-$5^6$-(9-methyl-3,9-diazaspiro[5.5]undecan-3-yl)-$5^1$H-11-oxa-4-az a-5(2,1)-benzo[d]imidazola-1(3,4),2(2,4)-dipyridinacycloundecaphan-3-one;
(R)-3-(9-($2^6$,7-dimethyl-3-oxo-$5^1$H-11-oxa-4-aza-5(2,1)-benzo[d]imidazola-1(3,4 ),2(2,4)-dipyridinacycloundecaphane-56-yl)-3,9-diazaspiro[5.5]undecan-3-yl)propana mide;
(R)-$2^6$,7-dimethyl-$5^6$-(9-methyl-3,9-diazaspiro[5.5]undecan-3-yl)-$5^5$-(oxetan-3-yl amino)-$5^1$H-11-oxa-4-aza-5(2,1)-benzo[d]imidazola-1(3,4),2(2,4)-dipyridinacyclound ecaphan-3-one;
(R)-2-(9-($1^6$-fluoro-$2^6$,7-dimethyl-3-oxo-$5^1$H-11-oxa-4-aza-5(2,1)-benzo[d]imida zola-1(3,4),2(2,4)-dipyridinacycloundecaphane-$5^6$-yl)-3,9-diazaspiro[5.5]undecan-3-y l)ethyl methylcarbamate;
(R)-3-(9-($2^6$,7-dimethyl-3-oxo-$5^1$H-11-oxa-4-aza-5(2,1)-benzo[d]imidazola-1(3,4 ),2(2,4)-dipyridinacycloundecaphane-$5^6$-yl)-3,9-diazaspiro[5.5]undecan-3-yl)-N,N-di methylpropanamide;
(R)-3-(9-($1^6$,$5^5$-difluoro-$2^6$,7-dimethyl-3-oxo-$5^1$H-11-oxa-4-aza-5(2,1)-benzo[d]i midazola-1(3,4),2(2,4)-dipyridinacycloundecaphane-$5^6$-yl)-3,9-diazaspiro[5.5]undeca n-3-yl)propanamide;
(R)-$1^6$,$5^5$-difluoro-$5^6$-(9-(2-methoxyethyl)-3,9-diazaspiro[5.5]undecan-3-yl)-$2^6$,7-dimethyl-$5^1$H-11-oxa-4-aza-5(2,1)-benzo[d]imidazola-1(3,4),2(2,4)-dipyridinacycloun decaphan-3-one;
(R)-3-(9-($1^6$-fluoro-$2^6$,7-dimethyl-3-oxo-$5^1$H-11-oxa-4-aza-5(2,1)-benzo[d]imida zola-1(3,4),2(2,4)-dipyridinacycloundecaphane-$5^6$-yl)-3,9-diazaspiro[5.5]undecan-3-y l)-N,N-dimethylpropanamide;
(R)-$2^6$,7-dimethyl-$5^6$-(9-(oxetan-3-yl)-3,9-diazaspiro[5.5]undecan-3-yl)-3-oxo-$5^1$ H-11-oxa-4-aza-5(2,1)-benzo[d]imidazola-1(3,4),2(2,4)-dipyridinacycloundecaphane-$1^6$-carbonitrile;
(R)-$5^6$-(9-(3-(azetidin-1-yl)-3-oxopropyl)-3,9-diazaspiro[5.5]undecan-3-yl)-$2^6$,7-dimethyl-$5^1$H-11-oxa-4-aza-5(2,1)-benzo[d]imidazola-1(3,4),2(2,4)-dipyridinacycloun decaphan-3-one;
(R)-$5^6$-(9-(3-(azetidin-1-yl)-3-oxopropyl)-3,9-diazaspiro[5.5]undecan-3-yl)-$1^6$-fl uoro-$2^6$,7-dimethyl-$5^1$H-11-oxa-4-aza-5(2,1)-benzo[d]imidazola-1(3,4),2(2,4)-dipyridi nacycloundecaphan-3-one;
(R)-$2^6$,7-dimethyl-$5^6$-(9-(2-(trifluoromethoxy)ethyl)-3,9-diazaspiro[5.5]undecan-3-yl)-$5^1$H-11-oxa-4-aza-5(2,1)-benzo[d]imidazola-1(3,4),2(2,4)-dipyridinacycloundec aphan-3-one;
(R)-$1^6$-fluoro-$2^6$,7-dimethyl-$5^6$-(9-(2-(trifluoromethoxy)ethyl)-3,9-diazaspiro[5.5 ]undecan-3-yl)-$5^1$H-11-oxa-4-aza-5(2,1)-benzo[d]imidazola-1(3,4),2(2,4)-dipyridinac ycloundecaphan-3-one;
(R)-$5^6$-(9-(3-(3,3-dimethylazetidin-1-yl)-3-oxopropyl)-3,9-diazaspiro[5.5]undeca n-3-yl)-$2^6$,7-dimethyl-$5^1$H-11-oxa-4-aza-5(2,1)-benzo[d]imidazola-1(3,4),2(2,4)-dipyr idinacycloundecaphan-3-one; and
(R)-2-(9-($2^6$,7-dimethyl-3-oxo-$5^1$H-11-oxa-4-aza-5(2,1)-benzo[d]imidazola-1(3,4 ),2(2,4)-dipyridinacycloundecaphane-$5^6$-yl)-3,9-diazaspiro[5.5]undecan-3-yl)ethyl carbamate.

**32.** A pharmaceutical composition, wherein the pharmaceutical composition comprises a therapeutically effective amount of at least one compound according to any one of claims 1-31, or its stereoisomer, tautomer, solvate, pharmaceutically acceptable salt or deuterated derivative, and at least one pharmaceutically acceptable excipient.

**33.** Use of the compound according to any one of claims 1 to 31, or its stereoisomer, tautomer, solvate, pharmaceutically acceptable salt or deuterated derivative, or the pharmaceutical composition according to claim 32, in the preparation of an anti-tumor drug; preferably, the anti-tumor drug is used for the following diseases: head and neck cancer, melanoma, bladder cancer, esophageal cancer, anaplastic large cell lymphoma, renal cell carcinoma, breast cancer, colorectal cancer, ovarian cancer, cervical cancer, pancreatic cancer, glioma, glioblastoma, prostate cancer, leukemia, lymphoma, non-Hodgkin's lymphoma, gastric cancer, lung cancer, hepatocellular carcinoma, gastrointestinal stromal tumor, thyroid cancer, bile duct cancer, endometrial cancer, multiple myeloma or mesothelioma.

**34.** The use according to claim 33, wherein the tumor is malignant tumor carrying EGFR gene mutation; preferably, the EGFR gene mutation is selected from the group consisting of: EGFR Del19 gene mutation, EGFR L858R gene mutation, EGFR T790M gene mutation, EGFR C797S gene mutation, or a combination thereof.

**INTERNATIONAL SEARCH REPORT**

International application No. **PCT/CN2023/103621**

**A. CLASSIFICATION OF SUBJECT MATTER**

C07D498/18(2006.01)i; C07D498/22(2006.01)i; A61K31/439(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D A61K A61P

Documentation searched other than minimum documentation to the extent that such documents are included in the fields searched

Electronic data base consulted during the international search (name of data base and, where practicable, search terms used)

CNABS, CNTXT, CNKI, 万方数据库, WANFANG DATABASE, 百度学术, BAIDU SCHOLAR, VEN, ENTXT, STN; 贝达药业, 大环化合物, 苯并[d]咪唑, 二氮杂螺[5.5]十一烷, 吡啶, EGFR抑制剂, macrocyclic, benzo[d]imidazole, diazaspiro[5.5]undecane, pyridin, EGFR inhibitors.

**C. DOCUMENTS CONSIDERED TO BE RELEVANT**

| Category* | Citation of document, with indication, where appropriate, of the relevant passages | Relevant to claim No. |
|---|---|---|
| X | CN 114007698 A (BOEHRINGER INGELHEIM INTERNATIONAL GMBH) 01 February 2022 (2022-02-01)<br>description, pp. 7-8, 15-24, 29-30, 34, and 93-99 | 1-34 |
| X | CN 114057771 A (BEIJING ANSHI BIOTECHNOLOGY CO., LTD.) 18 February 2022 (2022-02-18)<br>claims 1-6 and 8-10 | 1-34 |
| A | CN 114656482 A (NANJING SANHOME PHARMACEUTICAL CO., LTD.) 24 June 2022 (2022-06-24)<br>entire document | 1-34 |
| A | CN 114163454 A (SHANGHAI HANSOH BIOMEDICAL CO., LTD. et al.) 11 March 2022 (2022-03-11)<br>entire document | 1-34 |
| A | WO 2022011123 A1 (BLOSSOMHILL THERAPEUTICS INC.) 13 January 2022 (2022-01-13)<br>entire document | 1-34 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **27 September 2023** | **28 September 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/103621**

| Patent document cited in search report | | | Publication date (day/month/year) | Patent family member(s) | | | Publication date (day/month/year) |
|---|---|---|---|---|---|---|---|
| CN | 114007698 | A | 01 February 2022 | JP | 2022538228 | A | 01 September 2022 |
| | | | | US | 2022380382 | A1 | 01 December 2022 |
| | | | | EP | 3986564 | A1 | 27 April 2022 |
| | | | | WO | 2020260252 | A1 | 30 December 2020 |
| CN | 114057771 | A | 18 February 2022 | WO | 2022117051 | A1 | 09 June 2022 |
| CN | 114656482 | A | 24 June 2022 | WO | 2022135432 | A1 | 30 June 2022 |
| CN | 114163454 | A | 11 March 2022 | | None | | |
| WO | 2022011123 | A1 | 13 January 2022 | US | 2023257396 | A1 | 17 August 2023 |
| | | | | JP | 2023532946 | A | 01 August 2023 |
| | | | | CA | 3187834 | A1 | 13 January 2022 |
| | | | | IL | 299732 | A | 01 March 2023 |
| | | | | BR | 112023000463 | A2 | 28 March 2023 |
| | | | | KR | 20230037564 | A | 16 March 2023 |
| | | | | TW | 202216730 | A | 01 May 2022 |
| | | | | EP | 4178958 | A1 | 17 May 2023 |
| | | | | CN | 116171279 | A | 26 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020260252 A
- CN 114163454 A


### Non-patent literature cited in the description


- Design of Prodrugs. Elsevier, 1985